# EUROPEAN PATENT APPLICATION

(11) **EP 3 767 633 A1**
(43) Date of publication of application: **20.01.2021**
(21) Application number: 20190687.2
(22) Date of filing: 03.07.2013
(51) Int. Cl.: G16H 20/17, G16H 40/63, G16H 40/67, G16H 40/40, G16H 20/60

(54) **SYSTEMS AND METHODS FOR LEVERAGING SMARTPHONE FEATURES IN CONTINUOUS GLUCOSE MONITORING**

(30) Priority: 09.07.2012 US 201261669574 P; 13.03.2013 US 201313801445
(62) Divisional of application: 13745489.8
(71) Applicant: Dexcom, Inc., San Diego, CA 92121 (US)
(72) Inventor: MENSINGER, Michael, Robert, San Diego, CA California 92129 (US); BHAVARAJU, Naresh, C., San Diego, California 92131 (US); BOWMAN, Leif, N., Livermore, CA California 94550 (US); CARLTON, Alexandra, Lynn, San Marcos, CA California 92069 (US); DERENZY, David, Petaluma, California 94952 (US); GARCIA, Arturo, Chula Vista, CA California 91910 (US); GAUBA, Indrawati, San Diego, California 92127 (US); HALL, Ashley, Escondido, CA California 92027 (US); HALL, Thomas, San Diego, CA California 92131 (US); HAMPAPURAM, Hari, San Diego, CA California 92130 (US); KAZALBASH, Murrad, Placentia, CA California 92870 (US); MAHALINGAM, Aarthi, San Diego, CA California 92126 (US); PRYOR, Jack, Ladera Ranch, CA California 92694 (US); DAVIS, Anna Leigh, Cardiff by the Sea California 92007 (US); REIHMAN, Eli, San Diego, CA California 92129 (US); SAN VICENTE, Kenneth, San Diego, CA California 92130 (US); SIMPSON, Peter, C., Cardiff, CA California 92007 (US); STEELE, Alexander, La Jolla, CA California 92037 (US); VALDES, Jorge, San Diego, CA California 92130 (US); ESTES, Michael, J., Poway, CA California 92064 (US); COHEN, Eric, San Diego, CA California 92129 (US)
(74) Representative: Dentons UK and Middle East LLP

(57) **Abstract**

The present embodiments harness a wide variety of capabilities of modern smartphones, and combine these capabilities with information from a continuous glucose monitor to provide diabetics and related people with more information than the continuous glucose monitor can provide by itself. The increased information provides the diabetic with an increased likelihood of good diabetes management for better health.

## Description

### Incorporation By Reference To Related Applications

This application claims the benefit of U.S. Application No. 13/801,445, filed on March 13, 2013 and U.S. Provisional Application No. 61/669,574, filed on July 9, 2012, the disclosures of which are hereby expressly incorporated by reference in their entirety and are hereby expressly made a portion of this application.

### Technical Field

The present embodiments relate to continuous glucose monitoring, including enhancing such monitoring by leveraging features of smartphones, tablet computers, etc.

### Background

Diabetes mellitus is a disorder in which the pancreas cannot create sufficient insulin (Type I or insulin dependent) and/or in which insulin is not effective (Type 2 or non-insulin dependent). In the diabetic state, the victim suffers from high blood sugar, which can cause an array of physiological derangements associated with the deterioration of small blood vessels, for example, kidney failure, skin ulcers, or bleeding into the vitreous of the eye. A hypoglycemic reaction (low blood sugar) can be induced by an inadvertent overdose of insulin, or after a normal dose of insulin or glucose-lowering agent accompanied by extraordinary exercise or insufficient food intake.

Conventionally, a person with diabetes carries a self-monitoring blood glucose (SMBG) monitor, which typically requires uncomfortable finger pricks to obtain blood samples for measurement. Due to the lack of comfort and convenience associated with finger pricks, a person with diabetes normally only measures his or her glucose levels two to four times per day. Unfortunately, time intervals between measurements can be spread far enough apart that the person with diabetes finds out too late of a hyperglycemic or hypoglycemic condition, sometimes incurring dangerous side effects. It is not only unlikely that a person with diabetes will take a timely SMBG value, it is also likely that he or she will not know if his or her blood glucose value is going up (higher) or down (lower) based on conventional methods. Diabetics thus may be inhibited from making educated insulin therapy decisions.

Another device that some diabetics use to monitor their blood glucose is a continuous analyte sensor. A continuous analyte sensor typically includes a sensor that is placed subcutaneously, transdermally (e.g., transcutaneously), or intravascularly. The sensor measures the concentration of a given analyte within the body, and generates a raw signal that is transmitted to electronics associated with the sensor. The raw signal is converted into an output value that is displayed on a display. The output value that results from the conversion of the raw signal is typically expressed in a form that provides the user with meaningful information, such as blood glucose expressed in mg/dL.

### Summary

The various embodiments of the present systems and methods for leveraging smartphone features in continuous glucose monitoring have several features, no single one of which is solely responsible for their desirable attributes. Without limiting the scope of the present embodiments as expressed by the claims that follow, their more prominent features now will be discussed briefly. After considering this discussion, and particularly after reading the section entitled "Detailed Description," one will understand how the features of the present embodiments provide the advantages described herein.

While the various embodiments are described with reference to example combinations of features and/or concepts, it should be understood that the features and concepts described herein may be combinable in other ways not specifically described. For example, the various embodiments are described in the paragraphs below in terms of various aspects. A feature or concept appearing in reference to one of these aspects may be combined with features and concepts described in reference to any other aspect.

One aspect of the present embodiments includes the realization that data from a continuous analyte sensor, by itself, may not be sufficient to inform every decision that diabetics must make to manage their blood glucose. Another aspect of the present embodiments includes the realization that smartphones, tablet computers and other similar devices include many features that could be leveraged to provide diabetics with more information that would help them better manage their blood glucose. Accordingly, certain of the present embodiments provide an application that can be executed by a smartphone, tablet computer, or other similar device, that receives as inputs both sensor data and data from one or more other applications executed by the device, and that processes the combined data to provide an output that is more informative than an output that can be produced with sensor data alone.

In a first aspect, a machine-executed method of continuous analyte monitoring is provided, the method comprising: receiving a first input from a module executed by an electronic device; receiving a second input from a continuous analyte monitoring device; processing the first and second inputs; and producing an output. In an embodiment of the first aspect, the first input is received from a timekeeping/scheduling module associated with the electronic device. In an embodiment of the first aspect, the processing comprises synchronizing a continuous analyte monitoring (CAM) module executed by the electronic device with the timekeeping/scheduling module. In an embodiment of the first aspect, the output is a change in an operating mode of the electronic device. In an embodiment of the first aspect, the operating mode is a vibrate mode or a silent mode. In an embodiment of the first aspect, the output is a change in an operating mode of the CAM module. In an embodiment of the first aspect, the processing comprises analyzing a user's blood glucose data. In an embodiment of the first aspect, the output is to schedule an event on the timekeeping/scheduling module. In an embodiment of the first aspect, the event is insertion of a new continuous analyte sensor, to eat, or to exercise. In an embodiment of the first aspect, the output is a recommendation. In an embodiment of the first aspect, the recommendation is a therapy, to schedule a doctor's appointment, to call a caretaker, to send data to a caretaker, to send data to a doctor, to eat a meal, to exercise, to replace a sensor, to calibrate a sensor, to check blood glucose, to upload or sync data to a cloud computing system. In an embodiment of the first aspect, the recommendation is provided to the user, a caretaker, a parent, a guardian, or a healthcare professional. In an embodiment of the first aspect, the recommendation is provided via screen prompt, text message, email message, or social network posting. In an embodiment of the first aspect, the output is a prompt to the user. In an embodiment of the first aspect, the prompt is an indication of when a next calibration is due, to check blood glucose, to schedule a doctor's appointment, to send data to a caretaker, to display in-case-of-emergency contact information, a time to next calibration, or a time to replace a sensor. In an embodiment of the first aspect, the first input is received from a personal contacts module. In an embodiment of the first aspect, the processing comprises analyzing a user's blood glucose data. In an embodiment of the first aspect, the personal contacts module includes a list of personal contacts stored on the electronic device or remotely. In an embodiment of the first aspect, the output is a display of emergency contact information on a display of the electronic device. In an embodiment of the first aspect, the personal contacts module includes an online social network. In an embodiment of the first aspect, the output is a posting to the online social network. In an embodiment of the first aspect, the first input is received from an audio module. In an embodiment of the first aspect, the processing comprises correlating an effect of a given song on a user's blood glucose level. In an embodiment of the first aspect, the output is data stored for later retrospective viewing, or an input to a cloud computing system. In an embodiment of the first aspect, the first input is received from an activity monitor. In an embodiment of the first aspect, the processing comprises correlating an effect of a user's physical activity on his or her blood glucose level. In an embodiment of the first aspect, the physical activity is sleeping, light exercise, moderate exercise, intense exercise, waking sedentary activity, driving, or flying. In an embodiment of the first aspect, the output is a pattern of how the physical activity affects the user's blood glucose level. In an embodiment of the first aspect, the output is a warning of a low blood glucose level while the user is engaged in certain activities that might amplify a level of danger associated with the low blood glucose level, wherein the warning is distinct from a standard alert or alarm typically issued when the user is not engaged one of the certain activities. In an embodiment of the first aspect, the output is a warning of a low blood glucose level while the user is engaged in certain activities that might amplify a level of danger associated with the low blood glucose level, wherein the warning is provided at a different blood glucose threshold than a threshold that would trigger an alert when the user is not engaged one of the certain activities. In an embodiment of the first aspect, the output is a warning to a user that sensor data may not be accurate because of the user's surroundings or activity. In an embodiment of the first aspect, the output is used as an input to a blood glucose profile, an alarm algorithm, an insulin algorithm, an interaction log. In an embodiment of the first aspect, the output to the alarm algorithm is to provide a stronger alarm when the user is sleeping. In an embodiment of the first aspect, the first input is received from an image capture module. In an embodiment of the first aspect, the first input is information about food consumed. In an embodiment of the first aspect, the processing comprises correlating the information about food consumed with a user's blood glucose level. In an embodiment of the first aspect, the output is data stored for later retrospective viewing, or an input to a cloud computing system. In an embodiment of the first aspect, the first input is information about an activity engaged in. In an embodiment of the first aspect, the processing comprises correlating the information about food consumed with a user's blood glucose level. In an embodiment of the first aspect, the output is data stored for later retrospective viewing, or an input to a cloud computing system. In an embodiment of the first aspect, the first input is a blood glucose meter value. In an embodiment of the first aspect, the blood glucose meter value is based on a photo taken of a blood glucose meter. In an embodiment of the first aspect, the output is calibrated sensor data. In an embodiment of the first aspect, the first input is a location of a sensor of the continuous analyte monitoring device positioned on a user's body. In an embodiment of the first aspect, the processing comprises correlating a quality of data obtained during a sensor session with the sensor's location on the body. In an embodiment of the first aspect, the first input is a location on a user's body where at least one previous insulin injection was made. In an embodiment of the first aspect, the first input is information about food to be consumed. In an embodiment of the first aspect, the first input is an estimate of a quantity of carbohydrates in the food. In an embodiment of the first aspect, the output is a recommended therapy, such as an insulin dosage, a recommendation not to eat the food, or a recommendation to eat only a fraction of the food. In an embodiment of the first aspect, the output is an estimate of a user's future blood glucose level if the food is consumed. In an embodiment of the first aspect, the first input is indicative of a user's location. In an embodiment of the first aspect, a location module provides the first input based on information received from a global positioning system (GPS) receiver. In an embodiment of the first aspect, the processing comprises determining that the user's blood glucose is low, and obtaining information on nearby locations where food can be obtained. In an embodiment of the first aspect, the output is the information on nearby locations where food can be obtained. In an embodiment of the first aspect, the processing comprises evaluating restaurants in a predetermined area and ranking those restaurants based on diabetic considerations. In an embodiment of the first aspect, the output is a recommendation on where to eat. In an embodiment of the first aspect, the processing comprises comparing blood glucose data and location data against threshold values. In an embodiment of the first aspect, the threshold values are a predetermined blood glucose level indicating a hypoglycemic event, and a distance from the user's home. In an embodiment of the first aspect, when the threshold values are met, the output is a warning that is distinct from a standard alert or alarm. In an embodiment of the first aspect, the processing comprises comparing a battery level of a CAM and location data against threshold values. In an embodiment of the first aspect, the output is a warning when the battery level is below a first one of the threshold values, and the user's location is greater than a second one of the threshold values. In an embodiment of the first aspect, the processing comprises correlating a user's location and his or her blood glucose level. In an embodiment of the first aspect, the first input is indicative of a user's motion. In an embodiment of the first aspect, the processing comprises determining that the user is driving or riding in a vehicle, and correlating that determination an effect thereof on his or her blood glucose level.

It is noted any embodiment of the first aspect is generally applicable with one, some or all of the other embodiments of the first aspect or any other aspect (i.e. independently combinable with any of the aspects or embodiments identified herein).

In a second aspect, a system for continuous analyte monitoring is provided, the system comprising: a computing device; a continuous analyte monitoring (CAM) module executed by the computing device; an auxiliary interface executed by the computing device; and a CAM; wherein the CAM module is configured to receive a first input from the auxiliary interface, receive a second input from the CAM, process the first and second inputs, and produce an output. In an embodiment of the second aspect, the first input is received from a timekeeping/scheduling module associated with the electronic device. In an embodiment of the second aspect, the processing comprises synchronizing the CAM module with the timekeeping/scheduling module. In an embodiment of the second aspect, the output is a change in an operating mode of the electronic device. In an embodiment of the second aspect, the operating mode is a vibrate mode or a silent mode. In an embodiment of the second aspect, the output is a change in an operating mode of the CAM module. In an embodiment of the second aspect, the processing comprises analyzing a user's blood glucose data. In an embodiment of the second aspect, the output is to schedule an event on the timekeeping/scheduling module. In an embodiment of the second aspect, the event is insertion of a new continuous analyte sensor, to eat, or to exercise. In an embodiment of the second aspect, the output is a recommendation. In an embodiment of the second aspect, the recommendation is a therapy, to schedule a doctor's appointment, to call a caretaker, to send data to a caretaker, to send data to a doctor, to eat a meal, to exercise, to replace a sensor, to calibrate a sensor, to check blood glucose, to upload or sync data to a cloud computing system. In an embodiment of the second aspect, the recommendation is provided to the user, a caretaker, a parent, a guardian, or a healthcare professional. In an embodiment of the second aspect, the recommendation is provided via screen prompt, text message, email message, or social network posting. In an embodiment of the second aspect, the output is a prompt to the user. In an embodiment of the second aspect, the prompt is an indication of when a next calibration is due, to check blood glucose, to schedule a doctor's appointment, to send data to a caretaker, to display in-case-of-emergency contact information, a time to next calibration, or a time to replace a sensor. In an embodiment of the second aspect, the first input is received from a personal contacts module. In an embodiment of the second aspect, the processing comprises analyzing a user's blood glucose data. In an embodiment of the second aspect, the personal contacts module includes a list of personal contacts stored on the electronic device or remotely. In an embodiment of the second aspect, the output is a display of emergency contact information on a display of the electronic device. In an embodiment of the second aspect, the personal contacts module includes an online social network. In an embodiment of the second aspect, the output is a posting to the online social network. In an embodiment of the second aspect, the first input is received from an audio module. In an embodiment of the second aspect, the processing comprises correlating an effect of a given song on a user's blood glucose level. In an embodiment of the second aspect, the output is data stored for later retrospective viewing, or an input to a cloud computing system. In an embodiment of the second aspect, the first input is received from an activity monitor. In an embodiment of the second aspect, the processing comprises correlating an effect of a user's physical activity on his or her blood glucose level. In an embodiment of the second aspect, the physical activity is sleeping, light exercise, moderate exercise, intense exercise, waking sedentary activity, driving, flying. In an embodiment of the second aspect, the output is a pattern of how the physical activity affects the user's blood glucose level. In an embodiment of the second aspect, the output is a warning of a low blood glucose level while the user is engaged in certain activities that might amplify a level of danger associated with the low blood glucose level, wherein the warning is distinct from a standard alert or alarm typically issued when the user is not engaged one of the certain activities. In an embodiment of the second aspect, the output is a warning of a low blood glucose level while the user is engaged in certain activities that might amplify a level of danger associated with the low blood glucose level, wherein the warning is provided at a different blood glucose threshold than a threshold that would trigger an alert when the user is not engaged one of the certain activities. In an embodiment of the second aspect, the output is a warning to a user that sensor data may not be accurate because of the user's surroundings or activity. In an embodiment of the second aspect, the output is used as an input to a blood glucose profile, an alarm algorithm, an insulin algorithm, an interaction log. In an embodiment of the second aspect, the output to the alarm algorithm is to provide a stronger alarm when the user is sleeping. In an embodiment of the second aspect, the first input is received from an image capture module. In an embodiment of the second aspect, the first input is information about food consumed. In an embodiment of the second aspect, the processing comprises correlating the information about food consumed with a user's blood glucose level. In an embodiment of the second aspect, the output is data stored for later retrospective viewing, or an input to a cloud computing system. In an embodiment of the second aspect, the first input is information about an activity engaged in. In an embodiment of the second aspect, the processing comprises correlating the information about food consumed with a user's blood glucose level. In an embodiment of the second aspect, the output is data stored for later retrospective viewing, or an input to a cloud computing system. In an embodiment of the second aspect, the first input is a blood glucose meter value. In an embodiment of the second aspect, the blood glucose meter value is based on a photo taken of a blood glucose meter. In an embodiment of the second aspect, the output is calibrated sensor data. In an embodiment of the second aspect, the first input is a location of a sensor of the continuous analyte monitoring device positioned on a user's body. In an embodiment of the second aspect, the processing comprises correlating a quality of data obtained during a sensor session with the sensor's location on the body. In an embodiment of the second aspect, the first input is a location on a user's body where at least one previous insulin injection was made. In an embodiment of the second aspect, the first input is information about food to be consumed. In an embodiment of the second aspect, the first input is an estimate of a quantity of carbohydrates in the food. In an embodiment of the second aspect, the output is a recommended therapy, such as an insulin dosage, a recommendation not to eat the food, or a recommendation to eat only a fraction of the food. In an embodiment of the second aspect, the output is an estimate of a user's future blood glucose level if the food is consumed. In an embodiment of the second aspect, the first input is indicative of a user's location. In an embodiment of the second aspect, a location module provides the first input based on information received from a global positioning system (GPS) receiver. In an embodiment of the second aspect, the processing comprises determining that the user's blood glucose is low, and obtaining information on nearby locations where food can be obtained. In an embodiment of the second aspect, the output is the information on nearby locations where food can be obtained. In an embodiment of the second aspect, the processing comprises evaluating restaurants in a predetermined area and ranking those restaurants based on diabetic considerations. In an embodiment of the second aspect, the output is a recommendation on where to eat. In an embodiment of the second aspect, the processing comprises comparing blood glucose data and location data against threshold values. In an embodiment of the second aspect, the threshold values are a predetermined blood glucose level indicating a hypoglycemic event, and a distance from the user's home. In an embodiment of the second aspect, when the threshold values are met, the output is a warning that is distinct from a standard alert or alarm. In an embodiment of the second aspect, the processing comprises comparing a battery level of a CAM and location data against threshold values. In an embodiment of the second aspect, the output is a warning when the battery level is below a first one of the threshold values, and the user's location is greater than a second one of the threshold values. In an embodiment of the second aspect, the processing comprises correlating a user's location and his or her blood glucose level. In an embodiment of the second aspect, the first input is indicative of a user's motion. In an embodiment of the second aspect, the processing comprises determining that the user is driving or riding in a vehicle, and correlating that determination an effect thereof on his or her blood glucose level.

It is noted any embodiment of the second aspect is generally applicable with one, some or all of the other embodiments of the second aspect or any other aspect (i.e. independently combinable with any of the aspects or embodiments identified herein).

In a third aspect, a machine-executed method of continuous analyte monitoring for a host to facilitate management of the host's blood glucose level is provided, the method comprising: receiving a first input from an activity monitor executed by an electronic device, wherein the activity monitor provides information regarding the activity; receiving a second input from a continuous analyte monitoring (CAM) device, wherein the CAM device measures a concentration of an analyte within the host and outputs a value that represents the host's blood glucose level; processing the first and second inputs, wherein correlations or impacts of the activity on the host's blood glucose level are determined to associate the host's blood glucose level with the activity; and producing an output associated with the activity responsive to the determined associations. In an embodiment of the third aspect, the first and second inputs comprise applying one or more algorithms to two streams of input, including algorithms useful for correlating data and/or recognizing patterns. In an embodiment of the third aspect, the output is data that is stored locally or remotely for retrospective analysis. In an embodiment of the third aspect, the activities include exercising and sleeping.

It is noted any embodiment of the third aspect is generally applicable with one, some or all of the other embodiments of the third aspect or any other aspect (i.e. independently combinable with any of the aspects or embodiments identified herein).

In a fourth aspect, a machine-executed method of continuous analyte monitoring for a host to facilitate management of the host's blood glucose level is provided, the method comprising: optionally receiving a first input from a timekeeping/scheduling module executed by an electronic device, the first input including information about an upcoming event; receiving a second input from a continuous analyte monitoring (CAM) device including analyte concentration data of the host; processing the first and second inputs by analyzing an event or an operational mode associated with either of the timekeeping/scheduling module or the CAM device; and producing an output by synchronizing the event or the operational mode of at least one of the timekeeping/scheduling module and the CAM device with the other of the timekeeping/scheduling module and the CAM device. In an embodiment of the fourth aspect, the output is to the timekeeping/scheduling module to schedule an event. In an embodiment of the fourth aspect, the event is to eat, to obtain a reference glucose value, or to inject insulin. In an embodiment of the fourth aspect, the wherein the output is sent to a user, a caretaker, a parent, a guardian, or a healthcare professional. In an embodiment of the fourth aspect, the output is a recommendation provided via screen prompt, text message, e-mail message, or a post to a social network.

It is noted any embodiment of the fourth aspect is generally applicable with one, some or all of the other embodiments of the fourth aspect or any other aspect (i.e. independently combinable with any of the aspects or embodiments identified herein).

In a fifth aspect, a machine-executed method of continuous analyte monitoring for a host to facilitate management of the host's blood glucose level is provided, the method comprising: receiving a first input from an image capture module executed by an electronic device, the first input providing information about at least one of a location, an activity, or a food; receiving a second input from a continuous analyte monitoring (CAM) device including analyte concentration data of the host; processing the first and second inputs by correlating information associated with the first input with the analyte concentration data of the host; and outputting information associated with the first input. In an embodiment of the fifth aspect, the second input is an estimated glucose value at a time substantially corresponding to a time when the photograph was taken. In an embodiment of the fifth aspect, the output is data that is stored in a first location and referenced for algorithmic processing, wherein the algorithm runs a correlation analysis or pattern recognition. In an embodiment of the fifth aspect, the first input is a photograph of a display of a blood glucose meter.

It is noted any embodiment of the fifth aspect is generally applicable with one, some or all of the other embodiments of the fifth aspect or any other aspect (i.e. independently combinable with any of the aspects or embodiments identified herein).

In a sixth aspect, a method of placing a sensor of a continuous analyte monitor (CAM) on a host is provided, the method comprising: receiving a first input from an image capture module executed by an electronic device, wherein the first input is an image of the host's body; processing the first input by referencing one or more stored locations on the body where the sensor was previously placed; and producing an output including a recommendation of a new location on the body where the sensor should be placed.

It is noted any embodiment of the sixth aspect is generally applicable with one, some or all of the other embodiments of the sixth aspect or any other aspect (i.e. independently combinable with any of the aspects or embodiments identified herein).

In a seventh aspect, a machine-executed method of continuous analyte monitoring for a host to facilitate management of the host's blood glucose level is provided, the method comprising: receiving a first input from a contacts module executed by an electronic device, the first input including at least contact information for one or more third parties; receiving a second input from a continuous analyte monitoring (CAM) device including analyte concentration data of the host; processing the first and second inputs by determining that the analyte concentration data should be shared with the one or more third parties; and outputting the analyte concentration data to the one or more third parties. In an embodiment of the seventh aspect, the first input is a name and contact information for an emergency contact. In an embodiment of the seventh aspect, the second input is an estimated glucose value (EGV). In an embodiment of the seventh aspect, the processing comprises comparing the EGV to a threshold value. In an embodiment of the seventh aspect, the output is to display the name and contact information for the emergency contact on a display. It is noted any embodiment of the seventh aspect is generally applicable with one, some or all of the other embodiments of the seventh aspect or any other aspect (i.e. independently combinable with any of the aspects or embodiments identified herein).

In an eighth aspect, a machine-executed method of continuous analyte monitoring for a host to facilitate management of the host's blood glucose level is provided, the method comprising: receiving a first input from a location module executed by an electronic device, wherein the first input is a location of the user; receiving a second input from a continuous analyte monitoring (CAM) device including analyte concentration data of the host; processing the first and second inputs by comparing the location against a first criterion and comparing the analyte concentration data to a second criterion; and outputting an alarm if the first and second criteria are met. In an embodiment of the eighth aspect, the second input is an estimated glucose value (EGV).It is noted any embodiment of the eighth aspect is generally applicable with one, some or all of the other embodiments of the eighth aspect or any other aspect (i.e. independently combinable with any of the aspects or embodiments identified herein).

In a ninth aspect, a machine-executed method of continuous analyte monitoring for a host to adaptively adjust the alarm sounds, tunes, songs, pitches and/or levels of an alert based on the analysis of or correlation to good glucose control is provided, the method comprising: triggering a first type of alert associated with a condition of the host based on analyte concentration data from a continuous analyte monitoring (CAM) device, wherein the alert comprise an alarm sound, tune, song, pitch and/or level; receiving input from the CAM device including analyte concentration data of the host indicative of the host's analyte response to the first type of alert; analyzing a correlation between the first type of alert and the input from the CAM device responsive to the first type of alert to determine a level of glucose control achieved responsive to the alert; and adaptively adjusting an alarm sound, tune, song, pitch and/or level of the first type of responsive to analysis.

It is noted any embodiment of the ninth aspect is generally applicable with one, some or all of the other embodiments of the ninth aspect or any other aspect (i.e. independently combinable with any of the aspects or embodiments identified herein).

In a tenth aspect, a machine-executed method of continuous analyte monitoring for a host to facilitate management of the host's blood glucose level is provided, the method comprising: receiving a first input from a continuous analyte monitor (CAM) device including analyte concentration data of the host; receiving or determining an expected maximum value and an expected minimum value of blood glucose from a blood glucose meter; receiving a second input from a blood glucose meter of a blood glucose value of the host; processing the second input by comparing it to the expected maximum value and the expected minimum value; and producing an output of an alert to obtain a new blood glucose value when the second input is outside a range defined by the expected maximum value and the expected minimum value.

It is noted any embodiment of the tenth aspect is generally applicable with one, some or all of the other embodiments of the tenth aspect or any other aspect (i.e. independently combinable with any of the aspects or embodiments identified herein).

In an eleventh aspect, a machine-executed method of continuous analyte monitoring for a host to facilitate management of the host's blood glucose level is provided, the method comprising: receiving a first input including information about one or more foods to be consumed by the host; receiving a second input from a continuous analyte monitoring (CAM) device including analyte concentration data of the host; processing the first and second inputs by applying one or more algorithms to the first and second inputs to determine recommendation predicted effect of the one or more foods to the analyte concentration in the host; and producing an output of the insulin dosage recommendation to compensate for the predicted effect of the one or more foods. In an embodiment of the eleventh aspect, wherein the first input is information about food recently consumed or to be consumed in the near future. In an embodiment of the eleventh aspect, the second input is an estimated glucose value (EGV). In an embodiment of the eleventh aspect, the output is to a display of an electronic device. In an embodiment of the eleventh aspect, the information about the one or more foods comprises an estimated time of ingestion and an amount of carbohydrates to be ingested. In an embodiment of the eleventh aspect, the information about the one or more foods is derived from the image capture module.

It is noted any embodiment of the eleventh aspect is generally applicable with one, some or all of the other embodiments of the eleventh aspect or any other aspect (i.e. independently combinable with any of the aspects or embodiments identified herein).

In a twelfth aspect, a machine-executed method of continuous analyte monitoring for a host to facilitate management of the host's blood glucose level is provided, the method comprising: receiving an estimated glucose value (EGV) and upper and lower boundaries for a next blood glucose (BG) value from a continuous glucose monitor (CGM); storing the upper and lower boundaries for the next BG value; receiving the next BG value; processing the next BG value by comparing it with the upper and lower boundaries for the next BG value; and producing an output in the form of a request for another BG value if the next BG value is outside the upper and lower boundaries for the next BG value. In an embodiment of the twelfth aspect, the processing comprises comparing the next BG value to the upper and lower boundaries for the next BG value.

It is noted any embodiment of the twelfth aspect is generally applicable with one, some or all of the other embodiments of the twelfth aspect or any other aspect (i.e. independently combinable with any of the aspects or embodiments identified herein).

In a thirteenth aspect, a machine-executed method of continuous analyte monitoring for a host to facilitate management of the host's blood glucose level is provided, the method comprising: receiving an input from an alert module executed by a general purpose electronic device, the general purpose electronic device comprising a continuous analyte monitor (CAM) module, wherein the input is a notification of one or more alerts, settings, modes and/or profiles associated with the general purpose electronic device; synchronizing one or more alerts, settings, modes and/or profile of the CAM module responsive to the notification.

It is noted any embodiment of the thirteenth aspect is generally applicable with one, some or all of the other embodiments of the thirteenth aspect or any other aspect (i.e. independently combinable with any of the aspects or embodiments identified herein).

In a fourteenth aspect, a machine-executed method of continuous analyte monitoring for a host to facilitate management of the host's blood glucose level is provided, the method comprising: displaying a blood glucose trend graph on a display of an electronic device; receiving an input of at least one location on the trend graph; processing the input by determining a value of the host's blood glucose at the at least one location; and producing an output of the value on the display. In an embodiment of the fourteenth aspect, the input is locations of two points on the trend graph where a user has placed his or her fingers. In an embodiment of the fourteenth aspect, the processing comprises determining values of blood glucose at the two points. In an embodiment of the fourteenth aspect, the output is a value of a difference between blood glucose values at the two points. In an embodiment of the fourteenth aspect, the output is a value of a rate of change of blood glucose between the two points. In an embodiment of the fourteenth aspect, the output is shown on the display. In an embodiment of the fourteenth aspect, the blood glucose trend graph comprises a macro trend graph that displays the user's blood glucose data for a given length of time, and a micro trend graph that displays the user's blood glucose data for a segment of the time shown on the macro trend graph. In an embodiment of the fourteenth aspect, a movable bar on the macro trend graph highlights the segment of the time on the macro trend graph that is displayed on the micro trend graph. In an embodiment of the fourteenth aspect, the bar is movable to select any desired window of time on the macro trend graph to be displayed on the micro trend graph. In an embodiment of the fourteenth aspect, a width of the bar is adjustable to adjust a length of time displayed on the micro trend graph. In an embodiment of the fourteenth aspect, the width of the bar is adjustable by selecting one of a plurality of time select icons. In an embodiment of the fourteenth aspect, if a scale of the macro trend graph is changed by a given factor, values of the time select icons change by a corresponding factor.

It is noted any embodiment of the fourteenth aspect is generally applicable with one, some or all of the other embodiments of the fourteenth aspect or any other aspect (i.e. independently combinable with any of the aspects or embodiments identified herein).

In a fifteenth aspect, a machine-executed method of continuous analyte monitoring for a host to facilitate management of the host's blood glucose level is provided, the method comprising: detecting that a new operating system has been installed on an electronic device; querying a remote server to inquire whether a module executed by the electronic device is compatible with the new operating system; and producing an output. In an embodiment of the fifteenth aspect, the output is to continue operating the module. In an embodiment of the fourteenth aspect, the output is to shutdown the module.

It is noted any embodiment of the fifteenth aspect is generally applicable with one, some or all of the other embodiments of the fifteenth aspect or any other aspect (i.e. independently combinable with any of the aspects or embodiments identified herein).

In a sixteenth aspect, a machine-executed method of continuous analyte monitoring for a host to facilitate management of the host's blood glucose level is provided, the method comprising: changing a pairing of an electronic device from a first transmitter associated with a first analyte sensor to a second transmitter associated with a second analyte sensor; querying a remote server for data associated with the second transmitter; and displaying the data associated with the second transmitter on a display of the electronic device.

It is noted any embodiment of the sixteenth aspect is generally applicable with one, some or all of the other embodiments of the sixteenth aspect or any other aspect (i.e. independently combinable with any of the aspects or embodiments identified herein).

In a seventeenth aspect, a machine-executed method of continuous analyte monitoring for a host to facilitate management of the host's blood glucose level is provided, the method comprising: receiving an input of an action taken or about to be taken; receiving a second input from a continuous analyte monitoring (CAM) device including analyte concentration data of the host; processing the first and second inputs by determining what impact the action will have on the host's blood glucose level; and producing an output of a prediction of the host's blood glucose level at a specified future time. In an embodiment of the seventeenth aspect, the output is shown on a display.

It is noted any embodiment of the seventeenth aspect is generally applicable with one, some or all of the other embodiments of the seventeenth aspect or any other aspect (i.e. independently combinable with any of the aspects or embodiments identified herein).

In an eighteenth aspect, a machine-executed method of continuous analyte monitoring for a host to facilitate management of the host's blood glucose level is provided, the method comprising: a module executed by an electronic device associated with a first user receiving an input of glucose data for a second user; processing the input by preparing it for display on a display of the electronic device; and producing an output by displaying information associated with the input on the display. In an embodiment of the eighteenth aspect, the input is a screen shot. In an embodiment of the eighteenth aspect, the screen shot is displayed in a manner such that it is unlikely the first user will mistake the data for his own.

It is noted any embodiment of the eighteenth aspect is generally applicable with one, some or all of the other embodiments of the eighteenth aspect or any other aspect (i.e. independently combinable with any of the aspects or embodiments identified herein).

In a nineteenth aspect, a machine-executed method of continuous analyte monitoring for a host to facilitate management of the host's blood glucose level is provided, the method comprising: receiving an input of a number of sensors associated with a continuous analyte monitoring (CAM) device; receiving an input each time one of the sensors is used; processing the inputs by deducting one from a count of the number of sensors each time one of the sensors is initiated and comparing the count to a threshold value; and producing an output by ordering more sensors when the count drops below the threshold value.

It is noted any embodiment of the nineteenth aspect is generally applicable with one, some or all of the other embodiments of the nineteenth aspect or any other aspect (i.e. independently combinable with any of the aspects or embodiments identified herein).

Any of the features of any of the embodiments of any of the above aspects is applicable to all aspects and embodiments identified herein. Moreover, any of the features of an embodiment of any of the aspects is independently combinable, partly or wholly with other embodiments described herein in any way, e.g., one, two, or three or more embodiments may be combinable in whole or in part. Further, any of the features of an embodiment of any aspect may be made optional to other aspects or embodiments. Any aspect or embodiment of a method can be performed by a system or apparatus of another aspect or embodiment, and any aspect or embodiment of a system can be configured to perform a method of another aspect or embodiment.

### Brief Description of the Drawings

The various embodiments of the present systems and methods for leveraging smartphone features in continuous glucose monitoring now will be discussed in detail with an emphasis on highlighting the advantageous features. These embodiments depict the novel and non-obvious systems and methods shown in the accompanying drawings, which are for illustrative purposes only. These drawings include the following figures, in which like numerals indicate like parts:
Figure 1 is a schematic view of a continuous analyte sensor system attached to a host and communicating with a plurality of example devices;
Figure 2 is a functional block diagram of one embodiment of a system for leveraging smartphone features in continuous glucose monitoring;
Figure 2A is a representation of a graphical user interface configured for use with the present embodiments;
Figure 3 is a flowchart illustrating one embodiment of a method for leveraging smartphone features in continuous glucose monitoring;
Figure 3A illustrates one example of an output that the present embodiments may produce, in the form of a historical glucose trend graph displayed on a smartphone;
Figure 4 is a flowchart illustrating another embodiment of a method for leveraging smartphone features in continuous glucose monitoring;
Figure 5 is a flowchart illustrating another embodiment of a method for leveraging smartphone features in continuous glucose monitoring;
Figure 6 is a flowchart illustrating another embodiment of a method for leveraging smartphone features in continuous glucose monitoring;
Figure 7 is a flowchart illustrating another embodiment of a method for leveraging smartphone features in continuous glucose monitoring;
Figure 8 is a screenshot of a smartphone display illustrating one embodiment of emergency information that enables a bystander to assist a diabetic who is experiencing a hypoglycemic event;
Figure 9 is a flowchart illustrating another embodiment of a method for leveraging smartphone features in continuous glucose monitoring;
Figure 10 is a flowchart illustrating another embodiment of a method for leveraging smartphone features in continuous glucose monitoring;
Figure 11 is a flowchart illustrating another embodiment of a method for leveraging smartphone features in continuous glucose monitoring;
Figure 11A is a flowchart and a front elevation view of a smartphone, illustrating another embodiment of a method for leveraging smartphone features in continuous glucose monitoring;
Figure 12 is a flowchart illustrating another embodiment of a method for leveraging smartphone features in continuous glucose monitoring;
Figures 13A-13D are screenshots of a smartphone display illustrating example embodiments of interfaces for customizing alerts;
Figure 14 is a screenshot of a smartphone display illustrating one embodiment of an alert indicting that the user's blood glucose is dropping and will soon be in a low range;
Figure 15 is a screenshot of a smartphone display illustrating one embodiment of a blood glucose trend graph;
Figure 15A is an embodiment of a blood glucose trend arrow;
Figure 16 is an example of a color gradient that may be used with certain of the present embodiments;
Figure 17 is a front elevation view of a smartphone, illustrating one embodiment of displaying information to a user; and
Figures 18-21 are front elevation views of various graphical displays for indicating blood glucose levels.

### Detailed Description

The following detailed description describes the present embodiments with reference to the drawings. In the drawings, reference numbers label elements of the present embodiments. These reference numbers are reproduced below in connection with the discussion of the corresponding drawing features.

### Sensor System and Applicator

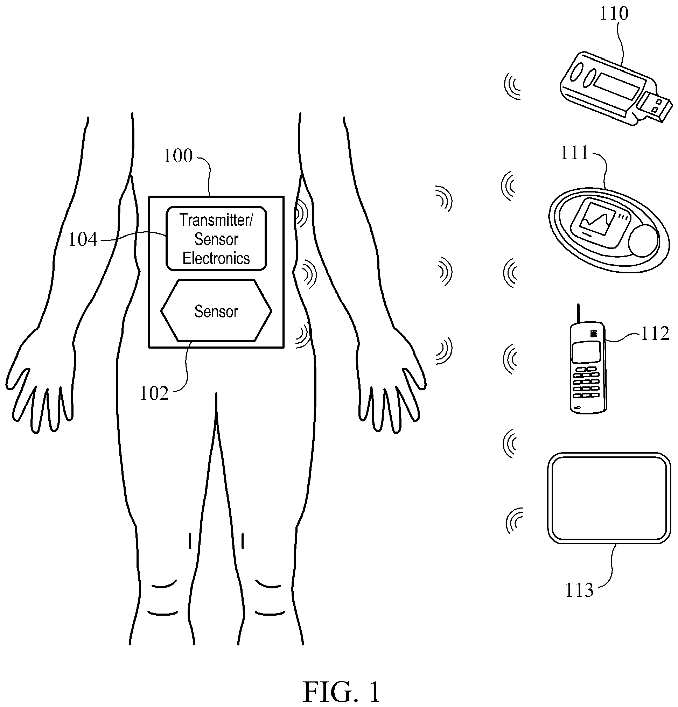

Figure 1 is a schematic view of a continuous analyte sensor system 100 attached to a host and communicating with a number of example devices 110-113. A transcutaneous analyte sensor system comprising an on-skin sensor assembly 100 is fastened to the skin of a host via a disposable housing (not shown). The system includes a transcutaneous analyte sensor 102 and a transmitter/sensor electronics unit 104 for wirelessly transmitting analyte information to a receiver. In alternative embodiments, the sensor may be non-invasive.

During use, a sensing portion of the sensor 102 is under the host's skin, and a contact portion of the sensor 102 is electrically connected to the electronics unit 104. The electronics unit 104 engages a housing (not shown), and the sensor extends through the housing. The housing, which maintains the assembly 100 on the skin and provides for electrical connection of the sensor 102 to sensor electronics provided in the electronics unit 104, is attached to an adhesive patch fastened to the skin of the host.

The on-skin sensor assembly 100 may be attached to the host with an applicator (not shown) adapted to provide convenient and secure application. Such an applicator may also be used for attaching the electronics unit 104 to a housing, inserting the sensor 102 through the host's skin, and/or connecting the sensor 102 to the electronics unit 104. Once the electronics unit 104 is engaged with the housing and the sensor 102 has been inserted and is connected to the electronics unit 104, the applicator detaches from the sensor assembly.

In general, the continuous analyte sensor system 100 includes any sensor configuration that provides an output signal indicative of a concentration of an analyte. The output signal, which may be in the form of, for example, sensor data, such as a raw data stream, filtered data, smoothed data, and/or otherwise transformed sensor data, is sent to the receiver, which is described in more detail below. In various embodiments, the analyte sensor system 100 includes a transcutaneous glucose sensor, such as that described in U.S. Patent Application Publication No. 2011/0027127, for example, which is incorporated by reference herein in its entirety. In various other embodiments, the sensor system 100 includes a subcutaneous glucose sensor, such as that described in U.S. Patent No. 6,579,690 to Bonnecaze et al. or U.S. Patent No. 6,484,046 to Say et al., for example. In various other embodiments, the sensor system 100 includes a continuous, refillable, subcutaneous glucose sensor, such as that described in U.S. Patent No. 6,512,939 to Colvin et al., for example. In various other embodiments, the sensor system 100 includes a continuous intravascular glucose sensor, such as that described in U.S. Patent No. 6,477,395 to Schulman et al., or U.S. Patent No. 6,424,847 to Mastrototaro et al., for example. Other signal processing techniques and glucose monitoring system embodiments suitable for use with the present embodiments are described in U.S. Patent Application Publication Nos. 2005/0203360 and 2009/0192745, both of which are incorporated herein by reference in their entireties.

For convenience, terms such as glucose sensor, blood glucose (BG), estimated glucose value (EGV), etc. are used herein for convenience. The present embodiments are not limited, however, to measuring glucose. The sensor 102 may be configured to measure a concentration of any substance in the body of the host. Accordingly, terms such as glucose sensor, blood glucose (BG), estimated glucose value (EGV), etc. should not be interpreted as limiting.

In some embodiments, the sensor 102 extends through a housing (not shown), which maintains the sensor on the skin and provides for electrical connection of the sensor to sensor electronics, provided in the electronics unit 104. In various embodiments, the sensor 102 is formed from a wire. For example, the sensor can include an elongated conductive body, such as a bare elongated conductive core (e.g., a metal wire) or an elongated conductive core coated with one, two, three, four, five, or more layers of material, each of which may or may not be conductive. The elongated sensor may be long and thin, yet flexible and strong. For example, in some embodiments the smallest dimension of the elongated conductive body is less than about 0.1 inches, 0.075 inches, 0.05 inches, 0.025 inches, 0.01 inches, 0.004 inches, or 0.002 inches. Preferably, a membrane system is deposited over at least a portion of electroactive surfaces of the sensor 102 (including a working electrode and optionally a reference electrode) and provides protection of the exposed electrode surface from the biological environment, diffusion resistance (limitation) of the analyte if needed, a catalyst for enabling an enzymatic reaction, limitation or blocking of interferents, and/or hydrophilicity at the electrochemically reactive surfaces of the sensor interface.

In general, the membrane system includes a plurality of domains, for example, an electrode domain, an interference domain, an enzyme domain (for example, including glucose oxidase), and a resistance domain, and can include a high oxygen solubility domain, and/or a bioprotective domain, such as is described in more detail in U.S. Patent Application Publication No. 2005/0245799. The membrane system may be deposited on the exposed electroactive surfaces using known thin film techniques (for example, spraying, electro-depositing, dipping, etc.). In various embodiments, one or more domains are deposited by dipping the sensor into a solution and drawing out the sensor at a speed that provides the appropriate domain thickness. However, the membrane system can be disposed over (or deposited on) the electroactive surfaces using any known method.

In the illustrated embodiment, the electronics unit 104 is releasably attachable to the sensor 102, which together form the on-skin sensor assembly 100. The electronics unit 104 includes electronic circuitry associated with measuring and processing the continuous analyte sensor data, and is configured to perform algorithms associated with processing and calibration of the sensor data. For example, the electronics unit 104 can provide various aspects of the functionality of a sensor electronics module as described in U.S. Patent Application Publication No. 2009/0240120, which is incorporated herein by reference in its entirety. The electronics unit 104 may include hardware, firmware, and/or software that enable measurement of levels of the analyte via a glucose sensor, such as the analyte sensor 102. For example, the electronics unit 104 can include a potentiostat, a power source for providing power to the sensor 102, other components useful for signal processing and data storage, and preferably a telemetry module for one- or two-way data communication between the electronics unit 104 and one or more receivers, repeaters, and/or display devices, such as the devices 110-113. Sensor electronics within the electronics unit 104 can be affixed to a printed circuit board (PCB), etc., and can take a variety of forms. For example, the electronics can take the form of an integrated circuit (IC), such as an application-specific integrated circuit (ASIC), a microcontroller, and/or a processor. The electronics unit 104 may include sensor electronics that are configured to process sensor information, such as storing data, analyzing data streams, calibrating analyte sensor data, estimating analyte values, comparing estimated analyte values with time corresponding measured analyte values, analyzing a variation of estimated analyte values, etc. Examples of systems and methods for processing sensor analyte data are described in more detail herein and in U.S. Patent Nos. 6,931,327, 7,310,544 and in U.S. Patent Application Publication Nos. 2005/0043598, 2007/0032706, 2007/0016381, 2008/0033254, 2005/0203360, 2005/0154271, 2005/0192557, 2006/0222566, 2007/0203966 and 2007/0208245, each of which is incorporated herein by reference in its entirety.

One or more repeaters, receivers and/or display devices, such as a key fob repeater 110, a medical device receiver 111, a smartphone 112, a portable or tablet computer 113, etc. are operatively linked to the electronics unit 104. The repeaters, receivers and/or display devices receive data from the electronics unit 104, which is also referred to as the transmitter and/or sensor electronics body herein. In some embodiments the repeaters, receivers and/or display devices transmit data to the electronics unit 104. For example, the sensor data can be transmitted from the sensor electronics unit 104 to one or more of the key fob repeater 110, the medical device receiver 111, the smartphone 112, the portable or tablet computer 113, etc. Also, in some embodiments the repeaters, receivers and/or display devices may transmit data to one another through a wireless connection or a wired connection.

In various embodiments, a display device includes an input module with a quartz crystal operably connected to a radio-frequency (RF) transceiver (not shown) that together function to transmit, receive and synchronize data streams from the electronics unit 104 and/or a continuous glucose monitor (CGM). However, the input module can be configured in any manner that is capable of receiving data from the electronics unit 104/CGM. Once the data stream is received, the input module sends it to a processor that processes the data stream, such as described in more detail below. The processor may be internal or external to the display device. For example, the input module may send some or all of the data to a remote processor, such as a processor in the cloud (described further below). The remote processor may then send the processed data back to the input module, or store it remotely. The processor is the central control unit that performs the processing, such as storing data, analyzing data streams, calibrating analyte sensor data, estimating analyte values, comparing estimated analyte values with time corresponding measured analyte values, analyzing a variation of estimated analyte values, downloading data, and controlling the user interface by providing analyte values, prompts, messages, warnings, alarms, etc. The processor includes hardware that performs the processing described herein. Storage provides permanent or semi-permanent storage of data, storing data such as a sensor ID, a receiver ID, and programming to process data streams (for example, programming for performing estimation and other algorithms described elsewhere herein). Random access memory (RAM) stores the system's cache memory and is used in data processing. An output module, which may be integral with and/or operatively connected with the processor, includes programming for generating output based on the data received from the electronics unit 104/CGM (and any processing incurred in the processor).

In some embodiments, analyte values are displayed on a display device. In some embodiments, prompts or messages can be displayed on the display device to convey information to the user, such as reference outlier values, requests for reference analyte values, therapy recommendations, deviation of the measured analyte values from the estimated analyte values, etc. Additionally, prompts can be displayed to guide the user through calibration or troubleshooting of the calibration.

Additionally, data output from the output module can provide wired or wireless, one- or two-way communication between the receiver and an external device. The external device can be any device that interfaces or communicates with the receiver. In some embodiments, the external device is a computer, and the receiver is able to download current and/or historical data for retrospective analysis by a physician, for example. In some embodiments, the external device is a modem, and the receiver is able to send alerts, warnings, emergency messages, etc., via telecommunication lines to another party, such as a doctor and/or a family member. In some embodiments, the external device is an insulin pen or insulin pump, and the receiver is able to communicate therapy recommendations, such as an insulin amount and a time to the insulin pen or insulin pump. The external device can include other technology or medical devices, for example pacemakers, implanted analyte sensor patches, other infusion devices, telemetry devices, etc. The receiver may communicate with the external device, and/or any number of additional external devices, via any suitable communication protocol, including radio frequency (RF), Bluetooth, universal serial bus (USB), any of the wireless local area network (WLAN) communication standards, including the IEEE 802.11, 802.15, 802.20, 802.22 and other 802 communication protocols, ZigBee, wireless (e.g., cellular) telecommunication, paging network communication, magnetic induction, satellite data communication, GPRS, ANT, and/or a proprietary communication protocol.

### Definitions

As a preliminary note, terms such as "application," "component," "module," "system," etc., as used herein, are used interchangeably and are intended to refer to any computer-related entity, such as hardware, firmware, software, or any combination thereof. For example, a component may be, but is not limited to being, a process running on a processor, a processor, an object, an executable, a thread of execution, a program, and/or a computing device.

By way of illustration, both an application running on a computing device and the computing device may be a module. One or more modules may reside within a process and/or thread of execution, and a module may be localized on one computing device and/or distributed between two or more computing devices. Also, applications may be executed from various non-transitory computer readable media having various data structures stored thereon. Components may communicate via local and/or remote processes such as in accordance with a signal having one or more data packets (e.g., data from one component interacting with another component in a local system, distributed system, and/or across a network such as the Internet with other systems via the signal).

The systems and processes described below are applicable and useful in a cloud computing environment. Cloud computing pertains to computing capability that provides an abstraction between the computing resource and its underlying technical architecture (e.g., servers, storage, networks), enabling convenient, on-demand network access to a shared pool of configurable computing resources that can be rapidly provisioned and released with minimal management effort or service provider interaction. The term "cloud" is intended to include the Internet, and cloud computing allows shared resources, for example, software and information, to be available on-demand.

Typical cloud computing providers deliver common business applications online, which are accessed from another web service or software like a web browser, while the software and data are stored remotely on servers. The cloud computing architecture uses a layered approach for providing application services. A first layer is an application layer that is executed at client computers. In this example, the application allows a client to access storage via a cloud. After the application layer is a cloud platform and cloud infrastructure, followed by a server layer that includes hardware and computer software designed for cloud-specific services.

### System

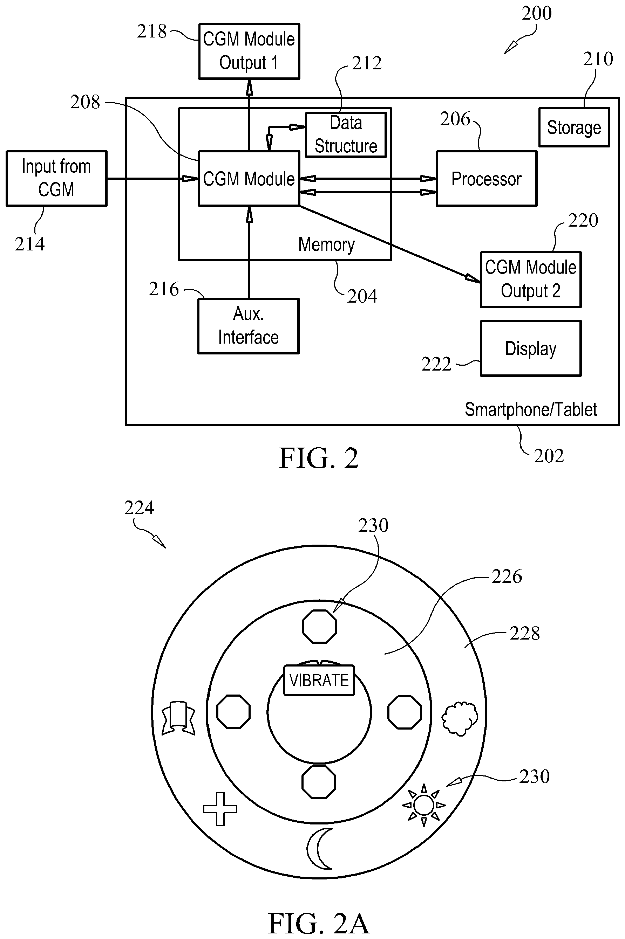

Figure 2 is a functional block diagram of one embodiment of a system 200 for leveraging smartphone 202 features in continuous glucose monitoring, according to the present embodiments. The system 200 includes a smartphone 202, or tablet computing device, or other device. The term smartphone 202 is used herein for convenience to illustrate the described features. Thus, it should be understood that any type of computing device capable of receiving one or more inputs and producing an output can be used in place of smartphone 202 if suitable. Accordingly, the present embodiments should not be interpreted as limited to any type of hardware.

The smartphone 202 includes a memory 204 and a processor 206. The memory 204 provides the processor 206 access to data and program information that is stored in the memory 204 at execution time. Typically, the memory 204 includes random access memory (RAM) circuits, read-only memory (ROM), flash memory, etc., or a combination of such devices. The processor 206 may be, or may include, one or more programmable general-purpose or special-purpose microprocessors 206, digital signal processors 206 (DSPs), programmable controllers, application specific integrated circuits (ASICs), programmable logic devices (PLDs), etc., or a combination of such hardware-based devices.

In accordance with the present embodiments, the processor 206 executes a continuous glucose monitoring (CGM) module 208 out of the memory 204. As used herein, the term continuous glucose monitoring module or CGM module should be construed broadly to include not just the module itself, but also integrations with other diabetes management devices, including insulin delivery therapies such as insulin pumps, insulin pens, or other drugs useful for the treatment of diabetes. In other words, the CGM module may perform other functions besides monitoring blood glucose. It could, for example, determine that a user's blood glucose level is high, and then transmit a signal to the user's insulin pump to administer a quantity of insulin to bring the user's blood glucose level down.

A software and/or firmware component of the CGM module 208 is stored in storage 210 available to the smartphone 202, and loaded into the memory 204 at execution time. The storage 210 may be any non-transitory computer readable media including, but not limited to, a hard disk, EEPROM (electrically erasable programmable read only memory), a memory stick, or any other storage device type. The memory 204 may contain one or more data structures 212 that the CGM module 208 accesses during execution. For example, the CGM module 208 may receive an input and store the input as an input parameter in a data structure 212 in the memory 204 for later processing.

In certain embodiments, the CGM module 208 may be embodied as downloadable software that a user may download from a remote server through a wired or wireless connection. For example, the user may access the server using an application already installed on the user's smartphone. The user may then download and install the CGM module 208 with the aid of the application. The user may then configure the CGM module 208. For example, the configuration may include setting the user's personal preferences and/or settings, such as contacts, events, modes, profiles, etc. The configuration may be done manually, such as by selecting various options from menus, or automatically. In automatic configuration, the CGM module 208 reads the user's preferences and/or settings that are stored on the smartphone. The CGM module 208 would first discover what other applications are installed on the smartphone, and then access those applications' data stored in the smartphone's storage and/or remote storage accessible by the smartphone 202 to initially populate the CGM module 208 during set up.

One embodiment of manually configuring the user's personal preferences and/or settings may include a profile wheel, such as the one illustrated in Figure 2A. The profile wheel 224 is a graphical user interface, which may be displayed on the smartphone's display 222 when the user inputs a command to configure personal preferences and/or settings. The profile wheel 224 includes an inner wheel 226 and an outer wheel 228. Both wheels 226, 228 include icons 230 that the user can select to adjust preferences and/or settings. The inner wheel 226 sets the alarm type, such as silent, vibrate, loud, etc., while the outer wheel 228 sets the profile (day, night, meeting, exercise, etc.). By clicking on a profile icon 230, the user can set up the active alarms within the profile, the sounds, as well as separate thresholds for each alarm.

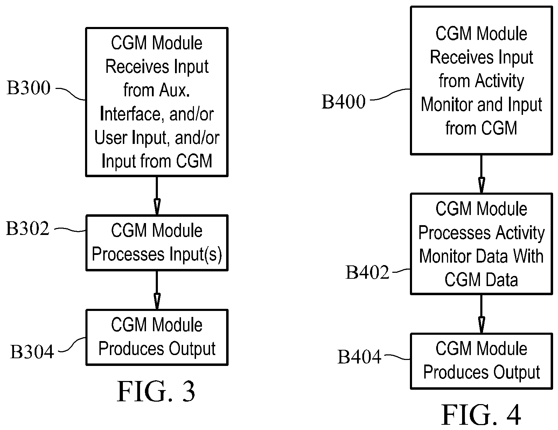

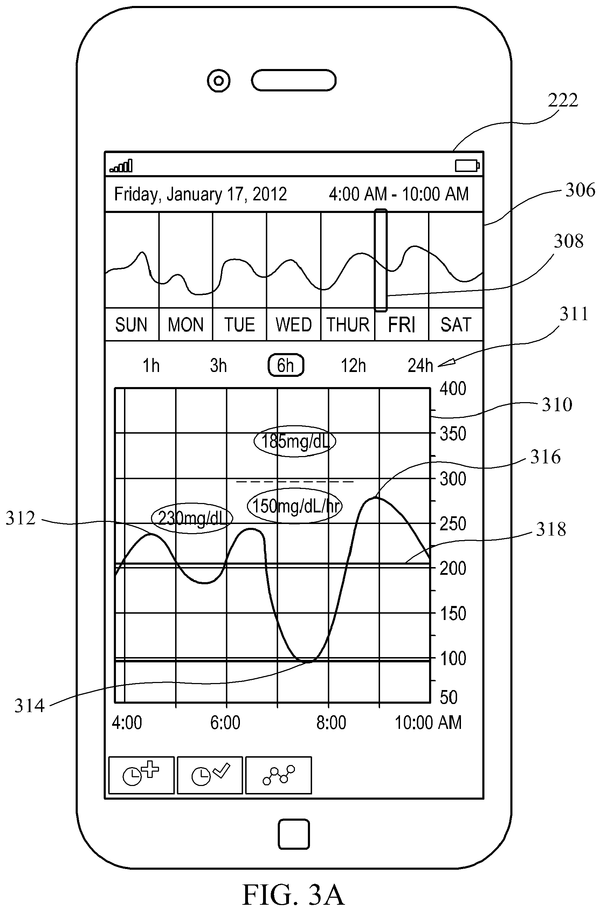

Figure 3 is a flowchart illustrating one embodiment of a process executed by the CGM module 208. With reference to Figures 2 and 3, during execution, the CGM module 208 receives at least one input 214 at block B300. A first input may be from the CGM, which may comprise a current estimated glucose value (EGV) for the user. A second input may be user input. A third input may be from an auxiliary interface 216. The auxiliary interface 216 may be any of hardware, software, firmware, or a combination of any of these, and may comprise anything that may be combined with EGV data and processed to produce an output that can provide the user with information that can help him or her make more informed decisions about how to manage his or her blood glucose. For example, the auxiliary interface 216 may be a sensor, which may be internal or external to the smartphone 202, or may be an application executed by the smartphone 202. Examples for the auxiliary interface 216 are described below with respect to specific embodiments.

The CGM module 208 processes the inputs at block B302 in conjunction with the processor 206 to produce one or more outputs 218, 220 at block B304. The output 218 may be to a device or receiver external to the smartphone 202 (CGM Module Output 1, 218), or to a device internal to the smartphone 202 (CGM Module Output 2, 220), such as to a display 222 or storage 210. For example, the output may be to the user's car, to another smartphone, to 911 emergency services, projected on a wall, to a social network application (to monitor friends' BG), a voice while exercising, to the cloud, etc. The output may be to a trend graph 310 displayed on the smartphone's display 222, as shown in the example illustrated in Figure 3A. Additional examples of outputs from the CGM module 208 include data that is stored locally or remotely for retrospective analysis, changing an operational mode of the smartphone 202, to schedule a time to eat, or to obtain a reference glucose value at a certain time, or to inject a certain dose of insulin at a certain time, a recommendation, a pattern, a reminder, a message, an immediate alert, or an output to a timekeeping/scheduling module to schedule an event for a later time, data written to a database for use in making future determinations, text shown in the smartphone's display 222, and/or a voice response delivered through one or more speakers of the smartphone 202, to an alert module with a notification to change its setting, turning on/off and/or changing timing of certain algorithm features and/or alarm settings, etc. Additional examples of outputs from the CGM module 208, and how they may be used, are described below with respect to specific embodiments.

Correlative algorithms and/or pattern recognition algorithms may be used to process the various inputs and to provide outputs. The algorithms may correlate and/or identify relationships between the various inputs (e.g., EGV data and one or more auxiliary input(s)). The auxiliary input may be an occurrence of an action or condition, but can be a non-occurrence of an action or condition.

In various embodiments, the auxiliary interface 216 may comprise a sensor that senses any of sleep/brain waves, heart rate, blood pressure, gait, weight, eye patterns, breathalyzer, skin temperature, sweat, blood oxygen, retina, optical pressure, EKG, lie detector, respiration, girth, ketone, etc. Some analytes that may be useful to measure using auxiliary analyte sensors include ketone bodies (e.g., acetone, acetoacetic acid and beta hydroxybutyric acid, lactate, etc.), glucagon, acetyl co a, triglycerides, fatty acids, intermediaries in the citric acid cycle, choline, insulin, cortisol, testosterone, etc. The foregoing lists are not exhaustive. The auxiliary interface 216 may comprise a sensor that senses anything, whether listed above or not. Furthermore, the auxiliary interface 216 need not be a sensor, but may instead be another type of device, and, again, may be any of hardware, software, firmware, or a combination of any of these.

### Auxiliary Interface 216: Activity Monitor

One example of an auxiliary interface 216 that can be used in accordance with the present embodiments is an activity monitor. As used herein the term activity monitor should be construed broadly to include both user input and any device and/or instructions capable of receiving as an input characteristics of user motion, or lack of motion, processing the input, and producing an output that is indicative of the user's current physical activity. Examples of types of activity monitors and/or data associated with activities include, without limitation, an accelerometer, a pedometer, a gyroscope, a heart rate monitor, a location monitor, such as a GPS, personal fitness tracker, a heat flux sensor, skin conductivity sensor, temperature sensor, calories burned, steps taken, distance travelled, physical activity duration, physical activity intensity, time till sleep, number of times awakened during sleep, actual sleep time, motion sensor, 3-dimensional motion sensor, skin temperature sensor, skin perspiration sensor, air temperature sensor, physical activity duration and intensity, distance travelled sensors that measure analytes as indicators of certain types of activity (e.g., oxygen, carbon dioxide, lactate, testosterone, cortisol, glucagon, glycogen, insulin, starch, free fatty acid, triglycerides, monoglycerides, glycerol, pyruvate, lipids, other carbohydrates, ketone bodies, choline) and combinations thereof.

In certain embodiments, the CGM module 208 provides a means for indicating what impact, if any, a user's physical activity may have on his or her blood glucose. For example, high levels of activity can be indicative of a greater likelihood of hypoglycemic events and/or a high level of adrenaline can be indicative of an increase in blood glucose levels. Thus, certain of the present embodiments leverage capabilities of smartphones 202 that relate to detecting physical activity, or lack thereof. Sensed characteristics of motion are then combined with CGM data to produce an output that provides a user with more information than a CGM reading alone would, so that the user is better able to manage his or her blood glucose going forward.

In various of the present embodiments, outputs from the CGM module 208, when used in conjunction with the activity monitor, may be used to inform: graphs, profiles, alarm algorithms, insulin algorithms, interaction logs, a closed loop algorithm, providing a warning to the user of low blood glucose while engaged in certain activities, such as driving, activity monitoring, providing a warning to the user that sensor data may not be accurate because of surroundings or activity, such as when flying, etc.

In various embodiments, with reference to Figure 4, the CGM module 208 may be used in conjunction with the activity monitor to correlate how exercise impacts blood glucose levels. In these embodiments, the activity monitor may comprise a heart rate monitor, for example. The CGM module 208 receives as inputs at block B400 the user's heart rate from the activity monitor and the user's current EGV from a CGM. The CGM module 208 may receive substantially continuous EGV's from the CGM. As used herein, the terms "substantially continuous," "continuously," etc., may refer to a data stream of individual measurements taken at time-spaced intervals, which may range from fractions of a second up to, for example, 1, 2, or 5 minutes or more. The CGM module 208 processes the inputs at block B402 and produces an output at block B404. The processing may be applying one or more algorithms to two streams of input, for example, algorithms useful for correlating data and/or recognizing patterns. For example, the processing may be correlating the data to match EGV's with substantially time corresponding values of heart rate. The output may be, for example, data that is stored locally or remotely for retrospective analysis. The data may include a series of matched data pairs wherein the user's EGV at chronological points in time is matched with substantially time corresponding values of heart rate. The matched data pairs may, for example, be displayed on a graph, which can be displayed on the smartphone display 222 or projected onto a screen or wall using a projector built into the smartphone 202. The user is thus able to correlate what impact, if any, exercise has on his or her blood glucose. Additionally or alternatively, algorithms process the activity information with the CGM information to provide a recommendation, such as recommendations for diet, exercise, therapy, supplements, etc. The recommendations can be performed in real time on the smart device and/or retrospectively and accessed through the cloud. In certain embodiments, an additional input to the CGM module may be a type of exercise that the user is engaged in, such as cycling, running, swimming, etc. This additional information may inform how different types of exercise affect blood glucose.

In various other embodiments, again with reference to Figure 4, the CGM module 208 may be used in conjunction with the activity monitor to detect when the user is not moving, and is thus likely to be sleeping. In these embodiments, the activity monitor may comprise a gyroscope and/or an accelerometer, for example. The activity monitor detects that the user is positioned horizontally and/or not moving, and sends a signal to the CGM module 208 indicating that the user is likely to be sleeping. At block B400, the CGM module 208 receives the signal from the activity monitor and one or more inputs from a CGM, which are current EGV's. The CGM module 208 processes the inputs at block B402 and produces an output at block B404. The processing may be applying one or more algorithms to two streams of input, for example, algorithms useful for correlating data and/or recognizing patterns. For example, the processing may be tracking the user's EGV over time as he or she sleeps. The output may be, for example, data that is stored locally or remotely for retrospective analysis. The data may include a plot of the user's EGV over time as he or she sleeps. The data may be analyzed retrospectively to better understand optimal basal rates and/or nighttime trends of glucose patterns. Additionally, results of the analysis may be used to modify other aspects of the CGM module 208 and/or smart device, such as, for example, changing timing, modes, thresholds or other settings of sounds, alarms, alerts, rings, etc. In one example, a predetermined level of activity can be used to determine the timing of a calibration schedule for the CGM module 208. As another example, a predetermined level of activity (or lack thereof) can be used to switch a timing schedule (or turn on or off) a low glucose suspend feature (e.g. configured to suspend a basal or bolus delivery of glucose in an insulin delivery device responsive to sensor data meeting a predetermined criterion) and/or hypoglycemia/hyperglycemia minimizer algorithm (e.g. an algorithm configured to control a user's blood glucose to a target range using an automatic insulin delivery device). As another example, a predetermined pattern of activity could trigger a change in predetermined alarm settings from a predefined exercise setting to a different predefined resting setting.

In an alternative embodiment, the CGM module 208 may be alerted by user input to the fact that the user is exercising. For example, an interface of the CGM module 208 may include an exercise start/stop button. When the user begins exercising, he or she presses the exercise start button. EGV's input to the CGM module 208 are then processed and outputs produced as described above with respect to the previous embodiments.

### Auxiliary Interface 216: Timekeeping/Scheduling Module

Another example of an auxiliary interface 216 that can be used in accordance with the present embodiments is a timekeeping/scheduling module. As used herein the term timekeeping/scheduling module should be construed broadly to include any device and/or instructions capable of receiving an input relating to time and/or scheduling, processing the input, and producing an output relating to time and/or scheduling. Examples include, without limitation, a clock, a calendar, etc. A calendar may store data about past, present and future events relating to the user. This data could be stored locally on the smartphone 202, or remotely, such as in a cloud computing system 200.

In certain embodiments, the CGM module 208 is integrated with other devices carried by a user, thereby eliminating inconsistencies and lack of continuity between data held by the different devices. Thus, certain of the present embodiments leverage capabilities of smartphones 202 that relate to timekeeping and scheduling. Inputs from the timekeeping/scheduling module may then be combined with CGM data to produce an output that provides a user with more information than a CGM reading alone would, so that the user is better able to manage his or her blood glucose going forward. Additionally, by integrating the scheduling and timing features already existing on a smartphone with various important scheduling and timing features associated with CGM (and insulin delivery therapies), a user's disease management can be synchronized to fit into the daily life of the user in a manner that encourages compliance to best practices for their disease management.

In various embodiments, the CGM module 208 may be used in conjunction with the timekeeping/scheduling module to synchronize the CGM module 208 with the timekeeping/scheduling module so that an operational mode of the smartphone 202 can be automatically changed in anticipation of scheduled activities. For example, if the user has an event scheduled where it would be embarrassing for his or her phone to ring, such as a business meeting, the smartphone 202 may automatically enter silent mode (or vibrate mode) for the expected duration of the meeting. Thus, some or all CGM alerts while in the silent mode may only be vibratory - not auditory. In some embodiments, less severe events (e.g., mild hyperglycemia) may cause only a vibratory alert, whereas more sever events (e.g. sever hypoglycemia) still cause an auditory alert. In some embodiments, a user can modify events that have auditory alerts regardless of whether the smartphone 202 is in silent mode. In these embodiments, the timekeeping/scheduling module receives as an input an indication of a scheduled event, processes that input and provides an output to the CGM module 208. The CGM module 208 receives the output from the timekeeping/scheduling module, processes the input and produces an output. The output may be, for example, changing an operational mode of the smartphone 202.

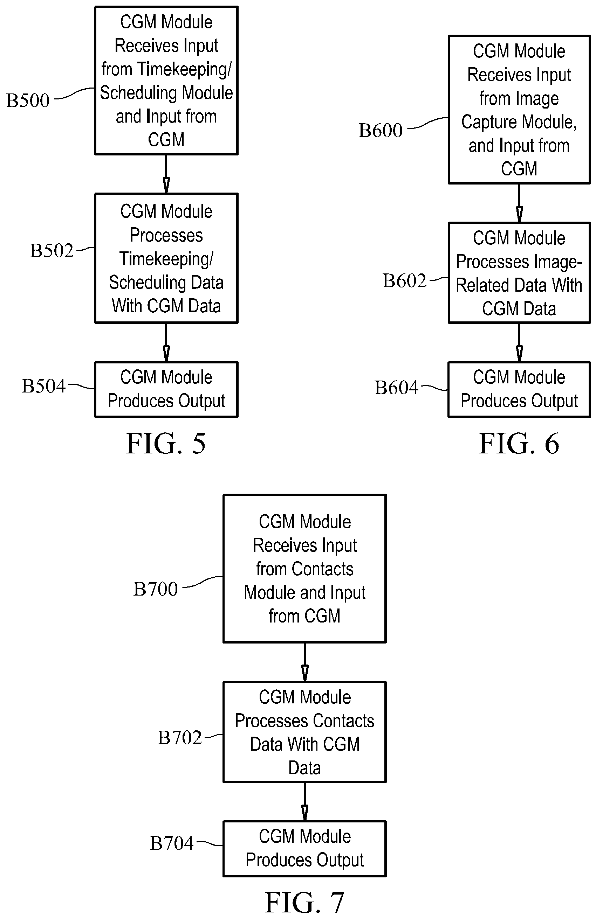

In various other embodiments, again with reference to Figure 5, the CGM module 208 may be used in conjunction with the timekeeping/scheduling module to schedule events based on blood glucose data. For example, at block B500 the CGM module 208 may receive an input from a CGM indicating that the user's current EGV is dropping, and an input from the timekeeping/scheduling module of a current day and time. The CGM module 208 processes the inputs at block B502 and produces an output at block B504. The output may be, for example, to schedule a time to eat, or to obtain a reference glucose value at a certain time, or to inject a certain dose of insulin at a certain time. The user is thus less likely to experience a hypoglycemic event. Alternatively, the output may be, for example, to schedule a time to insert a new analyte sensor, or to exercise.

In various other embodiments, the CGM module 208 may be used in conjunction with the timekeeping/scheduling module to change an operational mode of the smartphone 202 and/or the CGM module 208. For example, the CGM module 208, in combination with other input, may determine that CGM should go into a do not disturb mode. The CGM module 208 thus produces an output to the smartphone 202 to go into the same mode. Alternatively, the smartphone 202 goes into a do not disturb mode, which may result from the user changing a setting of the smartphone 202 to go into the do not disturb mode. After the smartphone 202 goes into the do not disturb mode, the CGM module 208 automatically synchronizes with the smartphone's mode and changes settings to match the smartphone 202. Additionally, the CGM module 208 may be configured to read the smartphone's modes at start up and ask the user to determine how they want the CGM module 208 to act during each mode (e.g., what types of alarm sounds/vibrations, what thresholds for alert, what delivery options for alerts, what mode for the insulin pump (e.g., turn low glucose suspend off), etc. Another output that the CGM module 208 may generate may be to send information to the user's insulin pump including CGM data and the additional information discovered through the integration with the smartphone 202 (e.g., activity levels).

In various other embodiments, again with reference to Figure 5, the CGM module 208 may be used in conjunction with both the timekeeping/scheduling module and the activity monitor. For example, at block B500 the CGM module 208 may receive one or more of an input from a GPS locator indicating the user's current location, an input from the timekeeping/scheduling module indicating the current day and time, and an input from a CGM indicating the user's current EGV trend. The CGM module 208 processes the inputs at block B502 and produces an output at block B504. The output may be, for example, a recommendation, a pattern, a reminder, a message, etc. The output may be an immediate alert, or an output to the timekeeping/scheduling module to schedule an event for a later time. The recommendation may be, for example, anything that may be more difficult to do while away from home, that must be done while at home, that may need more lead time when away from home, and/or something that should be done differently when away from a particular location, such as a therapy, to schedule a doctor's appointment, to call a caretaker, to send data to a caretaker, to send data to a doctor, to eat a meal, to exercise, to replace a sensor, to calibrate a sensor, to check blood glucose, to order pump supplies, to change the insulin pump insertion site, to run reports, to bring an extra sensor, to upload or synchronize data to a cloud computing system. The recommendation may be provided to the user, a caretaker, a parent, a guardian, or a healthcare professional. The recommendation may be provided via screen prompt, text message, e-mail message, or a post to a social network. For example, if a blood glucose is low and a location is remote (away from restaurants or distant from home), a prompt to find food may appear on the smartphone's display 222.

In various other embodiments, again with reference to Figure 5, the CGM module 208 may be used in conjunction with the timekeeping/scheduling module to prompt the user based on past events. For example, at block B500 the CGM module 208 may receive an input from the timekeeping/scheduling module indicating when the user last ate, when the user last exercised, when the user last calibrated his or her sensor, when the user last changed his or her sensor, last slept, last saw a doctor, or when the user last checked his or her blood glucose. The CGM module 208 processes the input at block B502, for example an analysis of how past events (inputs) from the timekeeping/scheduling module correlated with the user's blood glucose, and produces an output at block B504, which may be, for example, a prompt to eat, a prompt to exercise, or a prompt to calibrate or change the sensor, a recommendation of how to change or optimize the timing, duration and/or type of future events, or a prompt to check blood glucose. The analysis can be based on the user's data alone and/or can be compared to other user's data, for example using comparative analysis or pattern recognition algorithms. Alternatively, the CGM module 208 may receive an input from a CGM. The CGM module 208 processes the input and produces an output, which may be, for example, a prompt to eat, to schedule a doctor's appointment, to send data to a caretaker, or to display 222 in-case-of-emergency contact information. In the foregoing examples, rather than a prompt, which may be delivered to the user immediately in the form of an audible tone and/or a screen prompt, the output may instead be to the timekeeping/scheduling module to schedule an event for a later date/time.

In various other embodiments, again with reference to Figure 5, the CGM module 208 may be used in conjunction with the timekeeping/scheduling module to prompt the user based on future events. For example, at block B500 the CGM module 208 may receive an input from the timekeeping/scheduling module indicating that an event is drawing near. For example, a doctor's appointment is drawing near and the output recommends the user run a report of the last 90 days of CGM data. As another example, the sensor is on its last day and a prompt to insert a new sensor may be provided. The CGM module 208 may also receive an input from a CGM of the user's EVG. The CGM module 208 processes the input(s) at block B502 and generates an output at block B504. The output may be, for example, a recommendation. For example, where the CGM module 208 detects a problem with the sensor 102, a prompt to upload data to the manufacturer could be useful in resolving the problem.

In various other embodiments, the CGM module 208 may change the user's alert profile automatically at certain times of the day. For example, the user may have a nighttime alert profile that is automatically activated at a specified time of day, and deactivated at another specified time of day. The nighttime profile may change, for example, alert levels, sounds, etc. according to the user's preferences, which may be programmable. In some embodiments, the settings associated with the timekeeping/scheduling module and/or a settings module of the smartphone activate or de-activate an aspect of closed loop or semi-closed loop control of an insulin pump or CGM algorithm. For example, during a night time profile or do not disturb profile, the CGM module can be configured to activate a low glucose suspend feature of a semi-closed loop algorithm. In these embodiments, the CGM module 208 receives as an input a current time of day from the timekeeping/scheduling module. The CGM module 208 processes the input by comparing it to a stored time of day when the nighttime profile is to be activated. If there is a match, then the CGM module 208 produces as an output a signal to an alert module to activate the nighttime profile. A similar process commences when the input time of day to the CGM module 208 matches a stored time of day when the nighttime profile is to be deactivated. In some embodiments, the settings of the CGM module 208 are configured to be synchronized with the settings of the smartphone, either by default at set up or when the CGM module 208 is in use, whereby a change in one or more of the smartphone settings (e.g., profiles or modes) triggers a change in one or more of the CGM module 208 settings. Alternatively, one or more of the CGM module 208 settings (e.g., profiles or modes) are different from the settings (profiles or modes) of the smartphone.

In various other embodiments, the CGM module 208 may provide a countdown to monitor the time until a next sensor calibration is due. For example, the CGM module 208 may receive an input from the user and/or the CGM that he or she has just calibrated the sensor 102. The CGM module 208 may process the input to determine when the next calibration is due based on the CGM's calibration scheme (e.g., based on sensor insertion, time of last calibration, quality of calibration, amount of measured sensor drift, etc.), and produce an output to the timekeeping/scheduling module to begin a countdown. The duration of the countdown may be based on a preprogrammed value in the CGM module 208 of a recommended duration between sensor calibrations. The countdown may be displayed on the smartphone's display 222 so that the user can monitor the time to the next sensor calibration.

In an alternative embodiment, the countdown described above could be programmed by the user, rather than a preprogrammed duration set by the CGM module 208. This functionality enables the user to allow himself or herself a time buffer. An example of when this functionality is useful is when the user wants to be alerted of his or her glucose half an hour after a meal or half an hour after an insulin bolus, in the event that the user wants to maintain a certain glucose level after a meal or ensure that his or her glucose is at a certain level before commencing or continuing exercise.

### Auxiliary Interface 216: Image Capture Module

Another example of an auxiliary interface 216 that can be used in accordance with the present embodiments is an image capture module. As used herein the term image capture module should be construed broadly to include any device and/or instructions capable of receiving as an input image information, processing the image information, and producing an output based on the image. Examples include, without limitation, a digital camera, a bar code scanner, a Quick Response Code (QR) reader, etc.

In certain embodiments, the CGM module 208, when performing a retrospective analysis of EGV's, enables the user to determine where he or she was at the time a given EGV was recorded, or what he or she was doing at that time, or what food(s) he or she may have consumed at that time. Thus, certain of the present embodiments leverage capabilities of smartphones 202 that relate to image capture so that the user can take photos of places visited, activities engaged in, food consumed, etc., and those photos can be associated with substantially time corresponding EGV's to provide insight into how certain places/activities/foods/etc. affect blood glucose for real time or retrospective display and/or analysis.

In various embodiments, with reference to Figure 6, the CGM module 208 may be used in conjunction with the image capture module to take photos of places visited, activities engaged in, food consumed, etc. Those photos are then associated with substantially time corresponding EGV's received from a CGM. In these embodiments, the image capture module receives as an input image information pertaining to a photo taken by a digital camera, which may be built into the smartphone 202. The image capture module processes the image information and provides an output to the CGM module 208. At block, B600, the CGM module 208 receives the output from the image capture module and at least one input from a CGM, which is a current EGV. The CGM module 208 may continue to receive additional inputs of current EGV's from the CGM for a predetermined amount of time after receiving the input from the image capture module, so that further data can be output about the effect of the place visited/activity engaged in/food consumed/etc. on EGV over the predetermined amount of time. The CGM module 208 processes the inputs at block B602. For example, the data may be stored in a first location and referenced for algorithmic processing, where the algorithm runs a correlation analysis or pattern recognition. Alternatively, the processing may comprise associating the image information with the current EGV/EGV's over the predetermined amount of time. The CGM module 208 then produces an output at block B604, which may be, for example, data that is stored locally or remotely for retrospective analysis. The data may include one or more images that are matched to substantially time corresponding EGV's or to EGV's over the predetermined amount of time. The user is thus able to correlate what impact, if any, the place visited/activity engaged in/food consumed/etc. had on his or her blood glucose.

In various other embodiments, again with reference to Figure 6, the CGM module 208 may be used in conjunction with the image capture module to take photos of food that is about to be consumed in order to estimate how many carbohydrates are in the food so that the user can be shown what effect the food is likely to have on his or her blood glucose and/or to estimate a dosage of insulin that should be injected after the food is consumed. In these embodiments, the image capture module receives as an input image information pertaining to a photo taken by a digital camera, which may be built into the smartphone 202. The image capture module processes the image information and provides an output to the CGM module 208. At block B600 the CGM module 208 receives the output from the image capture module and at least one input from a CGM, which is a current EGV. The CGM module 208 processes the inputs at block B602 in one of several different ways. The processing may be applying one or more algorithms to two inputs, for example, algorithms useful for analyzing images and/or correlating data. For example, if the output from the image capture module is to be an estimate of the carbohydrates contained in the food, the processing may comprise analyzing the image of the food and obtaining carbohydrate information about the food from a database, which may be stored locally or remotely. In these embodiments, the CGM module 208 may not need to receive an input from the CGM. If the output is to be communicating to the user what effect the food is likely to have on his or her blood glucose, the processing may comprise making that determination based upon the user's current blood glucose, which is known from the CGM, which may in turn comprise analyzing the composition of the food and obtaining carbohydrate information about the food from a database, which may be stored locally or remotely. If the output is to be an estimate of a dosage of insulin that should be injected after the food is consumed, the processing may comprise determining what effect the food is likely to have on the user's current glucose level, which may be known from the CGM, and determining what amount of insulin would be appropriate to counteract the expected rise in blood glucose. The CGM module 208 then produces an output at block B604, which may be, for example, an output to a display 222 of the smartphone 202 that shows the user what effect (graphically, textually and/or predictively) consuming the food will have on his or her blood glucose, providing an estimated insulin dosage, providing a recommended therapy, providing a recommendation not to eat the food or to eat only a portion of it, etc. The user is thus able to make informed decisions about whether to eat the food, eat only half of it, etc. And, if all the food is consumed, the user is able to more accurately gauge how much insulin he or she should inject. Other examples of outputs include recommendations of how much of a particular food (e.g., from an image) should be consumed, (e.g., by recommending the user eat only half of the plate of food), or a resulting prediction of how the amount of food (on the plate) consumed might affect the user's glucose levels.

In alternative embodiments, the input to the CGM module 208 regarding food consumed or to be consumed may be user input in addition or rather than information from an image capture module. For example, the user may input food information via a graphical user interface on the display 222 of the smartphone 202. The form of input may be typing descriptions of the food and/or selecting food choices from predefined onscreen menus. In some embodiments, the results of the captured image (e.g., estimated carbohydrates and/or fat) can be sent to an insulin determination module, for example, a closed loop type algorithm that informs an insulin pump of an amount of insulin to administer. In one example, the user takes a picture of the food on his or her plate, then selects the portion of the food to be (or that has been) consumed and enters that as the input for a bolus calculator, closed loop insulin algorithm, or semi-closed loop insulin algorithm.

In various other embodiments the CGM module 208 may be used in conjunction with the image capture module to input a reference value (e.g., a blood glucose value from a reference blood glucose finger-stick meter) to the CGM module 208. CGM's may require entry of a reference glucose value in order to calibrate and/or validate the sensor data. Additionally, a reference glucose value may be used to calculate or validate a therapy recommendation for the user. Unfortunately, the user may not accurately input the time stamp of the reference glucose value (for example, the time at which the reference glucose value was actually obtained) at the time of reference data input into the smartphone. Additionally, the accuracy of data entry may be subject to human error (for example, due to inconsistencies in reading or entering the reference glucose value from the meter). In contrast, using the image capture module enables the user to automatically and accurately obtain the reference glucose value and the time it was obtained from the reference glucose meter. Additionally, the process of obtaining reference data is simplified and made convenient using image capture module because of fewer loose parts (for example, cables, etc.) and less required data entry. Once the user has taken a picture of the reference value using the image capture module, the image is processed to determine the reference value therefrom, stamped with the time of the picture (or based on a prompt to validate the time stamp), and the value sent to the CGM module 208 for use in calibration of the CGM data, validation by the CGM module, input into an insulin therapy recommendation and/or other semi-closed loop system calculation.

In various other embodiments the CGM module 208 may be used in conjunction with the image capture module to pair a transmitter (sensor electronics 104) to the smartphone 202 or to initiate use of a new sensor 102. In these embodiments, the user takes a photo of a transmitter/sensor, or scans a barcode, such as a UPC barcode or matrix barcode, affixed to the transmitter/sensor. The photo could be taken with a digital camera, and the scans could be made with a UPC barcode reader or QR reader, any of which may be built into the smartphone 202. The image capture module then receives as an input image information pertaining to the photo or scan, processes the information and produces an output, which is passed to the CGM module 208. The CGM module 208 receives and processes the input from the image capture module. The processing may be applying one or more algorithms to the input, for example, algorithms useful for analyzing images. For example, the processing may comprise transmitting the input to a remote authentication module, which analyzes the transmission and sends back either an approval or denial. The CGM module 208 then produces an output, which may be, for example, an on-screen prompt that the transmitter has been associated or the sensor has been enabled.

With regard to pairing of the transmitter to the smartphone, pairing may be accomplished during a channel establishment process between the two devices. Establishing a channel may involve broadcasting a unique ID by one device and a search and acquisition of this ID by the other device. In general, the sensor electronics of the CGM device may send one or more message beacons that include the device ID and optionally other security means. The smartphone 202 may receive the transmission and determine whether to pair with the sensor electronics of the CGM device by checking for a match between the device ID in the received beacon and the device ID it is searching for. If the device ID does not match, the pairing process can end. If the device ID does match, a communication channel is established. The part of the communication process involved in establishing a communication channel may be handled by the sensor electronics in accordance with the protocols established for standardized communication and embedded therein. In some embodiments, the pairing process may be initiated by and/or assisted by the image capture module, wherein a user captures an image including a device ID (in any numeric or coded format), initiated by the user and/or requested by the CGM module 208, after which the CGM module 208 uses the device ID derived from the image to pair the transmitter with the smartphone and/or provide other information associated with the CGM device.

The CGM device may include a sensor system identifier, such as a series of alphanumeric characters (e.g., a series of 5, 6, 7 or 8 alphanumeric characters) printed, etched or otherwise affixed on a housing of the CGM device (sensor, transmitter or packaging associated therewith), or any other known identifier, such as a bar code or quick response (QR) code. The sensor system identifier may be used to generate both the device ID used in the master beacons to establish a channel and to generate the sensor security code used for additional security in the glucose monitoring system. To maintain good data security, the alphanumeric characters and the sensor security code need not be transmitted over a wireless communication channel at any time.

With regard to other information that may be captured by the image capture module, one or more codes may be provided in any readable format, whereby the CGM module 208 can derive sensor information therefrom, for example, sensor expiration, sensor lot information, sensor calibration information, duration of sensor session information, a license code to enable particular functionality, etc. The CGM module 208 may initiate a request for a code, in response to which the user may capture an image of the code, after which the CGM module 208 reads and interprets the code to obtain information useful for the function and/or control of the sensor and/or display and processing of the sensor data. Either of the sensor electronics or the smartphone 202 may be configured as the master or the slave depending on the protocols of the particular smartphone and/or CGM device configuration.

In various other embodiments the CGM module 208 may be used in conjunction with the image capture module to take photos of locations on the user's body. For example, the user may take a photo of a newly placed sensor and its location on the body, and the data obtained during that sensor session may be stored and associated with the location of the sensor to determine what locations on the body provide the best sensor data. The user could then leverage this information to determine where to place a new sensor by taking a photo of his or her body prior to sensor placement. In these embodiments, the image capture module receives as an input image information pertaining to a photo of a newly placed sensor on the body. The image capture module processes the information and produces an output, which is passed to the CGM module 208. The CGM module 208 receives and processes the input from the image capture module. The processing may be applying one or more algorithms to the input, for example, algorithms useful for analyzing images. For example, the processing may comprise associating the location of the sensor with all data obtained during that sensor session. The CGM module 208 then produces an output, which may be data written to a database for use in making future determinations about where to place new sensors to obtain good quality sensor data. Additionally or alternatively, the location and quality of sensor data information can be uploaded to a data repository (e.g., in the cloud), where analysis of correlations of sensor quality with sensor location (and other related information such as BMI, age, sex, etc.) can be performed to improve future recommendations for sensor insertion sites. The sensor insertion site information can be combined with infusion pump insertion site information in a similar manner. Additionally, feedback can be provided to the user as to where a next recommended insertion site might be based on historic information of use of various sites over time.

In another example, with each new insulin injection, the user may take a photo of the injection site. The photos may be stored in a database for use in making future determinations about where to inject insulin. In this example, the image capture module receives as an input image information pertaining to a photo of an insulin injection site on the body. The image capture module processes the information and produces an output, which is passed to the CGM module 208. The CGM module 208 receives and processes the input from the image capture module. The output may be data written to a database for use in making future determinations about where to inject insulin. As above, the location information can be combined with quality, accuracy, reliability, BMI, sex, age, sensor insertion location information, etc., to output information about recommended future sites and/or for general analysis of trends in a population of users.

### Auxiliary Interface 216: Contacts Module

Another example of an auxiliary interface 216 that can be used in accordance with the present embodiments is a contacts module. As used herein the term contacts module should be construed broadly to include any device and/or instructions capable storing, maintaining and/or processing information about one or more people or entities, such as personal contact information. An example of a contacts module includes an electronic directory of information about people that a user of the smartphone 202 may know, such as names, phone numbers, addresses, pictures, friend lists, social networks, Twitter account information, Facebook account information, diabetes blogs, etc.

In certain embodiments, the CGM module 208 provides a mechanism for communicating to bystanders what can be done to help a diabetic who is in distress due to, for example, a hypoglycemic event. Thus, certain of the present embodiments leverage capabilities of smartphones 202 that relate to personal contact information so that bystanders can better assist a diabetic who is experiencing a hypoglycemic event.

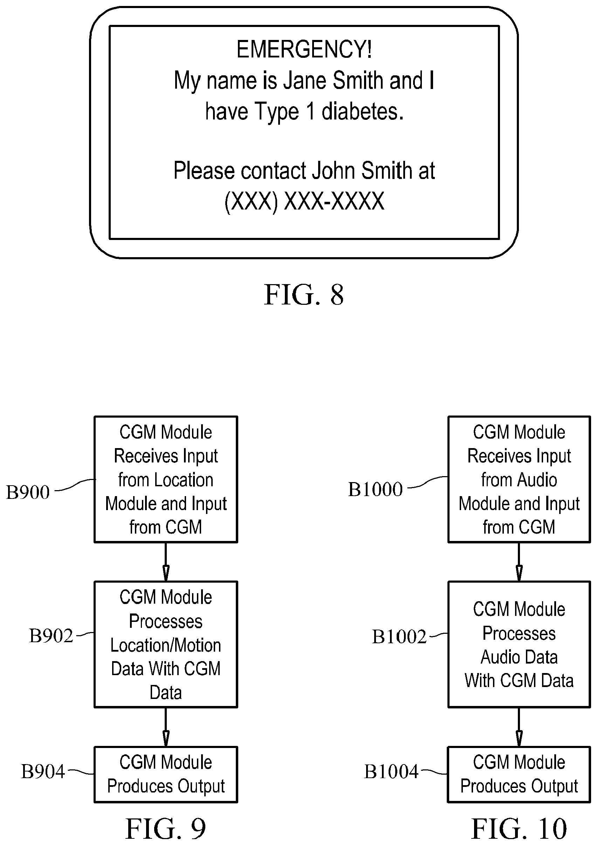

In various embodiments, with reference to Figure 7, the CGM module 208 may be used in conjunction with the contacts module to provide in case of emergency (ICE) information for bystanders during a hypoglycemic event. In these embodiments, at block B700 the CGM module 208 receives an input from a CGM, which is the user's current EGV. The CGM module 208 processes the input at block B702, which may comprise comparing it to a threshold value. If the EGV is lower than the threshold value, indicating that the user is experiencing a hypoglycemic event, the CGM module 208 may query the contact module to obtain ICE information for the user. The CGM module 208 may then output the information to the display 222 of the smartphone 202 at block B704, together with an audible alarm and/or visual indication, such as flashing the display 222 on and off. The output may also include a projection of a help signal from a projector built into the smartphone 202. The audible and/or visual alarm may alert a bystander that something is wrong, and the bystander can use the displayed information to help the diabetic, such as by summoning help. The ICE information displayed may be fully customizable so that the user can determine what ICE information will be displayed. Examples of ICE information that could be displayed include, with reference to Figure 8, the user's name, a statement that the user has diabetes and may require medical assistance, an emergency contact person with phone number, etc. The user may also program when the ICE information will be displayed, such as after a selected duration following a reading of <55 mg/dl alert, and for how long the ICE information will be displayed. For example, the normal sleep mode of the display 222 may be disabled during this time so that the ICE information is readily viewable by first responders.

In various other embodiments, the CGM module 208 may be used in conjunction with the contacts module to provide updates to a social network. In some embodiments, the user's location and/or other attributes associated with the user (such as type of diabetes, age, sex, demographic, etc.), the smartphone 202, and/or the CGM device can be used to find other people in the area, with similar attributes and/or using a similar CGM device, to recommend as a social connection. For example, the CGM module 208 and/or a social media site in concert with the CGM module 208 enables the user to select from options such as find other people with diabetes, or find other people with diabetes near me or find recommendation of diabetes-friendly restaurant in the area.

In some embodiments, the CGM module 208 enables a user to selectively upload or share information from their CGM module 208 electronically and/or via a social network site. Example information that could be shared includes a success metric, a current EGV value, a screen shot, an achievement, an award, a pattern trend graph, activity information, location information, and/or any other parameter described as a possible input into or output from the CGM module 208 elsewhere herein. For example, the CGM module 208 may have user selectable actions such as share EGV on Facebook, share EGV on Twitter, share screen via Facebook, Twitter, e-mail, MMS, send trend screen to printer, etc. Additionally, or alternatively, the CGM module 208 may enable a user to add preset and/or custom captions, or change a status with their shared information, such as check out my no hitter, which can be shared selectively (by a user or based on parameters) and/or automatically. In one example, a user can "like" a particular CGM from the CGM module 208 directly to a particular social site. In certain embodiments, when the user selects to share information, options may be shown on the display 222 that enable the user to select what to share and with whom. The user may predefine groups and or individuals to share information with. For example, the user may create a group of friends, and when the user chooses to share something he or she selects to share it with the predefined friends, and a notification is then sent to each person in the group. This functionality is useful, for example, for parents who want to monitor their child's blood glucose. The child can choose to share a BG value, and then select parents, or mom, or dad, and the BG value is then sent to the selected person(s).

In some embodiments, the CGM module 208, in concert with a social network, can be configured to allow users to compare EGV, trends, number of lows, etc. with friends or a group on the social network site (e.g., Facebook). In some embodiments, the CGM module 208, using CGM information from a plurality of users, compares one or more parameters to determine a comparison of data from a single person to the average (grouped by some similarity, for example). In some embodiments, the CGM module 208 calculates achievements, points, badges, or other rewards based on predetermined criteria (keeping blood glucose in a target range, use of CGM, etc.), which can be selectively or automatically posted to a social network site (e.g., Facebook). In some embodiments, when a user would like to share a learning from the CGM module 208, e.g., a picture of food and resulting EGV or trend graph, the CGM module 208 enables the user to selectively upload the information to a site. In this context a learning is an event or a first situation, and the effect, output, or trend resulting therefrom.

Additionally or alternatively, data from CGM users can be aggregated, whereby the CGM module 208 is configured to enable a user to query for current active CGM users, xx% of people using a CGM that are in range, other CGM users with a similar glucose as him or her (within a margin of error, such as 80 mg/dL ± 5). These queries can also be narrowed by geography, by doctor, age, gender, ethnicity, diabetes type, therapy type (pump, syringes, exenatide, metformin, etc.), etc.

In some embodiments, the CGM module 208 on a first smartphone communicates with another CGM module 208 on a second smartphone (e.g., via a social network site or other network connection) to allow CGM users to play or compete with others in a group (or with a particular friend) with one or more metrics (e.g., amount of time in target range, reduction of hypoglycemia, continuous use of CGM, etc.).

In some embodiments, the CGM module allows users to share problems or difficulties associated with their device and/or disease management by uploading screen shots, questions, data, etc. Further, the CGM module 208 may allow others to post solutions or answers to similar problems, which solutions or answers may be sent to the smartphone for local review by the sharer.

In some embodiments, the CGM module 208 comprises a database of doctors that treat patients using CGM (prescribe CGM), and which allows a user to find a CGM-prescribing doctor with a prescribed distance.

In various embodiments, the CGM module 208 enables a user to 'pin' (using a site such as Pinterest) a CGM screen shot, trend graph, etc. that results from cooking and consuming a recipe. For example, a recipe for eggs that also includes the CGM graph of what the recipe did to the user's glucose.

The following are some examples of activities that may be considered quantifiable achievements, and that could be used by the CGM module 208 to reward the user, and/or could be uploaded to share or compete with others: first sensor worn, one-month streak of continuous wear, two-month streak of continuous wear, x-month streak of continuous wear, one-year CGM anniversary, two-year CGM anniversary, x-year CGM anniversary, no-hitter day (e.g., no glucose values below a predetermined hypoglycemic threshold), no-hitter two-day streak, no-hitter x-day streak, quickly curbed a high (e.g., above hyperglycemic upper target for no more than 20 minutes, 40 minutes, 60 minutes, etc.), quickly corrected a low (below hypoglycemic lower target for no more than 20 minutes, 40 minutes, 60 minutes, etc.), fixed a problem area (when the pattern report indicates a pattern, then the pattern no longer appears), the fixed problem area could generate an alert, first upload (if not automatically uploaded), nth upload, shared CGM information with a friend (e.g. screenshot), posted CGM trend on a social media site, no highs today, no highs in x days, no double arrows day (e.g., rate of change of glucose levels stayed below a threshold), longest time in desired range (your longest in-range time is x hours), most screen views in a day, longest time without data gaps (e.g. not being out of range). In some cases, such as longest time, challenges could be a stored record that a user would try to beat.

### Auxiliary Interface 216: Location Module

Another example of an auxiliary interface 216 that can be used in accordance with the present embodiments is a location module. As used herein the term location module should be construed broadly to include both user input and any device and/or instructions capable of receiving as an input information about a user's location, processing the location information, and producing an output indicative of the user's location. Examples of location modules include a global positioning system (GPS) receiver, other location electronics such as triangulation using radio towers, etc.

In certain embodiments, the CGM module 208 provides a mechanism for correlating what effect, if any, a user's location may have on his or her blood glucose, or for using a user's location to provide the user with information that would be useful in managing his or her blood glucose. Thus, certain of the present embodiments leverage capabilities of smartphones 202 that relate to location detection so that the user's location can be used to identify possible location-based effects on the user's blood glucose, and to provide the user with useful information.

In various embodiments, with reference to Figure 9, the CGM module 208 may be used in conjunction with the location module to provide the user with information on nearby locations where food can be obtained when the user's blood glucose is low, or in danger of becoming low. In these embodiments, at block B900 the CGM module 208 receives an input from a CGM, which is a current EGV. The CGM module 208 processes the input at block B902, which may comprise comparing it to a threshold value. If the EGV is lower than the threshold value, indicating that the user has low blood glucose, or may soon have low blood glucose, the CGM module 208 may produce an output at block B904 alerting the user with one or more audible or visual alarms. The CGM module 208 may receive further inputs at block B900 from the location interface of the user's current location and the location of one or more nearby locations where food can be obtained. The CGM module 208 then produces an output at block B904, which may be, for example, an output to a display 222 of the smartphone 202 that shows the user where to obtain food, and may provide turn-by-turn directions after the user selects a destination from a list and/or by selecting an icon on a map.

Additionally or alternatively, altitude information, geographic information, demographic information, socio-economic information, etc., can be combined with other inputs, including CGM data, to provide trends and reports regarding the influences or correlations of any of the information on CGM data and vice versa (glucose control, sensor accuracy or reliability, etc.) for selected populations.

In various other embodiments, again with reference to Figure 9, the CGM module 208 may be used in conjunction with the location module to provide the user with a recommendation on where to eat based on diabetic considerations. In these embodiments, at block B900 the CGM module 208 receives an input, which may be a user request for a recommendation on where to eat. The CGM module 208 receives a further input at block B900 from the location module of nearby locations where diabetic-friendly food can be obtained. The CGM module 208 processes the inputs at block B902, which may comprise ranking the eating locations according to the degree to which they are healthy choices for diabetics, and/or grouping the eating locations according to one or more criteria input by the user. The CGM module 208 then produces an output at block B904, which is a recommendation on where to eat. The recommendation may be provided on the display 222 of the smartphone 202, and may include an address and/or directions to one or more locations. The input from the location module may further include information about a restaurant in which the user is located or that is nearby. The processing may comprise determining that that restaurant is not a good choice for diabetics, and the output may include a recommendation of a nearby restaurant that would be a healthier choice.

In various other embodiments, again with reference to Figure 9, the CGM module 208 may be used in conjunction with the location module to detect when a user is driving or riding in a car, and to correlate how driving affects blood glucose levels. In these embodiments, at block B900 the CGM module 208 receives an input from the location module indicative of the user being in a moving vehicle. For example, the location module may detect that the user is moving at a high rate of speed, or is intermittently accelerating and decelerating. The CGM module 208 receives a further input at block B900 from a CGM, which is the user's EGV. The CGM module 208 may receive substantially continuous EGV's from the CGM. The CGM module 208 then processes the inputs at block B902, which may comprise matching EGV's to substantially time corresponding data about the user, such as his or her average velocity, or frequency of slowing down and speeding up, which may be indicative of the amount of traffic the user is encountering. The CGM module 208 then produces an output at block B904, which may be to send matched data pairs to storage 210 for retrospective analysis. The storage 210 may be local or remote.

In various other embodiments, again with reference to Figure 9, the CGM module 208 may be used in conjunction with the location module to detect when a user is located far from home or from a location where help could be obtained should the user experience a hypoglycemic event. In these embodiments, at block B900 the CGM module 208 receives an input from the location module indicative of the user's location. The CGM module 208 receives a further input at block B900 from a CGM, which is the user's EGV. The CGM module 208 then processes the inputs at block B902, which may comprise comparing the EGV and location information against threshold values, such as a minimum EGV and a maximum distance. That is, the CGM module 208 may determine, based on the processing, that a potentially dangerous situation exists where the user's EGV is below a minimum threshold and the user's location is more than a maximum threshold distance from home or from a location where help could be obtained. The CGM module 208 then produces an output at block B904, which may comprise an audible and/or visual warning that is distinct from a standard alert or alarm. Additionally or alternatively, alert thresholds, messages, events, prediction horizons, and other customizable parameters can be adjusted automatically responsive to the analysis of the user's blood glucose and their location combined.

In another example, the CGM module 208 receives an input from the location module indicative of the user's location. The CGM module 208 receives a further input from the CGM indicating a level of power left in a battery powering the CGM. Alternatively, or in addition to the input from the CGM indicative of battery power, the CGM module 208 may receive an input from a battery module of the smartphone 202 indicating a level of power left in a battery powering the smartphone 202. The CGM module 208 then processes the inputs, which may comprise comparing the battery level(s) and location information against threshold values, such as a minimum battery level and a maximum distance. That is, the CGM module 208 may determine, based on the processing, that a potentially dangerous situation exists where the battery level is below a minimum threshold and the user's location is more than a maximum threshold distance from home or from a location where help could be obtained and/or the device could be recharged. The CGM module 208 then produces an output, which may comprise an audible and/or visual warning that is distinct from a standard alert or alarm.

In various other embodiments, again with reference to Figure 9, the CGM module 208 may be used in conjunction with the location module to correlate an influence of a user's location on his or her blood glucose. In these embodiments, at block B900 the CGM module 208 receives an input from the location module indicative of the user's location. The CGM module 208 receives a further input at block B900 from a CGM, which is the user's EGV. The CGM module 208 then processes the inputs at block B902. The processing may be applying one or more algorithms to two streams of input (not yet matched), for example, algorithms useful for correlating data and/or recognizing patterns. For example, the processing may comprise associating or analyzing EGV's with substantially time corresponding data about the user, such as his or her location. The CGM module 208 then produces an output at block B904, which may be to send the related data streams of information and/or matched data pairs to storage 210 for retrospective analysis. The storage 210 may be local or remote.

### Auxiliary Interface 216: Audio Module

Another example of an auxiliary interface 216 that can be used in accordance with the present embodiments is an audio module. As used herein the term audio module should be construed broadly to include any device and/or instructions capable of receiving as an input information about an audio signal, processing the audio information, and producing an output in the form of audio and/or information about the audio signal. Examples of audio modules include a digital music player, a microphone, etc.

In certain embodiments, the CGM module 208 provides a mechanism for matching different songs to different alerts, or for determining what effect, if any, a given song may have on a user's blood glucose. Thus, certain of the present embodiments leverage capabilities of smartphones 202 that relate to audio so that the user may customize different alerts with different songs, and so that various algorithms may be adjusted based on the user's response to different songs. For example, the speed and/or effectiveness of a user's response to alarms with a particular song, tune, pitch, or level of intensity can be tracked, after which the CGM module 208 can learn which audio alarms best help a user manage their health. In various embodiments, the CGM module 208 is configured to adaptively adjust the user's alarm sounds, tunes, songs, pitches, levels, etc., based on the analysis of or correlation to good blood glucose control.

In various embodiments, with reference to Figure 10, the CGM module 208 may be used in conjunction with the audio module to provide customizable alerts. The CGM module 208 may provide various alerts, such as a reminder to inject insulin, a reminder to get a reference blood glucose value, a reminder to eat, a reminder to schedule a doctor's appointment, a warning that blood glucose is dropping or is low (or rising, too high), or even confirmation that a user is within a target range, etc. These alerts may be hard coded into the CGM module 208, or may be programmable by the user. Even if the alerts are hard coded, the user may still be able to assign different songs to different alerts. Thus, when the CGM module 208 receives an input indicating that it is time to provide an alert, the song that the user hears corresponds to the song that the user previously assigned to that alert. For example, the CGM module 208 may receive an input at block B1000 from the timekeeping/scheduling module indicating that it is time for the user's next insulin dose. The CGM may process the input at block B1002 by identifying what song is to be played in connection with a reminder to inject insulin, and then produce an output at block B1004, which may be an on-screen notification to the user coupled with a command to the audio module to play the identified song.

In various other embodiments, at block B1000 the CGM module 208 may receive a first input from the audio module indicating what song is currently being played, and a second input from a CGM, which is the user's EGV. The CGM module 208 processes the inputs at block B1002. The processing may be applying one or more algorithms to two streams of input (not yet matched), for example, algorithms useful for correlating data and/or recognizing patterns. For example, the CGM module 208 may analyze characteristics of the song, such as its tempo. In another example, the CGM module 208 may process the first and second inputs by matching EGV's over time with songs that were playing at the time each EGV was received. The CGM may then produce an output at block B1004 of matched data pairs that are stored in storage 210 of the smartphone 202 or remotely. The CGM may perform further retrospective processing of the stored data pairs to attempt to identify a pattern relating to the effect of certain songs, or certain types of songs, on the user's blood glucose. Based on the retrospective processing, the CGM may output a list or ranking of songs most closely correlating with good blood glucose control.

In certain embodiments, the CGM module 208 does not require the user to input information and commands using his or her fingers. This interface can be cumbersome when trying to accomplish certain tasks. Thus, certain of the present embodiments leverage capabilities of smartphones 202 that relate to audio so that the user may input information and commands using his or her voice. This aspect of the present embodiments improves the ease of use of the system 200, which in turn increases the likelihood of patient compliance.

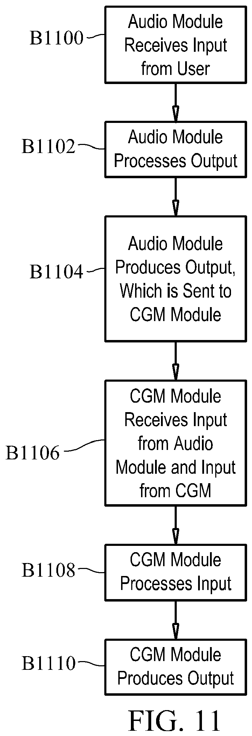

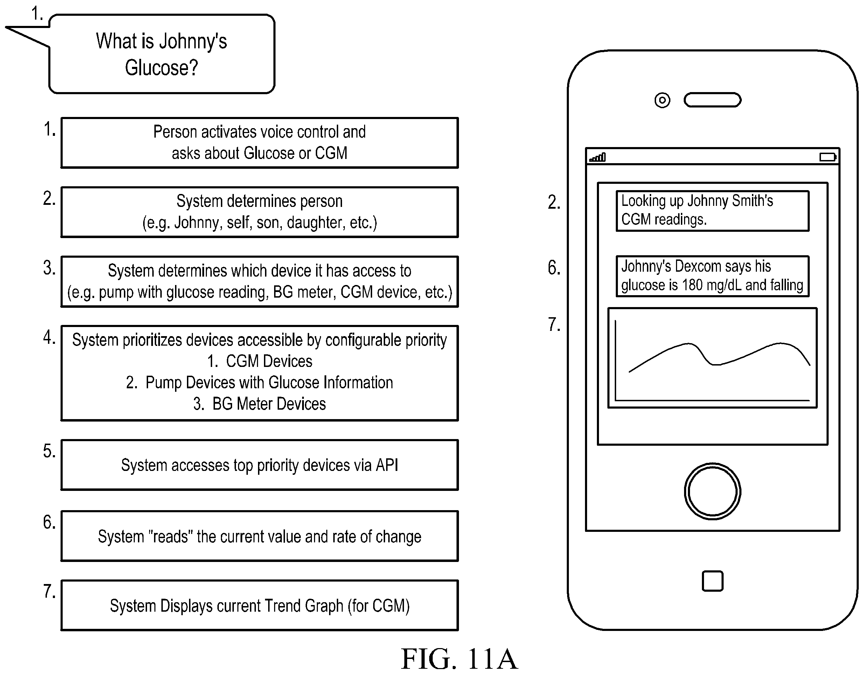

In various embodiments, the CGM module 208 may be used in conjunction with the audio module to recognize and respond to voice commands. In these embodiments, the audio module may comprise a microphone and related hardware/software/firmware for understanding voice commands. The CGM module 208 receives as an input from the audio module at block B1000 of information pertaining to a voice command. The CGM module 208 processes the input at block B1002 and produces an output at block B1004. The processing and output will vary according to the nature of the command. For example, with reference to Figure 11, in various embodiments the CGM module 208 receives a request from a user to output the user's current EGV. The request is received by the audio module at block B1100, which processes it at block B1102 and outputs information pertaining to the request to the CGM module 208 at block B 1104. At block B1106 the CGM module 208 receives the input from the audio module, and an input from a CGM, which is the user's current EGV. The CGM module 208 processes the inputs at block B1108 by accessing the user's current EGV, which is stored in local or remote storage 210. At block B1110 the CGM module 208 then outputs the user's current EGV, which may be provided as text information on the smartphone's display 222, or as a voice response delivered through one or more speakers of the smartphone 202. In alternative embodiments, the user may ask for the current EGV of another person, such as a family member. In these embodiments, the smartphone 202 would be wirelessly linked to devices associated with other people. An additional step in the process would involve the CGM module 208 sending a request to a CGM module 208 of the other person, and receiving a reply from that CGM module 208. In further alternative embodiments, the user may ask for different values, such as a last input BG value, predicted EGV for a future time period, or any of the recommendations or output described in more detail elsewhere herein. In still further alternative embodiments, the user may ask the CGM module 208 to output more than a single value. For example, the user may ask the CGM module 208 to display 222 an EGV history, such as for the past 24 hours, past two days, three days, etc. In some embodiments, the system asks the user to provide a single point blood glucose reference value, and the user may audibly provide the BG value to be received and processed by the CGM module 208 for calibration, validation, therapy recommendation, etc.

Figure 11A illustrates another example of how the CGM module 208 may be used in conjunction with the audio module to recognize and respond to voice commands. With reference to block B 1112 the user activates voice control for the smartphone, which may be done through a settings menu, for example, which may be associated with the CGM module 208. The user then asks the smartphone a question relating to glucose or CGM. For example, the user might ask for his or her own current EGV. Additionally or alternatively, the user might ask for another person's current EGV, such as the user's child or other relative. Preferably, the CGM module 208 is able to recognize a variety of natural language commands, such as "What is Johnny's glucose?" What is my son's blood sugar? What's my B G? What's Dad's meter reading? What does Johnny's CGM say? etc.

At block B1114, the CGM module 208 receives the user's inquiry and deciphers it by, in part, determining what person the user is inquiring about. At block B1116, the CGM module 208 determines which devices it has access to, and at block B1118, the CGM module 208 prioritizes the accessible devices according to a priority scheme, which may be configurable. For example, if the user has multiple devices that track their glucose, such as a blood glucose meter (with glucose data), a pump (with potential CGM data), as well as a stand-alone CGM device, and if all have glucose values at approximately the same timestamp, the algorithm prioritizes which of the three glucose values to display. At block B1120, the CGM module 208 accesses top priority devices via an application programming interface (API). At block B1122, the CGM module 208 reads the requested value from the relevant device as well as, optionally, a rate of change in the value, and at block B1124 the CGM module 208 produces an output to the display, which may be in the form of a numerical value and/or a trend graph.

### Sensor Calibration

in some embodiments, when a new sensor of a continuous analyte monitor is implanted, it may need to be calibrated so that is accurately converts its signal in units of current or counts to concentration in clinical units (mg/dL or mMolar for glucose) for outputting meaningful data to a user. Calibration of commercially available CGM's typically involves obtaining one or more reference analyte values. A reference analyte value can be an analyte value obtained from a self-monitored blood analyte test. One such test is a finger stick test, in which the user obtains a blood sample by pricking his or her finger, and tests the sample using any known analyte sensor. Where the analyte being sampled is glucose, the obtained value is often times referred to as a blood glucose (BG) value. The BG value is compared to a measurement of glucose taken by the continuous sensor at substantially the same time as the finger stick sample was obtained. In some embodiments, the calibration process generally involves correlating a BG value with a sensor value from the CGM to create a matched pair (which process may be iterated to create multiple matched pairs). The resulting matched pair(s) are typically regressed with or without additional information, from which the calibration parameters (sensitivity and in some cases baseline) of the sensor are derived. These parameters are then used to convert each CGM data into meaningful values in units of mg/dL or mM for storage/display to the user (and further processing).

The CGM module 208 may be used in various of the present embodiments to facilitate calibration of a continuous analyte sensor. In various embodiments, the image capture module may be used to capture image information of a blood glucose meter. For example, a digital camera of the smartphone 202 may take a photo of the display 222 of the blood glucose meter when the display 222 is showing information regarding a recent blood glucose measurement, such as a BG value in mg/dL. The CGM module 208 may receive the BG value and time stamp as an input. In some embodiments, the BG value may be entered manually using the smartphone directly into the CGM module or using a meter integrated with a smartphone (physically or wirelessly integrated).

However, in some embodiments the calibration process is performed by the sensor electronics 104 of the CGM device 100 (e.g., on the skin of the patient), remote from the CGM module 208 on the smartphone 202, where the BG (calibration) value is captured. In these embodiments, there may be a time delay between the time the user enters the BG value for calibration and the time the sensor data reflects an updated calibration responsive to the calibration process using the newly entered BG value. In some embodiments, this delay can be due, at least in part, to the sensor electronics 104 of the CGM device 100 not being in constant communication with the smartphone 202 (due to battery limitations). Thus a first delay can be due to waiting for the window of communication to occur between the CGM device 100 and the CGM module 208 on the smartphone 202. Once the sensor electronics 104 of the CGM device 100 receives the BG value for calibration and processes the calibration to calculate new sensor values with the BG value, another time delay occurs in waiting for the window of communication to send the data back to the smartphone 202 for display. For example, where the interval of communication is five minutes, this process could incur a ten minute delay. The delay can be even more frustrating to a user if the user entered a bad BG value, for example, wherein the conventional analyte meter produces an inaccurate value (BG meters are known to have a ±20% error in values), or other errors in readings known to occur due to patient error in self-administration of the blood glucose test. For example, if the user has traces of sugar on his or her finger while obtaining a blood sample for a glucose concentration test, then the measured glucose value will likely be much higher than the actual glucose value in the blood). In a scenario wherein the BG value is determined to be unacceptable by the sensor electronics of the CGM device 100 (e.g., based on a clinical or statistical analysis associated with the BG value, a set of matched data pairs, a regression of matched data pairs, etc.), the user will be prompted, up to ten minutes later (or similar time delay), to provide another BG value. This may be frustrating to the user and can be overcome by more communication between the sensor electronics 104 of the CGM device 100 and the CGM module 208 of the smartphone 202 to preempt such delayed feedback. Accordingly, the CGM module 208 may store a maximum acceptable value and a minimum acceptable value for a BG calibration value, and use these max/min values to determine, and provide immediate feedback, as to whether a BG value input to the CGM module 208 is likely to be in error.

Thus, in some of the present embodiments the CGM module 208 may store a minimum BG value and a maximum BG value that are calculated from previous EGVs using a cone of possibilities calculation and/or using *a priori* knowledge of possible blood glucose changes within a window of time. For example, if a user's glucose at noon is at 100 mg/dL and rising at 2 mg/dL/min, then an acceptable range for a BG value entered at 12:05 PM may be delineated by a maximum value of 120 mg/dL and a minimum value of 100 mg/dL. When the user inputs a next BG value, the CGM module 208 compares the input value to the stored max/min values, and, if the input value is outside the range defined by the minimum and maximum BG values, the CGM module 208 produces an output in the form of an alert notifying the user to obtain another BG value. The delay caused by intermittent communication between the sensor electronics and the CGM module 208 is thus eliminated.

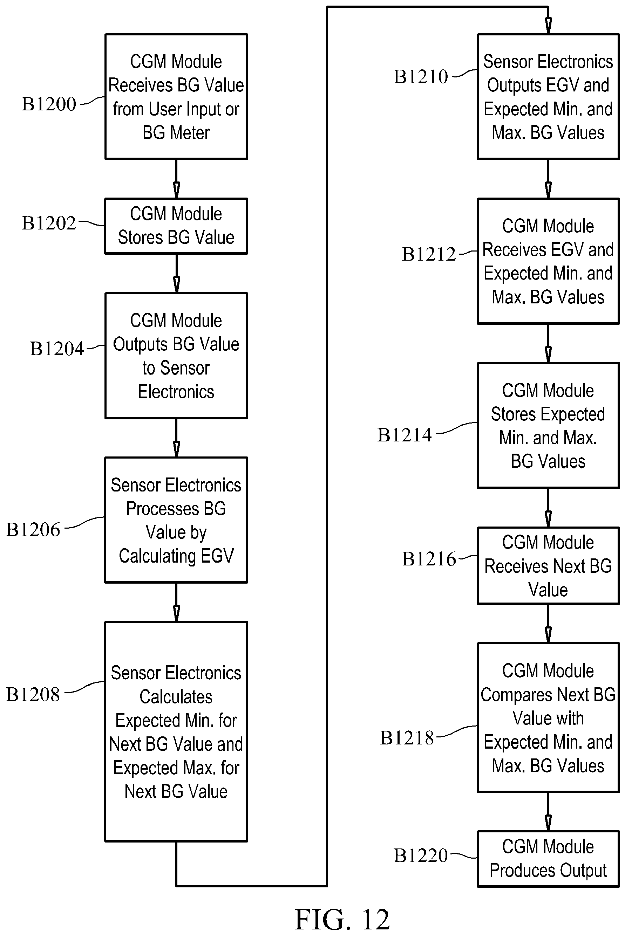

In these embodiments, with reference to Figure 12, at block B1200 the CGM module 208 receives as an input a BG value. The BG value may come from user input, or from a wireless transmission from the user's BG meter, for example. The CGM module processes the input at block B1202 by placing it in temporary storage 210. The CGM module then outputs the BG value to the sensor electronics at the next interval for communication at block B1204. The sensor electronics processes the BG value in connection with its EGV algorithm at block B1206. The sensor electronics also calculates an expected minimum for a next BG value and an expected maximum for a next BG value at block B1208. At block B1210 the sensor electronics outputs the values calculated at blocks B1206 and B1208 to the CGM module 208. The CGM module 208 receives the EGV and the expected minimum and maximum BG values at block B1212. At block B1214 the CGM module 208 stores the expected minimum and maximum BG values for later comparison (e.g., within the next 5 minutes, or other interval of time, prior to receipt of a next data packet from the sensor electronics). When the CGM module 208 receives an input of a next BG value at block B1216, the CGM module 208 processes this input at block B1218 by comparing it to the stored expected minimum and maximum next BG values. If the next BG input is outside the expected range, the CGM module 208 produces an output at block B1220. The output may be an error message and/or a request for a new BG value. In an alternative embodiment, the processing may be performed by the CGM module 208. In these embodiments, the CGM module 208 receives as an input a BG value, processes this input by calculating an expected minimum for a next BG value and an expected maximum for a next BG value based on recent EGVs, and optionally outputs these values to memory 204 or storage 210. When the CGM module 208 later receives an input of a next BG value, the CGM module 208 processes this input by comparing it to the expected minimum and maximum next BG values. Additionally or alternatively, the expected minimum and maximum BG values are calculated by the CGM module 208 only when a BG value is received. If the next BG input is outside the expected range, the CGM module 208 produces an output in the form of an error message (e.g., request for another BG value).

One example method of determining the range of acceptable BG values is to apply a simple error check to the last EGV, such as ± X%, where X may be any number. However, challenges for the correct detection of bad BG values include sensor drift, sensor noise, time delay between the most recent EGV and the current BG value, and glucose rate of change. One example method of compensating for drift is to use time during the week, measured drift (from BG values or impedance, for example), or predicted drift (from a model) to widen or narrow the acceptable range. In some embodiments, the acceptable minimum and maximum values are calculated differently depending upon time since implant. For example, upper and lower boundaries may be applied to limit the BG values, where the algorithmic parameters used to calculate the minimum and maximum values change over time. It is known, for example, that for some sensors sensitivity rises during the first day to three days after implantation, and eventually levels off. It is also known that for some sensors the slope of the sensor drift curve is greatest shortly after implantation, and gradually decreases. Accordingly, the time-dependent algorithmic functions in this case are the minimum and maximum values that delineate acceptable BG values (e.g., a wide range of acceptability during the first few days after implant when the sensitivity is likely to change the most). The time-dependent calculations may be derived, for example, from retrospective analysis of *in vivo* sensitivities and/or baselines of analyte sensors (and rates of change thereof). As this example illustrates, applied *a priori* knowledge doesn't have to be static. Rather, it can change over time in response to expected and/or measured changes in a given parameter and/or time. Further, applying *a priori* knowledge dynamically is not limited to the minimum and maximum values themselves. Namely, adaptive time-based parameters could be applied to any data or processing associated with processing of the continuous analyte data, including calculation of EGV's. Advantageously, drift correction of the EGV's and/or previous matched pairs in the calset improves the sensitivity/specificity of detecting bad BG values.

Noise detection algorithms for both the presence and severity of sensor noise may be used to influence the acceptable range of BG values (either widening or narrowing the range). For example, if a certain level of signal artifacts is not detected in the sensor data, then the EGVs are determined to be reliable. As another example, if a certain level of signal artifacts is detected in the sensor data, then the reference glucose data may be determined to be reliable. Additionally or alternatively, when a predetermined level of noise (or signal artifacts) in the sensor data is detected, the CGM module 208 (and/or sensor electronics of the CGM device) may indicate no BG value is acceptable until the level of noise (signal artifacts) drops below a second predetermined level. In some embodiments, if noise is detected when an input BG value is rejected, the CGM module 208 may produce an output in the form of a prompt to the smartphone's display 222 suggesting that the user wait a few minutes to recalibrate to provide an opportunity for the noise to dissipate.

Example methods of compensating for time delays include knowledge of physiological delays, inherent device delays, and a current level of filtering (related to noise detection). Each of these can be used to estimate an approximate time lag between the input BG value and a last EGV. Then a predictive algorithm could be applied to generate a center of the acceptable range. For example, if a trend indicates that glucose is rapidly falling, then the center of the range would be lower than the current value. A confidence interval could be applied to the prediction, or assigned based on the rate of change, to determine how wide or narrow the range should be.

In some embodiments, a measured rate of change itself could be used to widen/narrow the acceptable range. A direction of glucose change could also influence whether one boundary is stricter than the other. For example, if glucose was falling then the lower boundary could be ± X% while the upper boundary could be ± Y%, where X > Y, and the current EGV is the center of the range.

Certain of the present embodiments may also consider factors associated with clinical and/or statistical measures that evaluate the goodness of fit of matched data pairs as compared to predetermined acceptable boundaries (e.g., delineated by acceptable slopes and baselines) and/or a matched data pair as compared to a regression of a plurality matched data pairs (e.g., in a calibration set). For example, by evaluating the most recent sensor count value (uncalibrated sensor data) with the matched pairs that form a calibration set, (all or some component of them, such as the most recent matched pair only) a range of acceptable matched BG values for that uncalibrated sensor value that would not cause the calibration line (regression line formed from calibration set and optionally other parameters) to fall outside of the predetermined acceptable boundaries (e.g., delineated by acceptable slopes and baselines) could be used to further widen or narrow the range of acceptable max/min BG values allowed to be entered.

One advantage of the foregoing embodiments for determining whether an input BG value falls within an acceptable range is that the user is given immediate feedback as to whether or not their calibration BG value is accepted. The user thus does not receive a surprise alert that they need to redo their calibration, which alert may come many minutes later after the user has put his or her BG meter away, and which alert the user may miss because he or she is not expecting to take another BG value for several more hours. Another advantage is that the number of erroneous BG values accepted for calibration is limited so that accuracy of the sensor is improved.

In various other embodiments, the CGM module 208 may compensate for the delay in communication between the sensor electronics and the CGM module 208 by displaying an input BG value in a unique way until a new EGV is received from the sensor electronics. For example, the CGM module 208 may receive as an input a BG value. During the interval between the time that the BG value is input and the time that the sensor electronics provides an updated EGV, the CGM module 208 may produce as an output the input BG value, which is displayed on the display 222 of the smartphone 202 with a unique identifier so that the user understands that the value displayed is the BG value that was just input, and not an updated EGV. For example, the input BG value could be displayed in a different color or different shape, or it could blink on and off, or it could include additional text and/or symbols indicating that it is a BG value, etc. The BG value would continue to be displayed in the unique manner until the CGM module 208 receives as a further input the updated EGV from the sensor electronics. The CGM module 208 would then produce an output in the form of the updated EGV, which would replace the BG value on the smartphone's display 222.

In various other embodiments, the CGM module 208 may compensate for the delay in communication between the sensor electronics and the CGM module 208 by displaying an estimated next EGV until an updated EGV is received from the sensor electronics. For example, the CGM module 208 may receive as an input a BG value. The CGM module 208 may process the input BG value by calculating an estimate for a next EGV based on the input BG value and the most recent EGV. During the interval between the time that the BG value is input and the time that the sensor electronics provides an updated EGV, the CGM module 208 may produce as an output the estimated next EGV, which is displayed on the display 222 of the smartphone 202. The estimated next EGV would continue to be displayed until the CGM module 208 receives as a further input the updated EGV from the sensor electronics. The CGM module 208 would then produce an output in the form of the updated EGV, which would replace the estimated next EGV on the smartphone's display 222.

### Insulin Dosage Recommendation

In certain embodiments the CGM module 208 may comprise a bolus and/or basal insulin calculator that receives as an input information about food consumed or soon to be consumed, and an input from a CGM, and provides as an output an insulin bolus recommendation. In some embodiments, the insulin calculator uses inputs derived from auxiliary interfaces described elsewhere herein. The insulin calculator may take into consideration a rate of change, a trend, and/or directional information regarding the user's recent EGV's in the calculation of a dosing recommendation. In some embodiments, the bolus calculator may be configured to calculate a bolus recommendation based on 1) a current glucose value, 2) estimated meal information, and 3) rate of change, trend, and/or directional information. The output from the CGM module 208 may take the form of a numerical value displayed on the display 222 of the smartphone 202 and/or a transmission to an insulin delivery device.

In addition to real-time processing, retrospective analysis of one or a plurality of data sets (e.g., from one or a plurality of sensor sessions) can be useful in analyzing and understanding a user's unique diabetes information (e.g., blood glucose data over time). Thus, in some embodiments the CGM module 208 may be programmed with a series of therapy adjustment recommendation criteria that can be selected and/or deselected by the user, wherein the CGM module 208 is programmed to run the selected criteria against continuous glucose sensor data and provide highlighted problem areas and/or areas for target or focus. Thus, the CGM module 208 would receive as an input CGM data, process that input by applying the selected therapy adjustment recommendation criteria, and produce an output, which may be recommendations shown on the display 222 of the smartphone 202. In one example embodiment, a criterion is configured to evaluate overnight CGM data (e.g., glucose values, highs, lows, time spent above a target, time spent below a target, rate of change, glycemic variability, euglycemia, hypoglycemic events, and/or hyperglycemic events), whereby the CGM module 208 is programmed to recommend an adjusted basal insulin rate in response thereto. The information could be highlighted on a screen of data, provided as text-based tips, etc.

Referring again to retrospective analysis of CGM data, in some embodiments the CGM module 208 may be programmed to display 222 improvement information on the smartphone display 222 (e.g., graphs or charts). The improvement information may be indicative of a reduction in the number of hypoglycemic events, a reduction in time spent in hypo- or hyperglycemic regions, an increase in target and/or a reduction in glucose variability, for example. Improvement information can be presented for a variety of different time periods, for example, a day, a week, a month, or more. In these embodiments, the CGM module 208 would receive as an input retrospective data regarding a user's EGV history, process that data by identifying trends that indicate improvement, and generate an output such as text or graphical information on the smartphone's display 222.

### Alerts

Certain of the present embodiments relate to alerts provided to the user. For example, in certain embodiments, the CGM module 208 reduces the number of false alerts, provides alerts for certain events that are not typically provided for, provides a mechanism for customizing characteristics of alerts, provides a mechanism for overriding alert settings, and provide reminders to the user to perform certain tasks. Accordingly, certain of the present embodiments leverage the capabilities of smartphones 202 to provide the foregoing functionalities.

In some smartphone radio protocols, for example, a parameter that can be used in device pairing is the master device ID. In order to establish a communication channel, the master transmitter broadcasts its device ID (along with some other information) in a beacon, and the receiver checks received beacons for the presence of the device ID of the transmitter with which it wants to communicate.

Although the master device ID provides some level of security, in that a slave device can be programmed to communicate only with a master device having a particular device ID number, additional security can be useful in some embodiments. To provide additional security, some embodiments can use two pieces of information to pair a receiver with a particular transceiver device. These two pieces of information include the device ID described above and another value that is referred to herein as a sensor security code. The device ID is used as described above to filter receipt of non-matching messages at the lowest layer of the radio protocol stack of the smartphone. The sensor security code is used for a key-based authentication scheme at the software application layer of the system. In some embodiments, both the device ID and the sensor security code can be derived from an identifier (e.g., a manufacturer's serial number) associated with the CGM device per the description below. In some embodiments, the identifier may be etched into, printed on or otherwise attached to a housing of the sensor electronics (transmitter) of the CGM device.

In some embodiments, the sensor electronics of the CGM device is paired with the smartphone, thereby establishing and enabling one- or two-way communication between the CGM device and the CGM module. In various embodiments, the CGM module 208 may provide to the user an immediate notification regarding pairing the smartphone with the CGM device, and a later alert when pairing is complete. For example, current CGM's typically take five minutes or more to complete pairing, and during this interval the user is unaware of whether the pairing is successful, which can lead to user confusion. One of the present embodiments provides the user with an immediate notification that pairing may take up to a certain amount of time to complete, such as up to fifteen minutes. When pairing completes, the CGM module 208 provides the user with an alert notifying him or her that pairing is complete. In these embodiments, the CGM module 208 receives as an input a notification that pairing is in process from the transmitter and/or smartphone. The CGM module 208 processes the input by accessing a stored notification of how long the pairing process may take to complete. The CGM module 208 then provides an output to the user of an indication of how long the pairing process may take to complete. The output may be text shown in the smartphone's display 222, and/or a voice response delivered through one or more speakers of the smartphone 202. When pairing completes, the CGM module 208 receives as an input a notification from the sensor electronics that pairing is complete. The CGM module 208 processes the input by accessing a stored notification of pairing completion, and provides an output to the user of an indication that pairing is complete. The output may be text shown in the smartphone's display 222, and/or a voice response delivered through one or more speakers of the smartphone 202.

In various other embodiments, the CGM module 208 may synchronize the form of CGM alerts, settings, modes and/or profile with the ringer setting on the smartphone 202. For example, most smartphones 202 have a mechanism that enables the user to choose between audible tones for incoming calls/texts/etc., and vibrate mode or silent mode in which the phone only vibrates or does nothing in response to incoming calls/texts/etc. When the user switches the smartphone 202 from one mode to another, the CGM module 208 may receive as an input a notification of the change and automatically change the alerts associated with the CGM module 208 to the corresponding setting. The processing performed by the CGM module 208 on the input may be accessing individual alert settings, modes and/or profiles associated with the corresponding setting, and the output may be to an alert module with a notification to change its settings.

In various other embodiments, the CGM module 208 may enable a user to override various settings. For example, some users want the capability to override default alert settings of their devices, while other users do not. To meet the needs of both of these types of users, the present embodiments of the CGM module 208 may default to a first set of alert thresholds, but in a nighttime profile the user has the option to override the alert thresholds and specify their own custom thresholds. The custom threshold selection options don't appear unless the user selects an option to make them appear, such as by checking a box. The nighttime profile options are thus kept simple for those users who want a simple interface, but more advanced users have access to the more advanced options that they desire. In these embodiments, the CGM module 208 may receive as an input a user selection to enable advanced features. The CGM module 208 processes the input by enabling additional selections, modes, thresholds and/or options that would not otherwise be visible or enabled (by default) to a user, and produces an output, which is a signal to a feature module to unlock advanced features.

In various other embodiments, the CGM module 208 may time stamp or otherwise tracks how often a user looks at his or her historical blood glucose data, and provide a reminder if the user has not checked his or her historical blood glucose data for a given amount of time. In these embodiments, the CGM module 208 receives as an input a request from the user to display 222 his or her historical blood glucose data. The CGM module 208 processes the input by time stamping it, and produces an output, which is to set a timer to count for a predetermined length of time corresponding to a recommended maximum amount of time between user checks of historical blood glucose data. If the timer reaches the predetermined length of time before the user again checks his or her historical blood glucose data, the CGM module 208 determines that the timer has reached the predetermined length of time. The CGM module 208 then produces an output, which is a reminder to the user to check his or her historical blood glucose data. The output may be text shown in the smartphone's display 222, and/or a voice response delivered through one or more speakers of the smartphone 202.

In various other embodiments, the CGM module 208 may use a predictive algorithm to predict future glucose values, where the algorithm's parameters are fixed, rather than user-settable. Examples of parameters that may be fixed are prediction horizon and alert threshold. This predictive alert can be used in conjunction with a threshold alert (the user can still set the threshold value for this alert) with the goal of providing patients with sufficient warning time to avoid a hypoglycemic event without generating an excessive number of additional alarms. This prediction scheme can also be used to generate alerts for hyperglycemia, and to determine when to shut off insulin in a low glucose suspend type of device.

In various other embodiments, the CGM module tracks patterns of when a user manually changes modes or settings, and, upon finding a pattern automatically, or based on response to prompts, modifies profiles accordingly. For example, if the user regularly changes their hypoglycemic alarm thresholds at 9 PM, the CGM module may ask the user whether they would like to set up a night time profile to include their regular nighttime hypoglycemic alarm.

In various other embodiments, the CGM module 208 may provide alerts that are discreet, customizable, awakening, friendly, safe, and/or acknowledgeable. The alerts are adaptable to certain conditions such as day, sleep, school/work, noisy environment, etc., and the user may add as many alert profiles (day, sleep, meeting, etc.) as needed. Each profile allows the user to turn on/off the desired alerts, and to customize the alerts according to preference. The sleep profile can be customized to tell the smartphone 202 when the user is going to sleep and/or waking up, set a duration for sleep, detect when the user wakes up (in conjunction with the activity monitor), synchronize with alerts external to the CGM module 208, etc. Each profile can be synchronized with the smartphone 202's timekeeping/scheduling module. For example, certain alerts may be silenced when the timekeeping/scheduling module indicates that the user is busy, such as in a business meeting.

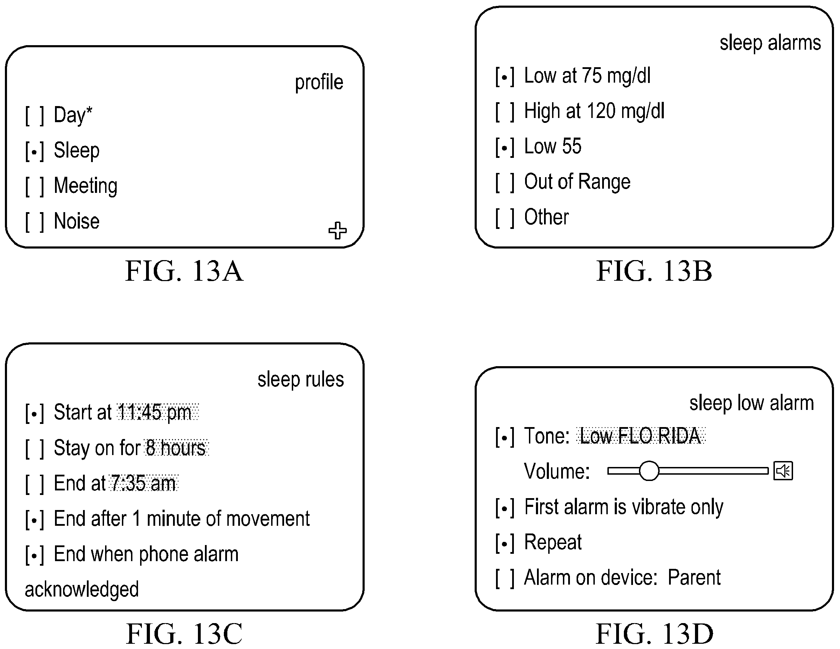

Figures 13A-13D are screenshots of a smartphone display 222 illustrating example embodiments of interfaces for customizing alerts. For example, Figure 13A illustrates a profile selection menu, where the user may choose from various profiles (Day, Sleep, Meeting, Noise, etc.) to customize. Figure 13B illustrates a sleep alarms settings menu, where the user may designate the criteria for when an alert should be provided. In the illustrated embodiment, an alert would be provided when the user is sleeping and his or her blood glucose goes outside of the designated range (75-120 mg/dL in the illustrated example). Figure 13C illustrates a sleep rules menu, where the user may designate when he or she is going to sleep and/or waking up, set a duration for sleep, detect when the user wakes up (in conjunction with the activity monitor), etc. Figure 13D illustrates a sleep low alarm menu, where the user may designate what tone/song should be played for a low blood glucose condition, and where the alert should be sent (to the parent's device in the illustrated example).

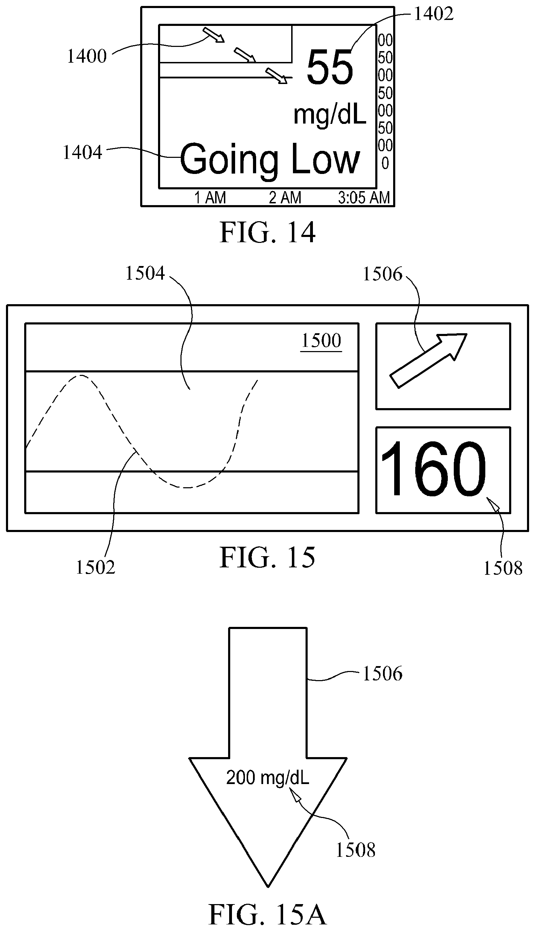

In various other embodiments, the CGM module 208 may provide a predictive alert on the smartphone's display 222 when a severe hypoglycemic event is predicted to occur in the near future. For example, the predictive alert may be shown when a severe hypoglycemic event is predicted to occur within 5 minutes, 10 minutes, 15 minutes, 20 minutes, 30 minutes, etc. With reference to Figure 14, an arrow 1400 may be displayed on a trend screen pointing towards a BG value 1402 that indicates a severe hypoglycemic event, such as 55 mg/dl. The arrow 1400 may change color as it transitions from euglycemia to hypoglycemia, to emphasize the change in glucose levels that is expected. Furthermore, the arrow 1400 may be animated to flash to emphasize the seriousness of the alert. The display may also show text 1404, such as Going LOW. This predictive alert may be configured to be prioritized over (override) whatever mode or application the smartphone 202 is in at the time the CGM module 208 determines that a severe hypoglycemic event is predicted to occur. In other words, the alert will interrupt whatever is currently on the smartphone's display 222.

In these embodiments, the CGM module 208 may be programmed with a blood glucose value corresponding to a threshold below which the user is considered to be hypoglycemic. As the CGM module 208 receives as inputs multiple EGV's at time-spaced intervals, it processes the inputs by comparing each one to the programmed value, and also to previously received EGV's. If the user's blood glucose shows a downward trend, and is approaching the programmed value, the CGM module 208 outputs an alert such as the one shown in Figure 14 to the smartphone's display 222. The user thus receives an advance warning of a potential hypoglycemic event, so that he or she can take appropriate action to avoid the hypoglycemic event.

In various other embodiments, the CGM module 208 may change the color of the display 222 to reflect the user's current blood glucose level. For example, the user's EGV may be displayed on the screen as a number, as a trend graph, a horizontal bar graph, etc. The text and/or background on the display 222 may change when the user's current blood glucose level transitions from one state to another. For example, the text/background may show a first color, such as green, if the user's blood glucose is within a healthy range, and a second color, such as red, if the user's blood glucose is low or high. Alternatively, a first color may be used for the healthy range, a second color for low, and a third color for high. Further, when in the low or high range, as the user's blood glucose becomes increasingly lower or higher, the intensity of the color may increase. The text/background may also flash, with the frequency of the flashing increasing as the user's blood glucose becomes increasingly lower or higher.

In these embodiments, the CGM module 208 may be programmed with blood glucose values corresponding to low and high threshold BG values. As the CGM module 208 receives as inputs multiple EGV's at time-spaced intervals, it processes the inputs by comparing each one to the programmed values. If the user's blood glucose value crosses one of the thresholds, the CGM module 208 outputs an alert to the smartphone's display 222 in the form of changing the color of the text and/or background. If the user's blood glucose value continues to become increasingly low or high, the CGM module 208 produces additional outputs, such as increasing the intensity of the color and/or causing the text/background to flash. These additional outputs may be generated in response to the CGM module 208 comparing input EGV's to additional programmed threshold values.

In various other embodiments, the CGM module 208 may use iconography and/or alert symbols that reflect real time data. For example, the user's blood glucose becomes low, an icon on the smartphone 202 may show an image of the user's BG trend graph using actual data points from EGV's. The input-processing-output for this embodiment would be substantially the same as that for the previous embodiment.

With extremely low blood glucose, a person can lose consciousness. Thus, in certain of the present embodiments, at a predetermined level or event (low glucose level, no button pressing after alert, etc.) that might signify a loss of consciousness, the CGM module 208 may go into an emergency response instruction mode. This mode may include an alarm to alert others in close proximity to the user that something is wrong. For example, step-by-step instructions on how to assist the unconscious user may be shown on the smartphone's display 222, such as administering glucose tabs or another form of carbohydrates, calling an ambulance, etc.

In these embodiments, the CGM module 208 receives an input from a CGM, which is the user's EGV. The CGM module 208 processes the input by comparing it to one or more threshold values, and determines that the user's blood glucose is low. The CGM module 208 produces an output in the form of an alert. If the user does not respond to the alert by pressing a button or an area on a touchscreen display 222, for example, the CGM module 208 determines that the user may be unconscious, and produces another output in the form of the emergency response instruction mode discussed above.

In various other embodiments, the CGM module 208 may provide differentiated visual high/low thresholds versus alert thresholds. For example, the CGM module 208 may be programmed with low and high blood glucose thresholds. These thresholds may be shown on a blood glucose trend graph on the display 222 as horizontal lines that the user should strive not to cross. Ordinarily, crossing either of the lines might generate an alert. However, excessive alerts can be annoying to the user, and can decrease patient compliance. Thus, in the present embodiments the visual high/low target range boundaries shown on the graph may be different from boundaries that generate an alert. For example, the boundaries that generate an alert might be wider than the visual target range threshold boundaries on the display 222, and the boundaries that generate an alert may be hidden from view. This configuration gives the user a little bit of a buffer zone to cross either of the visual boundaries without generating an alert. Alternatively, the boundaries that generate an alert might be visible, but distinguishable from the target range boundaries. Examples of visual distinctions include different colors, flashing vs. static, solid vs. dashed, different line weights, an alarm icon adjacent the alarm lines, etc. In some embodiments the high/low target boundaries may always be displayed, but the alert boundaries may be shown or not based on a user setting, a mode (e.g. silent), thresholds, etc.

In various other embodiments, a user interface of the CGM module 208 may be the first thing that the user sees when he or she activates the smartphone's display 222. For example, many smartphones 202 automatically put the display 222 to sleep when no activity is detected for a predetermined amount of time. This measure saves battery power. To reactivate the display 222, the user must press a button on the smartphone 202. In certain of the present embodiments, when the user reactivates the display 222, the first thing he or she sees is the user interface of the CGM module 208. In these embodiments, the CGM module 208 receives as an input a notification that the display 222 has entered sleep mode, followed by a later notification that the display 222 has been reactivated. The CGM module 208 processes these inputs and produces as an output a display 222 of the user interface of the CGM module 208 on the smartphone's display 222.

### Display 222

Certain of the present embodiments provide enhancements to information that the CGM module 208 displays on the smartphone's display 222. For example, with reference to Figure 3A, in various embodiments the CGM module 208 may display a trend graph of the user's historical blood glucose data. The trend graph may include a macro trend graph 306, which may display the user's blood glucose data for the past week, month, etc. A sliding bar 308 on the macro trend graph 306 may highlight a portion of the graph 306 that is displayed on a micro trend graph 310. By sliding the bar 308 left or right, the user may select a desired window of time to display on the micro trend graph 310. For smartphones having a touchscreen display, the user may slide the bar left or right by placing his or her finger on it and moving left or right. The data displayed on the micro trend graph 310 would adjust as the position of the bar 308 changes.

A width of the bar 308, and hence a length of the window of time displayed on the micro trend graph 310, may be adjusted by selecting one of a plurality of time select icons 311. In the illustrated embodiment, the time select icons 311 may include windows of 1 hour, 3 hours, 6 hours, 12 hours, 24 hours, etc. If the user changes the scale of the macro trend graph 306, the values of the time select icons 311 may change by a corresponding factor. For example, in the illustrated embodiment one week of data is shown on the macro trend graph 306, and the time select icons 311 include windows of 1 hour, 3 hours, 6 hours, 12 hours and 24 hours. If the user adjusts the view to show two weeks of data (factor of two increase), the values of the time select icons 311 may also increase by a factor of two, i.e. 2 hours, 6 hours, 9 hours, 24 hours and 48 hours.

With a smartphone 202 having a touchscreen display, when the user touches the micro trend graph 310 with his or her finger at a first point 312, it shows the blood glucose value at that point 312, or at the point that most closely aligns with the user's finger in the vertical direction. If the user slides his or her finger right or left, the displayed value changes as the user's finger lines up with different data points. The user can thus look at any desired data point on the graph. In these embodiments, the CGM module 208 receives as an input a location of where on the touchscreen the user has placed his or her finger. The CGM module 208 processes the input by determining which data point on the graph most closely aligns with the user's finger. The CGM module 208 then produces an output by displaying the value of the determined point on the smartphone's display 222 (230 mg/dL in Figure 3A).

In various other embodiments, the CGM module 208 may show a magnitude of a change in blood glucose between two points on the micro trend graph 310, and/or a rate of change between the two points. For example, if the user touches the graph at two points 314, 316 simultaneously, using two fingers, the change/rate of change in blood glucose between the points that most closely align with the user's fingers is displayed. In these embodiments, the CGM module 208 receives as an input location of where on the touchscreen the user has placed his or her two fingers. The CGM module 208 processes the input by determining which data points on the graph most closely align with the user's fingers. The CGM module 208 then produces an output by displaying the change/rate of change in blood glucose between the determined points on the smartphone's display 222 (185 mg/dL and 150 mg/dL/hr in Figure 3A).

In various other embodiments, the CGM module 208 may include a specialized menu of the actions that are most commonly performed, and this menu may be accessible from the default screen of the user interface of the CGM module 208. Example actions include changing an alarms profile, logging events (insulin, meal, etc.), calibrating, viewing/hiding events, social sharing, counting down to a next event (as described above with respect to the timekeeping/scheduling module), etc. Grouping the most common actions together in one convenient location saves the user time. In some embodiments, the menu enables one-click access to screens that traditionally require navigation through multiple screens and therefore result in poor user compliance.

In various other embodiments, the CGM module 208 may extend the blood glucose trend graph into the future and show a range of possible future glucose values. For example, the graph may extend fifteen minutes into the future, and show a range of possible future blood glucose values during that fifteen minutes. This embodiment increases the user's understanding of future trends for improved blood glucose control. This concept is further described in U.S. Patent Application Publication No. 2005/0203360, which is incorporated herein by reference in its entirety.

In various other embodiments, a trend graph displayed by the CGM module 208 is color coded. For example, with reference to Figure 15, the color of the graph 1500 (either the trend line 1502 or the background 1504) may be green if within a target range, yellow if ± 10% outside the target range, orange if ± 15% outside the target range, and red if ± 20% outside the target range. A trend arrow 1506 may be similarly color coded, and the angle at which the trend arrow 1506 is oriented may correspond to the actual rate of change of the user's glucose, i.e. a more horizontal arrow indicates a low rate of change, while a steeply sloping arrow indicates a high rate of change. In these embodiments, the CGM module 208 receives as inputs continuous EGV's from a CGM. In some embodiments, the rate of change is calculated by the CGM device and sent to the CGM module 208 for display (e.g., determination of how to display and resulting display), although the CGM module 208 may also perform rate of change calculations. The rate of change based on a linear or non-linear function is applied to a window of recent sensor data. In some embodiments, the rate of change calculation comprises calculating at least two point-to-point rate of change calculations, and wherein the rate of change calculation further comprises adaptively selecting a filter to apply to the point-to-point rate of change calculation based at least in part on a level of noise determined. The CGM module 208 outputs these values as data points on the trend graph 1500 on the display 222 and also updates the value 1508 shown in the box containing the user's most recent EGV. If the user's blood glucose is dropping, the CGM module 208 outputs this information by orienting the arrow 1506 downward, while if the user's blood glucose is rising the CGM module 208 outputs this information by orienting the arrow 1506 upward. In some embodiments, the trend arrow is located on the end of the trend graph (e.g., rather an in a separate box/area).

In certain embodiments, a size of the value 1508 shown in the box containing the user's most recent EGV may change size depending on how far off the user is from their target zone. For example, as the user's glucose gets farther and farther away from the target zone, the number could get bigger and bigger. This amplification could be one directional or either direction, meaning the EGV displayed on the trend graph could get bigger and bigger if it's outside the target range in either direction or only get bigger and bigger if it's outside the target range on the low side (e.g. hypo)). The same applies to the trend arrow 1506. With reference to Figure 15A, the trend arrow 1506 could be drawn large enough to fit the EGV 1508 inside the arrow 1506. The layout of the trend arrow 1506 / EGV 1508 in Figure 15A may be used independently of the foregoing embodiment in which the size of the trend arrow 1506 / EGV 1508 changes dynamically as the user's glucose changes.

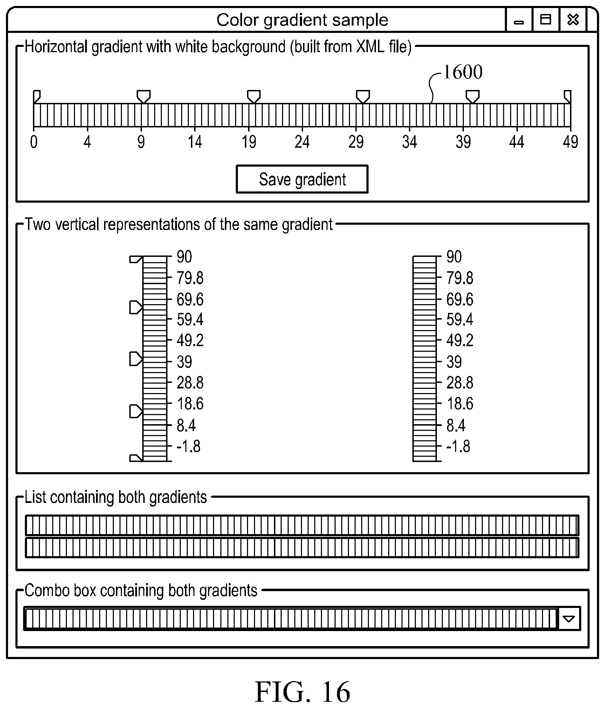

In various other embodiments related to that of Figure 15, rather than using a hard threshold for the transition from one color to the next, the display could instead show a gradient type of trend graph. That is rather than transitioning directly from green to yellow as soon as the user's glucose hits the threshold of, say ± 10% outside the target range, the display would instead gradually transition from green to yellow as the user's glucose moves away from the target range toward the established threshold. Thus, at ± 5% outside the target range, the display would show a color between green and yellow, with the color becoming gradually more yellow as the user's glucose moves through ± 6% outside the target range, ± 7% outside the target range, ± 8% outside the target range, etc. Figure 16 illustrates examples of a color gradient 1600 that could be used in connection with this embodiment. As the user's glucose moves farther away from the target range, the color transitions gradually through the colors on the gradient 1600.

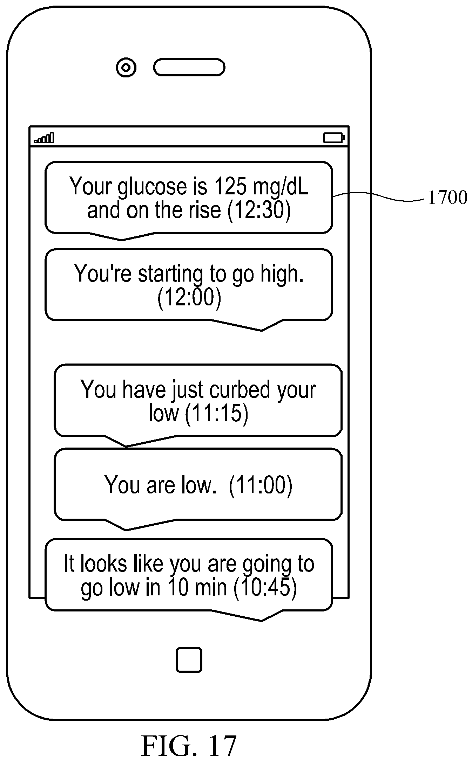

In various other embodiments, illustrated in Figure 17, information is displayed to the user as text rather than in graphical form. In the illustrated embodiment, the text is formatted to resemble a string of text messages 1700 (e.g., short message service (SMS)), which is a presentation that is familiar to many users. Some users may not be comfortable with, or able to understand information presented in graphical form. Thus, the format of Figure 17 may be better suited to these users. Each text message 1700 may or may not include a timestamp. Examples of short messages that could be shown to the user include, "Your glucose is 125 mg/dL and on the rise (12:30)" or "It looks like you are going to go low in 10 minutes (10:45)". In some embodiments, if the user touches any of the messages 1700, a popup window may enable the user to post that message 1700 to a social media site.

In various other embodiments, the CGM module 208 may compute user success metrics and display 222 them to the user and/or provide alerts. For example, the success metrics may include a percentage of counts captured, a percent distance from a midpoint of a target range of blood glucose, a percentage of times that the user calibrated the sensor within a predetermined amount of time from a reminder to calibrate, a percentage of a session completed, time spent in hypo during nighttime, SMBGs within the target glucose, a number of times the user looked at his or her trend graph, how quickly a user acknowledged an alert (e.g. low glucose), etc. This concept is further described in U.S. Patent Application Publication No. 2011/0004085, which is incorporated herein by reference in its entirety. Some metrics are more indicative of successful use of CGM than others (e.g. time spent in target zone), thus each of the foregoing metrics could be weighted in an analysis that provides the user with additional information. For example, logistic regression could be used. An example of logistic regression is in the diagnosing of heart disease. So if the subject is a smoker, doesn't work out, is X years old, has a family history of the disease, then it's X% likely that he or she will have heart disease. Success metrics may be calculated by the CGM module 208 locally on a smartphone and/or in the cloud. Additionally or alternatively, success metrics can be reported to another device (e.g., to a parent's device), to a social networking site (e.g., Facebook), or to a .pdf report for a medical record. In some embodiments, the success metric may be used in a gaming manner wherein CGM users may compete within the CGM module 208 and/or on a network against a computer game or another CGM user. Competing with CGM based data is described in more detail elsewhere herein.

In various other embodiments, the CGM module 208 may display 222 the user's blood glucose dynamic trend graph as wallpaper in the background of the smartphone's display 222. The wallpaper may display every time the user wakes up the smartphone. Further, through the use of e-ink type technology, the smartphone can seem as if it is always on and always displaying the trend graph. For example, smartphones typically enter sleep mode after a preset interval. That is, the display automatically shuts off to conserve power when the device is not being used. In these embodiments, when the smartphone would normally shut off, the display may switch to the home screen trend graph. Whenever the CGM module 208 gets additional information from the transmitter 104, the home screen could refresh. As long as the refresh interval is shorter than the interval for entering sleep mode, the trend graph always appears. Advantageously, e-ink technologies do not require power to keep a fixed image on the display. Thus, this embodiment would not drain the smartphone's battery.

In various other embodiments, the CGM module 208 may detect that the user is holding his or her finger down on an individual point on the trend graph, which is displayed on the smartphone's display 222. The CGM module 208 may then enable certain options, such as coloring the designated point differently, attaching notes to the designated point, event information, sensor data information or other information. Anything stored in connection with that point in time can be displayed. Also, certain options may be displayed, such as sharing information associated with that point on a social network, etc. If the user acts on the point, the CGM module 208 may automatically color code that point and save whatever action the user took. When the user downloads stored data from the smartphone, he or she can see what they did with respect to certain points. The CGM module 208 may also keep a log of the user's actions on points, and possibly a screen shot of a given amount of time, such as ± 3 hours, or give the user the option of how much data they want to store regarding a given point. In certain embodiments an algorithm may detect major changes in glucose (e.g. 100 mg/dl to 250 mg/dl in an hour), and an alert to the user may ask if he or she would like to write notes on this event. The actions in the foregoing paragraph would then be performed.

In certain embodiments, the display 222 may be interactive. For example, with reference to Figure 3A, the trend graph 310 may include one or more boundary lines 318. The boundary line 318 may indicate a high or low glucose level. If the user touches the boundary line 318, a popup bubble (not shown) may appear that explains what the boundary line 318 is, and/or indicates what the numerical value of the boundary line 318 is. The popup bubble may also ask the user if he or she wants to move the boundary line, e.g. raise or lower the glucose level at which the boundary line 318 appears.

In still further embodiments including an interactive display 222, one or more icons may appear on the trend graph 310. For example, certain events may be indicated along the curve with icons. If a user eats a meal at 6:00 PM, for example, he or she may enter information about that meal and an icon, such as a knife and fork, may then appear on the curve at 6:00 PM. If the user touches the icon, the information that he or she entered about the meal may appear in a popup bubble, or on a separate screen, for example. In general, information appearing on the trend graph 310 as icons may be derived from input data and processing, and the user can touch the various icons to get more detail about each event.

In still further embodiments including an interactive display 222, a status icon may be shown on the display 222. The status icon may appear on the screen that shows the trend graph 310. Alternatively, the status icon may appear on all screens associated with the CGM module 208, e.g., whatever screen the user navigates to, the status icon will appear somewhere. The status icon, when selected, may provide a shortcut to outstanding actions that the user may have skipped. For example, the user may have skipped a calibration. Selecting the status icon will remind the user that he or she was supposed to have performed a calibration at XX:XX AM/PM.

### Upgrading Smartphone 202 Operating System

In some embodiments, smartphone 202 may use a commercially available operating system in addition to CGM module 208. As a non-limiting example, smartphone 202 may be an iPhone® commercially available from Apple, Inc. and run an version of the iOS® operating system also commercially available from Apple, Inc. CGM module 208 can be software contained in an App downloaded from iTunes®, also commercially available from Apple, Inc. From time-to-time, Apple may release a new version of the iOS, and a user may want to upgrade a current version resident on smartphone 208 to the upgraded version. However, the new version of iOS may contain changes that cause some or even all features of CGM module 208 to not work.

In some embodiments, CGM module 208 can initiate an alert to a user over the smartphone 202 as to whether the new OS version will work with the CGM module prior to the user using (e.g. downloading and installing) the new version of the operating system. Further, it has been recognized that a new version of an operating system may be released with little notice. Thus, it may be difficult to know with enough lead time whether the new version of the operating is compatible with the CGM module 208. Thus, CGM module 208 can also initiate an alert to the user over the smartphone 202 to delay using the new version of the operating system until compatibility has been determined.

In addition, a new version of CGM module 208 may be available for the user to download. CGM module 208 can initiate an alert to the user over smartphone 202 as to the availability of the new version of the CGM module 208 and whether the new version of the CGM module 208 is compatible with the current version of the operating system on smartphone 202.

In some embodiments, CGM module 208 communicates with a remote server that contains information about which versions of CGM module are compatible with which versions of operating systems used by smartphone 202. The communication between CGM module 208 and the remote server can be via one or more communication networks, such as cellular, Wi-Fi and the like. The communication can be initiated programically by CGM module upon the occurrence of a detected or monitored event, such as the expiration of a predetermined period of time since a last communication, the initiation of the CGM module 208 (e.g., opening an software application comprising the CGM module), user initiation via smartphone 202 (e.g., selection of a menu item on smartphone), upgrade of a new operating system on the smartphone, and the like. The CGM module can programically transmit information about the current version of the CGM module 208 and the current version of the smartphone's operating system to the remote server. The remote server can then compare the versions with a table stored in a computer memory database of the remote server that contains a listing of compatibility checks and actions associated with the compatibility checks. For example, if a version of the CGM module 208 is not determined to not be compatible with the version of operating system, the action can include sending software instructions to the smartphone 202 to disable one or more features of the CGM module 208 and/or send a notification to the user over smartphone 202 that the versions are not compatible and the user should change the version of the CGM module and/or version of the operating system. The notification can be by way of text message or can be by way of a local notification from inside the CGM module 208 in accordance with some embodiments.

Remote server can also proactively notify a user about operating compatibility issues. Remote server can store information about the current versions of CGM module 208 and operating system used by the smartphone 202. If a new version of the operating system is being released that is known to have compatibility problems with the version of CGM module 208 on the user's smartphone, then the remote server can programically initiate transmitting a notification (via text message or local message via CGM module) to the user's smartphone about what action to take. The action can be one or more of not installing the upgraded version of the operating system and installing an upgraded version of the CGM module 208 prior to upgrading to the new version of the operating system.

It has been recognized by the inventors, that a user may not realize that the CGM module 208 is not working properly due to a new version of an operating system (or other software) running on the user's smart phone in accordance with some implementations. For instance, if the CGM module 208 is not working properly, the user's smartphone may not be communicating with the remote server; thereby not notifying the remote server of the change in operating system. Accordingly, in some embodiments, remote server can monitor the communication between the user's smartphone and the remote server and determine that the CGM module is not working based on a lack of communication. In such an instance, the server can initiate a notification to the smartphone (e.g., text message) that notifies the user that the CGM module 208 may not be working and/or to change the version of the CGM module or operating system so that the CGM module will work.

### Context-Sensitive Help

In some of the present embodiments, context-sensitive help is provided in areas that link to intra-CGM module 208 help documentation. For example, if pairing between the sensor and the smartphone 202 fails, the user can click a help button within the CGM module 208 and it will load common pairing mistakes to help the user correct his or her issue. In these embodiments, the CGM module 208 receives as an input a user command to display 222 a given help topic. The CGM module 208 processes the input by accessing the requested help topic, and outputs the help topic to the display 222. In a related embodiment, the help could be interactive, such as an electronic chat. In these embodiments, the CGM module 208 receives as an input a user command to open a chat session. The CGM module 208 processes the input by establishing a connection with a remote help center, and produces an output in the form of a chat session. In some embodiments, the CGM module processes the input by linking to a training video or other media available online to assist the user.

### Shared Device

Some of the present embodiments include security features so that a given user's data is not shown to the wrong person in the case of multiple people sharing a device. For example, two family members may share the same smartphone 202. In this scenario, it is desirable to avoid showing each family member data that belongs to other people that share the device. One way the present embodiments avoid showing data to the wrong person is that when the user changes the pairing of the smartphone 202 to his or her transmitter, the smartphone 202 only queries the database for data associated with that transmitter. Thus, when either person pairs their transmitter only their data is shown. The user doesn't need to stop and restart a sensor, they can instantly re-pair.

### Estimate of Future Glucose Level

In various other embodiments, the user may query the CGM module 208 for an estimate of his or her future blood glucose level. The nature of the query may be independent of any preset limits or alerts, and may include the capability to provide inputs to the CGM module 208 to determine the impact of those inputs on future potential prediction outcomes. In one example, the user may want to know what impact an action taken or about to be taken will have on their blood glucose. If the user's glucose is 150 mg/dL and rising about 2 mg/dL/min, a prediction might show the user to be at 210 mg/dL in 30 minutes. But the user may query the CGM module 208 with an input of taking insulin to find out that his or her 30 minute prediction is now 190 mg/dL, thus indicating that taking insulin was a good decision or that taking insulin might be a good decision to mitigate a rising glucose level. Other examples of actions that the user might input to the CGM module 208 include eating (e.g. specific types of food), exercising (e.g. specific exercises), etc.

In another example, it is 7:00 PM and the user's glucose level is approximately 200 mg/dL. The user is planning to go to bed at 10:00 PM, and wants to target having a bedtime glucose of 120 mg/dL. First the user can query the CGM module 208 for a prediction of their 10:00 PM glucose level, and then the user can further query the CGM module 208 for potential actions or input potential actions to see which steps may help to achieve their target bedtime glucose levels.

In another example, the user queries the CGM module 208 for both bedtime and morning predicted glucose levels. If the user has a goal of 120 mg/dL for a 10:00 PM bedtime and 80 mg/dL for a 6:00 AM morning glucose, the user can first see the prediction and then the user can further query the CGM module 208 for potential actions or input potential actions to see which steps may help achieve their desired bedtime and morning glucose levels.

In the foregoing examples, the CGM module 208 takes as inputs the user's EGV from a CGM, and the user's input(s) of actions taken or actions that the user might take. The inputs might also include the user inputting a future time at which the user wants to know his or her blood glucose. The CGM module 208 processes the inputs by determining what impact the user's actions might have on his or her future blood glucose. The CGM module 208 then outputs the user's predicted blood glucose at the future time.

In some embodiments, an alert (hard or soft) can be set as the target time approaches to indicate to the user whether the prediction is still reasonable to support his or her future blood glucose goal. The CGM module 208 may also have a learning mode to see how planned actions and predicted results compared to actual future glucose levels.

The CGM module 208 of these embodiments may further include a recommendation engine. That is, part of the output from the CGM module 208 may be a recommendation on how the user might achieve his or her target future glucose level. For example, the CGM module 208 may take as an input a user's current glucose trend and/or a user's planned action, process the input(s) by projecting the user's future glucose level, and produce an output in the form of a recommended action. The action may be, for example, to eat, optionally including how much to eat, to take insulin, optionally including how much to take, etc. The user might then input what, if any, action he or she took. If so, the recommendation engine wouldn't make new recommendations for a certain period of time, in order to allow the previous action to take effect.

Advantageously, the present embodiments enable a user to better plan his or her day, night, or future glucose states. Further, the feedback that the user receives from the CGM module 208 can be potentially motivational to indicate whether planned actions or actions already taken may have the intended effect on future glucose levels. Still further, the present embodiment is a great learning tool that enables the user to see how various behaviors may impact glucose levels.

### Data Integrity

Some users may want to see their glucose data from multiple sources, but data integrity may be imperative in some embodiments. Instead of sending glucose numbers to a remote viewing device, the validated transmitting device could send a screen shot or image of the information. In doing this, only validation of the image would have to occur. For example, when a user would like to send their data to a parent or friend, who may also be a CGM user, it is important that this other CGM user does not mistake their glucose data for the friend's or child's glucose data. Accordingly, the screen shot or image will appear distinct from a real-time CGM screen, using differentiating features (color, font, background, demarcation, etc.) and/or merely by showing a captured image of a real-time screen. Standard validating techniques normally used by a smartphone for processing images may be used. One example of differentiating remote-monitored data from a user's own real-time data is by laying out the screen differently, such as by putting the EGV and the trend arrow on the opposite side of where they usually are. Additionally, or alternatively, an icon or a photograph of the user may be provided somewhere on the screen.

### Store

In some embodiments, the CGM module 208 determines whether support from or communication with the manufacturer may be beneficial, for example in order to upgrade the system, order supplies, request help, etc. In some embodiments, the CGM module 208 can track how many sensors have been ordered and used, and recommend or initiate reordering of new sensors. In some embodiments, the CGM module can track sensor errors and upload data to a technical support site for troubleshooting by the manufacturer. In some embodiments, the CGM module 208 can recommend accessories for a particular CGM user, for example, can determine if a CGM user may like a skin of a particular genre in line with their profile. In some embodiments, the CGM module 208 may determine when a warranty period is expiring and recommend or initiate an order. In some embodiments, the CGM module 208 will determine when an upgrade is available and initiate the upgrade process for the user. In some embodiments, the CGM module 208 uses location information to find a local sales person.

### Upgradeable CGM Module

In any of the foregoing embodiments, the CGM module 208 may be upgradeable. For example, the CGM module 208 may be available for download from a remote server in various formats. A basic CGM module 208 may be free, or very inexpensive, to download, and may provide only basic features, such as receiving data, displaying EGV, and providing basic analysis (e.g. trends and simple alerts). One or more advanced versions of the CGM module 208 may also be available for download. The advanced version(s) would provide more features, such as predictive alerts, advanced statistics, therapy management tips, cloud features, degree of confidence measures (reliability measures), different indication or use case, or any of the other aspects described herein. The advanced version(s) may be available for direct download from a remote server, or available only by prescription. The advanced version(s) may also be available for purchase on a subscription basis, such as monthly, or purchase for an upfront lump sum. In addition, there may be multiple tiers of advanced versions, with higher tier versions offering more features. There may be options for rewarding the user by opening up features of the CGM module 208 free of charge as a reward for good compliance, staying within BG limit goals, etc.

With respect to the advanced version(s), the algorithms may be executed by the sensor electronics 104. But, depending on the version of the CGM module 208 being used, the CGM module 208 will receive specific data to be displayed. For example, the CGM module 208 may have an encrypted identification that is transmitted to the sensor electronics 104 before the user is allowed to access specific algorithms and features.

### Simplified Display

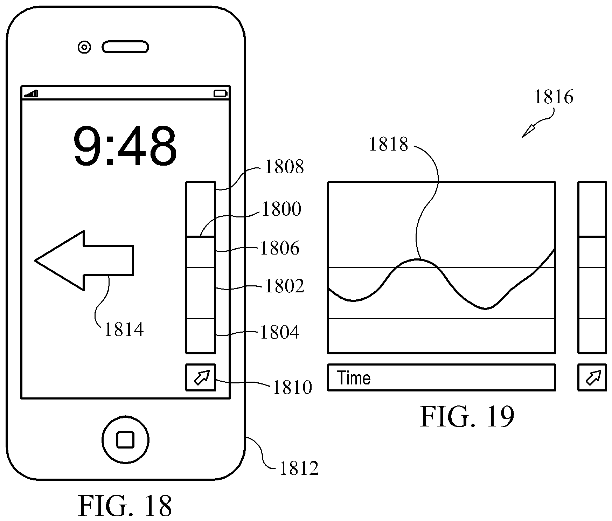

In any of the foregoing embodiments, the CGM module 208 may provide a simplified display. For example, with reference to Figure 18, the display may not provide a numerical glucose value. Instead, a bar 1800 moves up and down in a column 1802 that has differently colored zones to indicate the user's glucose level. In some embodiments, a lower zone 1804 may be colored red to indicate a hypoglycemic zone, a middle zone 1806 may be colored green to indicate the target glucose zone, and an upper zone 1808 may be colored yellow to indicate a hyperglycemic zone. The location of the bar 1800 within the column 1802, and the corresponding color, indicates the user's glucose level. Beneath the column 1802, an arrow 1810 may be provided to indicate the direction of a rate of change of the user's glucose. A predictive alarm may, for example, be indicated when the arrow 1810 blinks and/or changes color, for example to red or yellow, when the patient is predicted to cross into a hypo or hyperglycemic zone.

The bar 1800 has width to indicate the range the glucose is in. In this simplified version of CGM, the exact value is no longer emphasized. A wide bar 1800 or gradient of color indicates the range of glucose for the user. Using this range indicator, accuracy can be calculated by whether the true glucose is within the width of the bar 1800, improving the reported accuracy of the device.

In certain embodiments, the column 1802 of glucose information may be located on a front screen, or home screen 1812, of the smartphone. The home screen 1812 shows when the smartphone is first powered up, and when the display is reactivated after it has entered a sleep mode. By swiping an unlock bar 1814 on the display (if it is a touch screen), the trend graph 1816 shown in Figure 19 is displayed. The trend graph 1816 includes a background having similar background coloration to the column 1802, but greater width. A curve 1818 superimposed over the coloration indicates the user's glucose over time. This trend graph 1816 is similar to those described above, such as with respect to Figure 3A, except that the user's glucose is communicated using color instead of numerical values.

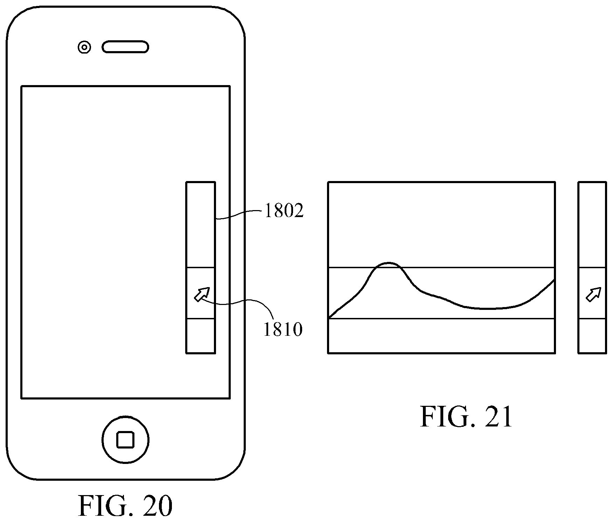

Figures 20 and 21 illustrate another alternative way of presenting direction or rate of change information. The embodiment of Figures 20 and 21 is similar to that of Figures 18 and 19, except that the arrow 1810 is provided inside the column 1802, rather than below the column 1802.

In some embodiments, a simplified display may be limited to only several glucose monitoring-related items. The items can consist of, for example, a numerical glucose value, a glucose directional arrow indicative of a rate-of-change of the user's glucose concentration, and an action icon that indicates an action that should be taken, if any. In some embodiments, the action icon can alert a user if an action should be taken due to the user's monitored glucose levels. As a non-limiting example, the action icon can indicate to a user that his or her glucose concentration is changing in a manner which may pose a clinical risk to the patient. Here, programmed high and low thresholds can be used along with a measured rate of change to predict if the user is in or about to enter a clinically risk state, such as impending hyperglycemia or hyperglycemia.

### Visual Aids for low-sighted users

Some embodiments include a manual "hard wired" button or action (such as shaking, swiping of a touch screen, pressing two buttons at the same time, etc.) or simplified set of key strokes causes display device 110, 111, 112, 113 (FIG.1) to show a displayed glucose value on the display in a large font, or large high contrast font, that fills or substantially fills the entire display screen. The display device 110, 111, 112, 113 may be configurable to suppress a nominal "home" screen in favor of a large font, or large font in high contrast, only reading. Many users of continuous glucose monitors may have poor, or transiently poor, vision due to their age, vision, or transient effects due to their glucose level. Additionally, users who would normally use vision aids, such as contacts or glasses, may find themselves in situations where they are not able to use such aids.

In many cases, the suite of information provided on the home screen of the display device 110, 111, 112, 113, while informational, may be more information than a user requires. The user with limited vision may have a difficult time determining the glucose reading due to the small font required for the inclusion of the additional information, which they may or may not need in a given situation. The inclusion of a fast sightless process, such as an external button, or manual swipe of a touchscreen will allow a sight limited user a simple one-step technique to get information which may be vital to them in a specific situation, such as at night, when they are not wearing vision aids, or when vision is distorted due to complications of diabetes.

### Continuous Glucose Monitoring Analysis Algorithms

In some embodiments, CAM module can include computer-readable instructions to perform one or more algorithms that include: 1) rate of change information in bolus estimators, 2) user-selectable criteria for retrospective analysis, 3) improvement information of continuous glucose data over time, 4) activity level data associated with alarms and/ or other computations and5) future glucose prediction inquires. Each of these algorithms can enhanced the usefulness of continuous glucose sensor data in analysis of patient's blood glucose information and diabetes management.

As discussed herein, continuous glucose sensor systems can include a sensor and one or more processing/display device(s), which devices(s) can be divided between two or more electronic components (e.g., sensor electronics physically connected to the sensor and one or more receivers located remote from the sensor) or performed mostly in a single device (e.g., receiver). In some implementations, it may be advantageous to provide a bolus insulin calculator/estimator (e.g., useful for determining a mealtime insulin bolus to be injected) within the processing/display device(s). In some embodiments, a bolus insulin calculator is provided wherein rate of change, trend, and/or directional information in included in the calculation of a dosing recommendation (e.g., via a bolus calculator) to be displayed on a display device and/or transmitted to an insulin delivery device. The bolus calculator can be used wherein rate of change, trend, and/or directional information can override a bolus recommendation. The bolus calculator can be configured to calculate a bolus recommendation based on 1) a current glucose value 2) estimated meal information and 3) rate of change, trend, and/or directional information.

In addition to using a continuous glucose monitoring system for real-time processing, retrospective analysis of one or a plurality of data sets (e.g., from one or a plurality of sensor sessions) can be useful to a healthcare provider in analyzing and understanding each patient's unique diabetes information (e.g., blood glucose data over time). In some embodiments, a computing system (e.g. display device 110, 11, 112, 113) is programmed with a series of therapy adjustment recommendation criteria that can be selected and/or deselected by a user, wherein the computing system is programmed to run the selected criteria against continuous glucose sensor data and provide highlighted problem areas and/or areas for target or focus on a display (e.g., computer screen). In one exemplary embodiment, a criterion is configured to evaluate overnight continuous glucose sensor data (e.g., glucose values, highs, lows, time spent above a target, time spent below a target, rate of change, glycemic variability, euglycemia, hypoglycemic events, and/or hyperglycemic events), whereby the computing system is programmed to recommend an adjusted basal insulin rate in response thereto. The information could be highlighted on a screen of data, provided as text; based tips, and/or the like.

Referring again to retrospective analysis of continuous glucose sensor data, some embodiments provide a computing system (e.g. display device 110, 11, 112, 113) programmed to display improvement information on a display screen (e.g., graph or charts), wherein the improvement information is indicative of a reduction in the number of hypoglycemic events, a reduction in time spent in hype or hyperglycemic regions, an increase in target and/or a reduction in glucose variability, for example. Improvement information can be presented for a variety of different time periods, for example, a day, a week, a month, or more.

In some embodiments, the computing system is configured to monitor an activity level associated with a host wearing a continuous glucose sensor. Two examples of activity level monitors include an accelerometer and/or heart rate monitor, however other activity level monitors can be used in combination with and/or alternative to the two cited examples. Preferably, data from the activity level monitor(s) is incorporated into algorithms associated with processing the continuous glucose sensor data. For example, a high measured activity level can be indicative of a greater likelihood of hypoglycemic events and/or a high level of adrenale can be indicative of an increase in blood glucose levels. Accordingly in one embodiment, a computer system is programmed to include data from an activity level monitor in its computations associated with alarms (e.g., alarms associated with a current glucose value and/or a predicted glucose level). In general, activity level data can be used to associate activity levels with blood glucose trends and values over time, thereby providing insight to a diabetic patient (and/or their parents) as to how an individual's activity levels affect their diabetes.

According to some embodiments, future glucose prediction queries can be used that allow a user can ask the CAM module to estimate their future glucose independent of pre-set limits or alerts, including providing inputs to impact future potential prediction outcomes. The following are non-limiting examples to help illustrate embodiments.

Example 1: a user wants to know the impact on their future glucose levels and glucose trend graph if they take an action or based on an action the user has already taken. If a user's glucose is 150 mg/dL and rising about 2 mg/dL/min, a prediction might show the user to be 210 mg/dL in 30 minutes, but a user could now query the system with an input of taking insulin to find out that their 30 minute prediction is now 190 mg/dL, thus indicating that taking insulin was a good decision or that taking insulin might be a good decision to mitigate their rising glucose levels.

Example 2: It is 7 pm and the user's glucose level is approximately 200 mg/dL. The user is planning to go to be at 10 pm and wants to target having a bedtime glucose of 120 mg/dL. First the user can query the system for a prediction of their 10 pm glucose level, and then the user can further query the system for potential actions or input potential actions to see which steps may help to achieve their target bedtime glucose levels.

Example 3: Similar to Example 2, except now the user wants to query the system for both bedtime and morning predicted glucose levels. If the user has a goal of 120 mg/dL for a 10pm bedtime and 80 mg/dL for a 6am morning glucose, the user can first see the prediction and then the user can further query the system for potential actions or input potential actions to see which steps may help achieve their desired bedtime and morning glucose levels. An alert (hard or soft) can be set as the target time is approached to indicate to the user whether the prediction or estimates are still reasonable to support their future glucose goals. The feature can further be set into a learning mode to see how planned actions and predicted results compared to actual future glucose levels.

Methods and devices that are suitable for use in conjunction with aspects of the preferred embodiments are disclosed in U.S. Pat. No. 4,757,022; U.S. Pat. No. 4,994,167; U.S. Pat. No. 6,001,067; U.S. Pat. No. 6,558,321; U.S. Pat. No. 6,702,857; U.S. Pat. No. 6,741,877; U.S. Pat. No. 6,862,465; U.S. Pat. No. 6,931,327; U.S. Pat. No. 7,074,307; U.S. Pat. No. 7,081,195; U.S. Pat. No. 7,108,778; U.S. Pat. No. 7,110,803; U.S. Pat. No. 7,134,999; U.S. Pat. No. 7,136,689; U.S. Pat. No. 7,192,450; U.S. Pat. No. 7,226,978; U.S. Pat. No. 7,276,029; U.S. Pat. No. 7,310,544; U.S. Pat. No. 7,364,592; U.S. Pat. No. 7,366,556; U.S. Pat. No. 7,379,765; U.S. Pat. No. 7,424,318; U.S. Pat. No. 7,460,898; U.S. Pat. No. 7,467,003; U.S. Pat. No. 7,471,972; U.S. Pat. No. 7,494,465; U.S. Pat. No. 7,497,827; U.S. Pat. No. 7,519,408; U.S. Pat. No. 7,583,990; U.S. Pat. No. 7,591,801; U.S. Pat. No. 7,599,726; U.S. Pat. No. 7,613,491; U.S. Pat. No. 7,615,007; U.S. Pat. No. 7,632,228; U.S. Pat. No. 7,637,868; U.S. Pat. No. 7,640,048; U.S. Pat. No. 7,651,596; U.S. Pat. No. 7,654,956; U.S. Pat. No. 7,657,297; U.S. Pat. No. 7,711,402; U.S. Pat. No. 7,713,574; U.S. Pat. No. 7,715,893; U.S. Pat. No. 7,761,130; U.S. Pat. No. 7,771,352; U.S. Pat. No. 7,774,145; U.S. Pat. No. 7,775,975; U.S. Pat. No. 7,778,680; U.S. Pat. No. 7,783,333; U.S. Pat. No. 7,792,562; U.S. Pat. No. 7,797,028; U.S. Pat. No. 7,826,981; U.S. Pat. No. 7,828,728; U.S. Pat. No. 7,831,287; U.S. Pat. No. 7,835,777; U.S. Pat. No. 7,857,760; U.S. Pat. No. 7,860,545; U.S. Pat. No. 7,875,293; U.S. Pat. No. 7,881,763; U.S. Pat. No. 7,885,697; U.S. Pat. No. 7,896,809; U.S. Pat. No. 7,899,511; U.S. Pat. No. 7,901,354; U.S. Pat. No. 7,905,833; U.S. Pat. No. 7,914,450; U.S. Pat. No. 7,917,186; U.S. Pat. No. 7,920,906; U.S. Pat. No. 7,925,321; U.S. Pat. No. 7,927,274; U.S. Pat. No. 7,933,639; U.S. Pat. No. 7,935,057; U.S. Pat. No. 7,946,984; U.S. Pat. No. 7,949,381; U.S. Pat. No. 7,955,261; U.S. Pat. No. 7,959,569; U.S. Pat. No. 7,970,448; U.S. Pat. No. 7,974,672; U.S. Pat. No. 7,976,492; U.S. Pat. No. 7,979,104; U.S. Pat. No. 7,986,986; U.S. Pat. No. 7,998,071; U.S. Pat. No. 8,000,901; U.S. Pat. No. 8,005,524; U.S. Pat. No. 8,005,525; U.S. Pat. No. 8,010,174; U.S. Pat. No. 8,027,708; U.S. Pat. No. 8,050,731; U.S. Pat. No. 8,052,601; U.S. Pat. No. 8,053,018; U.S. Pat. No. 8,060,173; U.S. Pat. No. 8,060,174; U.S. Pat. No. 8,064,977; U.S. Pat. No. 8,073,519; U.S. Pat. No. 8,073,520; U.S. Pat. No. 8,118,877; U.S. Pat. No. 8,128,562; U.S. Pat. No. 8,133,178; U.S. Pat. No. 8,150,488; U.S. Pat. No. 8,155,723; U.S. Pat. No. 8,160,669; U.S. Pat. No. 8,160,671; U.S. Pat. No. 8,167,801; U.S. Pat. No. 8,170,803; U.S. Pat. No. 8,195,265; U.S. Pat. No. 8,206,297; U.S. Pat. No. 8,216,139; U.S. Pat. No. 8,229,534; U.S. Pat. No. 8,229,535; U.S. Pat. No. 8,229,536; U.S. Pat. No. 8,231,531; U.S. Pat. No. 8,233,958; U.S. Pat. No. 8,233,959; U.S. Pat. No. 8,249,684; U.S. Pat. No. 8,251,906; U.S. Pat. No. 8,255,030; U.S. Pat. No. 8,255,032; U.S. Pat. No. 8,255,033; U.S. Pat. No. 8,257,259; U.S. Pat. No. 8,260,393; U.S. Pat. No. 8,265,725; U.S. Pat. No. 8,275,437; U.S. Pat. No. 8,275,438; U.S. Pat. No. 8,277,713; U.S. Pat. No. 8,280,475; U.S. Pat. No. 8,282,549; U.S. Pat. No. 8,282,550; U.S. Pat. No. 8,285,354; U.S. Pat. No. 8,287,453; U.S. Pat. No. 8,290,559; U.S. Pat. No. 8,290,560; U.S. Pat. No. 8,290,561; U.S. Pat. No. 8,290,562; U.S. Pat. No. 8,292,810; U.S. Pat. No. 8,298,142; U.S. Pat. No. 8,311,749; U.S. Pat. No. 8,313,434; U.S. Pat. No. 8,321,149; U.S. Pat. No. 8,332,008; U.S. Pat. No. 8,346,338; U.S. Pat. No. 8,364,229; U.S. Pat. No. 8,369,919; U.S. Pat. No. 8,374,667; U.S. Pat. No. 8,386,004; and U.S. Pat. No. 8,394,021.

Methods and devices that are suitable for use in conjunction with aspects of the preferred embodiments are disclosed in U.S. Patent Publication No. 2003-0032874-A1; U.S. Patent Publication No. 2005-0033132-A1; U.S. Patent Publication No. 2005-0051427-A1; U.S. Patent Publication No. 2005-0090607-A1; U.S. Patent Publication No. 2005-0176136-A1; U.S. Patent Publication No. 2005-0245799-A1; U.S. Patent Publication No. 2006-0015020-A1; U.S. Patent Publication No. 2006-0016700-A1; U.S. Patent Publication No. 2006-0020188-A1; U.S. Patent Publication No. 2006-0020190-A1; U.S. Patent Publication No. 2006-0020191-A1; U.S. Patent Publication No. 2006-0020192-A1; U.S. Patent Publication No. 2006-0036140-A1; U.S. Patent Publication No. 2006-0036143-A1; U.S. Patent Publication No. 2006-0040402-A1; U.S. Patent Publication No. 2006-0068208-A1; U.S. Patent Publication No. 2006-0142651-A1; U.S. Patent Publication No. 2006-0155180-A1; U.S. Patent Publication No. 2006-0198864-A1; U.S. Patent Publication No. 2006-0200020-A1; U.S. Patent Publication No. 2006-0200022-A1; U.S. Patent Publication No. 2006-0200970-A1; U.S. Patent Publication No. 2006-0204536-A1; U.S. Patent Publication No. 2006-0224108-A1; U.S. Patent Publication No. 2006-0235285-A1; U.S. Patent Publication No. 2006-0249381-A1; U.S. Patent Publication No. 2006-0252027-A1; U.S. Patent Publication No. 2006-0253012-A1; U.S. Patent Publication No. 2006-0257995-A1; U.S. Patent Publication No. 2006-0258761-A1; U.S. Patent Publication No. 2006-0263763-A1; U.S. Patent Publication No. 2006-0270922-A1; U.S. Patent Publication No. 2006-0270923-A1; U.S. Patent Publication No. 2007-0027370-A1; U.S. Patent Publication No. 2007-0032706-A1; U.S. Patent Publication No. 2007-0032718-A1; U.S. Patent Publication No. 2007-0045902-A1; U.S. Patent Publication No. 2007-0059196-A1; U.S. Patent Publication No. 2007-0066873-A1; U.S. Patent Publication No. 2007-0173709-A1; U.S. Patent Publication No. 2007-0173710-A1; U.S. Patent Publication No. 2007-0208245-A1; U.S. Patent Publication No. 2007-0208246-A1; U.S. Patent Publication No. 2007-0232879-A1; U.S. Patent Publication No. 2008-0045824-A1; U.S. Patent Publication No. 2008-0083617-A1; U.S. Patent Publication No. 2008-0086044-A1; U.S. Patent Publication No. 2008-0108942-A1; U.S. Patent Publication No. 2008-0119703-A1; U.S. Patent Publication No. 2008-0119704-A1; U.S. Patent Publication No. 2008-0119706-A1; U.S. Patent Publication No. 2008-0183061-A1; U.S. Patent Publication No. 2008-0183399-A1; U.S. Patent Publication No. 2008-0188731-A1; U.S. Patent Publication No. 2008-0189051-A1; U.S. Patent Publication No. 2008-0194938-A1; U.S. Patent Publication No. 2008-0197024-A1; U.S. Patent Publication No. 2008-0200788-A1; U.S. Patent Publication No. 2008-0200789-A1; U.S. Patent Publication No. 2008-0200791-A1; U.S. Patent Publication No. 2008-0214915-A1; U.S. Patent Publication No. 2008-0228054-A1; U.S. Patent Publication No. 2008-0242961-A1; U.S. Patent Publication No. 2008-0262469-A1; U.S. Patent Publication No. 2008-0275313-A1; U.S. Patent Publication No. 2008-0287765-A1; U.S. Patent Publication No. 2008-0306368-A1; U.S. Patent Publication No. 2008-0306434-A1; U.S. Patent Publication No. 2008-0306435-A1; U.S. Patent Publication No. 2008-0306444-A1; U.S. Patent Publication No. 2009-0018424-A1; U.S. Patent Publication No. 2009-0030294-A1; U.S. Patent Publication No. 2009-0036758-A1; U.S. Patent Publication No. 2009-0036763-A1; U.S. Patent Publication No. 2009-0043181-A1; U.S. Patent Publication No. 2009-0043182-A1; U.S. Patent Publication No. 2009-0043525-A1; U.S. Patent Publication No. 2009-0045055-A1; U.S. Patent Publication No. 2009-0062633-A1; U.S. Patent Publication No. 2009-0062635-A1; U.S. Patent Publication No. 2009-0076360-A1; U.S. Patent Publication No. 2009-0099436-A1; U.S. Patent Publication No. 2009-0124877-A1; U.S. Patent Publication No. 2009-0124879-A1; U.S. Patent Publication No. 2009-0124964-A1; U.S. Patent Publication No. 2009-0131769-A1; U.S. Patent Publication No. 2009-0131777-A1; U.S. Patent Publication No. 2009-0137886-A1; U.S. Patent Publication No. 2009-0137887-A1; U.S. Patent Publication No. 2009-0143659-A1; U.S. Patent Publication No. 2009-0143660-A1; U.S. Patent Publication No. 2009-0156919-A1; U.S. Patent Publication No. 2009-0163790-A1; U.S. Patent Publication No. 2009-0178459-A1; U.S. Patent Publication No. 2009-0192366-A1; U.S. Patent Publication No. 2009-0192380-A1; U.S. Patent Publication No. 2009-0192722-A1; U.S. Patent Publication No. 2009-0192724-A1; U.S. Patent Publication No. 2009-0192751-A1; U.S. Patent Publication No. 2009-0203981-A1; U.S. Patent Publication No. 2009-0216103-A1; U.S. Patent Publication No. 2009-0240120-A1; U.S. Patent Publication No. 2009-0240193-A1; U.S. Patent Publication No. 2009-0242399-A1; U.S. Patent Publication No. 2009-0242425-A1; U.S. Patent Publication No. 2009-0247855-A1; U.S. Patent Publication No. 2009-0247856-A1; U.S. Patent Publication No. 2009-0287074-A1; U.S. Patent Publication No. 2009-0299155-A1; U.S. Patent Publication No. 2009-0299156-A1; U.S. Patent Publication No. 2009-0299162-A1; U.S. Patent Publication No. 2010-0010331-A1; U.S. Patent Publication No. 2010-0010332-A1; U.S. Patent Publication No. 2010-0016687-A1; U.S. Patent Publication No. 2010-0016698-A1; U.S. Patent Publication No. 2010-0030484-A1; U.S. Patent Publication No. 2010-0036215-A1; U.S. Patent Publication No. 2010-0036225-A1; U.S. Patent Publication No. 2010-0041971-A1; U.S. Patent Publication No. 2010-0045465-A1; U.S. Patent Publication No. 2010-0049024-A1; U.S. Patent Publication No. 2010-0076283-A1; U.S. Patent Publication No. 2010-0081908-A1; U.S. Patent Publication No. 2010-0081910-A1; U.S. Patent Publication No. 2010-0087724-A1; U.S. Patent Publication No. 2010-0096259-A1; U.S. Patent Publication No. 2010-0121169-A1; U.S. Patent Publication No. 2010-0161269-A1; U.S. Patent Publication No. 2010-0168540-A1; U.S. Patent Publication No. 2010-0168541-A1; U.S. Patent Publication No. 2010-0168542-A1; U.S. Patent Publication No. 2010-0168543-A1; U.S. Patent Publication No. 2010-0168544-A1; U.S. Patent Publication No. 2010-0168545-A1; U.S. Patent Publication No. 2010-0168546-A1; U.S. Patent Publication No. 2010-0168657-A1; U.S. Patent Publication No. 2010-0174157-A1; U.S. Patent Publication No. 2010-0174158-A1; U.S. Patent Publication No. 2010-0174163-A1; U.S. Patent Publication No. 2010-0174164-A1; U.S. Patent Publication No. 2010-0174165-A1; U.S. Patent Publication No. 2010-0174166-A1; U.S. Patent Publication No. 2010-0174167-A1; U.S. Patent Publication No. 2010-0179401-A1; U.S. Patent Publication No. 2010-0179402-A1; U.S. Patent Publication No. 2010-0179404-A1; U.S. Patent Publication No. 2010-0179408-A1; U.S. Patent Publication No. 2010-0179409-A1; U.S. Patent Publication No. 2010-0185065-A1; U.S. Patent Publication No. 2010-0185069-A1; U.S. Patent Publication No. 2010-0185070-A1; U.S. Patent Publication No. 2010-0185071-A1; U.S. Patent Publication No. 2010-0185075-A1; U.S. Patent Publication No. 2010-0191082-A1; U.S. Patent Publication No. 2010-0198035-A1; U.S. Patent Publication No. 2010-0198036-A1; U.S. Patent Publication No. 2010-0212583-A1; U.S. Patent Publication No. 2010-0217557-A1; U.S. Patent Publication No. 2010-0223013-A1; U.S. Patent Publication No. 2010-0223022-A1; U.S. Patent Publication No. 2010-0223023-A1; U.S. Patent Publication No. 2010-0228109-A1; U.S. Patent Publication No. 2010-0228497-A1; U.S. Patent Publication No. 2010-0240975-A1; U.S. Patent Publication No. 2010-0240976 C1; U.S. Patent Publication No. 2010-0261987-A1; U.S. Patent Publication No. 2010-0274107-A1; U.S. Patent Publication No. 2010-0280341-A1; U.S. Patent Publication No. 2010-0286496-A1; U.S. Patent Publication No. 2010-0298684-A1; U.S. Patent Publication No. 2010-0324403-A1; U.S. Patent Publication No. 2010-0331656-A1; U.S. Patent Publication No. 2010-0331657-A1; U.S. Patent Publication No. 2011-0004085-A1; U.S. Patent Publication No. 2011-0009727-A1; U.S. Patent Publication No. 2011-0024043-A1; U.S. Patent Publication No. 2011-0024307-A1; U.S. Patent Publication No. 2011-0027127-A1; U.S. Patent Publication No. 2011-0027453-A1; U.S. Patent Publication No. 2011-0027458-A1; U.S. Patent Publication No. 2011-0028815-A1; U.S. Patent Publication No. 2011-0028816-A1; U.S. Patent Publication No. 2011-0046467-A1; U.S. Patent Publication No. 2011-0077490-A1; U.S. Patent Publication No. 2011-0118579-A1; U.S. Patent Publication No. 2011-0124992-A1; U.S. Patent Publication No. 2011-0125410-A1; U.S. Patent Publication No. 2011-0130970-A1; U.S. Patent Publication No. 2011-0130971-A1; U.S. Patent Publication No. 2011-0130998-A1; U.S. Patent Publication No. 2011 -0144465-A1; U.S. Patent Publication No. 2011-0178378-A1; U.S. Patent Publication No. 2011-0190614-A1; U.S. Patent Publication No. 2011-0201910-A1; U.S. Patent Publication No. 2011-0201911-A1; U.S. Patent Publication No. 2011-0218414-A1; U.S. Patent Publication No. 2011-0231140-A1; U.S. Patent Publication No. 2011-0231141-A1; U.S. Patent Publication No. 2011-0231142-A1; U.S. Patent Publication No. 2011-0253533-A1; U.S. Patent Publication No. 2011-0263958-A1; U.S. Patent Publication No. 2011-0270062-A1; U.S. Patent Publication No. 2011-0270158-A1; U.S. Patent Publication No. 2011-0275919-A1; U.S. Patent Publication No. 2011-0290645-A1; U.S. Patent Publication No. 2011-0313543-A1; U.S. Patent Publication No. 2011-0320130-A1; U.S. Patent Publication No. 2012-0035445-A1; U.S. Patent Publication No. 2012-0040101-A1; U.S. Patent Publication No. 2012-0046534-A1; U.S. Patent Publication No. 2012-0078071-A1; U.S. Patent Publication No. 2012-0108934-A1; U.S. Patent Publication No. 2012-0130214-A1; U.S. Patent Publication No. 2012-0172691-A1; U.S. Patent Publication No. 2012-0179014-A1; U.S. Patent Publication No. 2012-0186581-A1; U.S. Patent Publication No. 2012-0190953-A1; U.S. Patent Publication No. 2012-0191063-A1; U.S. Patent Publication No. 2012-0203467-A1; U.S. Patent Publication No. 2012-0209098-A1; U.S. Patent Publication No. 2012-0215086-A1; U.S. Patent Publication No. 2012-0215087-A1; U.S. Patent Publication No. 2012-0215201-A1; U.S. Patent Publication No. 2012-0215461-A1; U.S. Patent Publication No. 2012-0215462-A1; U.S. Patent Publication No. 2012-0215496-A1; U.S. Patent Publication No. 2012-0220979-A1; U.S. Patent Publication No. 2012-0226121-A1; U.S. Patent Publication No. 2012-0228134-A1; U.S. Patent Publication No. 2012-0238852-A1; U.S. Patent Publication No. 2012-0245448-A1; U.S. Patent Publication No. 2012-0245855-A1; U.S. Patent Publication No. 2012-0255875-A1; U.S. Patent Publication No. 2012-0258748-A1; U.S. Patent Publication No. 2012-0259191-A1; U.S. Patent Publication No. 2012-0260323-A1; U.S. Patent Publication No. 2012-0262298-A1; U.S. Patent Publication No. 2012-0265035-A1; U.S. Patent Publication No. 2012-0265036-A1; U.S. Patent Publication No. 2012-0265037-A1; U.S. Patent Publication No. 2012-0277562-A1; U.S. Patent Publication No. 2012-0277566-A1; U.S. Patent Publication No. 2012-0283541-A1; U.S. Patent Publication No. 2012-0283543-A1; U.S. Patent Publication No. 2012-0296311-A1; U.S. Patent Publication No. 2012-0302854-A1; U.S. Patent Publication No. 2012-0302855-A1; U.S. Patent Publication No. 2012-0323100-A1; U.S. Patent Publication No. 2013-0012798-A1; U.S. Patent Publication No. 2013-0030273-A1; U.S. Patent Publication No. 2013-0035575-A1; U.S. Patent Publication No. 2013-0035865-A1; U.S. Patent Publication No. 2013-0035871-A1; U.S. Patent Publication No. 2005-0056552-A1; U.S. Patent Publication No. 2005-0182451-A1; U.S. Patent Publication No. 2013000536650A1; and U.S. Patent Publication No. 2013-0053666-A1.

Methods and devices that are suitable for use in conjunction with aspects of the preferred embodiments are disclosed in U.S. Appl. No. 09/447,227 filed on November 22, 1999 and entitled "DEVICE AND METHOD FOR DETERMINING ANALYTE LEVELS"; U.S. Appl. No. 12/828,967 filed on July 1, 2010 and entitled "HOUSING FOR AN INTRAVASCULAR SENSOR"; U.S. Appl. No. 13/461,625 filed on May 1, 2012 and entitled "DUAL ELECTRODE SYSTEM FOR A CONTINUOUS ANALYTE SENSOR"; U.S. Appl. No. 13/594,602 filed on August 24, 2012 and entitled "POLYMER MEMBRANES FOR CONTINUOUS ANALYTE SENSORS"; U.S. Appl. No. 13/594,734 filed on August 24, 2012 and entitled "POLYMER MEMBRANES FOR CONTINUOUS ANALYTE SENSORS"; U.S. Appl. No. 13/607,162 filed on September 7, 2012 and entitled "SYSTEM AND METHODS FOR PROCESSING ANALYTE SENSOR DATA FOR SENSOR CALIBRATION"; U.S. Appl. No. 13/624,727 filed on September 21, 2012 and entitled "SYSTEMS AND METHODS FOR PROCESSING AND TRANSMITTING SENSOR DATA"; U.S. Appl. No. 13/624,808 filed on September 21, 2012 and entitled "SYSTEMS AND METHODS FOR PROCESSING AND TRANSMITTING SENSOR DATA"; U.S. Appl. No. 13/624,812 filed on September 21, 2012 and entitled "SYSTEMS AND METHODS FOR PROCESSING AND TRANSMITTING SENSOR DATA"; U.S. Appl. No. 13/732,848 filed on January 2, 2013 and entitled "ANALYTE SENSORS HAVING A SIGNAL-TO-NOISE RATIO SUBSTANTIALLY UNAFFECTED BY NON-CONSTANT NOISE"; U.S. Appl. No. 13/733,742 filed on January 3, 2013 and entitled "END OF LIFE DETECTION FOR ANALYTE SENSORS"; U.S. Appl. No. 13/733,810 filed on January 3, 2013 and entitled "OUTLIER DETECTION FOR ANALYTE SENSORS"; U.S. Appl. No. 13/742,178 filed on January 15, 2013 and entitled "SYSTEMS AND METHODS FOR PROCESSING SENSOR DATA"; U.S. Appl. No. 13/742,694 filed on January 16, 2013 and entitled "SYSTEMS AND METHODS FOR PROVIDING SENSITIVE AND SPECIFIC ALARMS"; U.S. Appl. No. 13/742,841 filed on January 16, 2013 and entitled "SYSTEMS AND METHODS FOR DYNAMICALLY AND INTELLIGENTLY MONITORING A HOST'S GLYCEMIC CONDITION AFTER AN ALERT IS TRIGGERED"; U.S. Appl. No. 13/747,746 filed on January 23, 2013 and entitled "DEVICES, SYSTEMS, AND METHODS TO COMPENSATE FOR EFFECTS OF TEMPERATURE ON IMPLANTABLE SENSORS"; U.S. Appl. No. 13/779,607 filed on February 27, 2013 and entitled "ZWITTERION SURFACE MODIFICATIONS FOR CONTINUOUS SENSORS"; U.S. Appl. No. 13/780,808 filed on February 28, 2013 and entitled "SENSORS FOR CONTINUOUS ANALYTE MONITORING, AND RELATED METHODS"; U.S. Appl. No. 13/784,523 filed on March 4, 2013 and entitled "ANALYTE SENSOR WITH INCREASED REFERENCE CAPACITY"; U.S. Appl. No. 13/789,371 filed on March 7, 2013 and entitled "MULTIPLE ELECTRODE SYSTEM FOR A CONTINUOUS ANALYTE SENSOR, AND RELATED METHODS"; U.S. Appl. No. 13/789,279 filed on March 7, 2013 and entitled "USE OF SENSOR REDUNDANCY TO DETECT SENSOR FAILURES"; U.S. Appl. No. 13/789,339 filed on March 7, 2013 and entitled "DYNAMIC REPORT BUILDING"; U.S. Appl. No. 13/789,341 filed on March 7, 2013 and entitled "REPORTING MODULES"; and U.S. Appl. No. 13/790,281 filed on March 8, 2013 and entitled "SYSTEMS AND METHODS FOR MANAGING GLYCEMIC VARIABILITY".

The above description presents the best mode contemplated for carrying out the present invention, and of the manner and process of making and using it, in such full, clear, concise, and exact terms as to enable any person skilled in the art to which it pertains to make and use this invention. This invention is, however, susceptible to modifications and alternate constructions from that discussed above that are fully equivalent. Consequently, this invention is not limited to the particular embodiments disclosed. On the contrary, this invention covers all modifications and alternate constructions coming within the spirit and scope of the invention as generally expressed by the following claims, which particularly point out and distinctly claim the subject matter of the invention. While the disclosure has been illustrated and described in detail in the drawings and foregoing description, such illustration and description are to be considered illustrative or exemplary and not restrictive.

All references cited herein are incorporated herein by reference in their entirety. To the extent publications and patents or patent applications incorporated by reference contradict the disclosure contained in the specification, the specification is intended to supersede and/or take precedence over any such contradictory material.

Unless otherwise defined, all terms (including technical and scientific terms) are to be given their ordinary and customary meaning to a person of ordinary skill in the art, and are not to be limited to a special or customized meaning unless expressly so defined herein. It should be noted that the use of particular terminology when describing certain features or aspects of the disclosure should not be taken to imply that the terminology is being re-defined herein to be restricted to include any specific characteristics of the features or aspects of the disclosure with which that terminology is associated. Terms and phrases used in this application, and variations thereof, especially in the appended claims, unless otherwise expressly stated, should be construed as open ended as opposed to limiting. As examples of the foregoing, the term 'including' should be read to mean 'including, without limitation,' 'including but not limited to,' or the like; the term 'comprising' as used herein is synonymous with 'including,' 'containing,' or 'characterized by,' and is inclusive or open-ended and does not exclude additional, unrecited elements or method steps; the term 'having' should be interpreted as 'having at least;' the term 'includes' should be interpreted as 'includes but is not limited to;' the term 'example' is used to provide exemplary instances of the item in discussion, not an exhaustive or limiting list thereof; adjectives such as 'known', 'normal', 'standard', and terms of similar meaning should not be construed as limiting the item described to a given time period or to an item available as of a given time, but instead should be read to encompass known, normal, or standard technologies that may be available or known now or at any time in the future; and use of terms like 'preferably,' 'preferred,' 'desired,' or 'desirable,' and words of similar meaning should not be understood as implying that certain features are critical, essential, or even important to the structure or function of the invention, but instead as merely intended to highlight alternative or additional features that may or may not be utilized in a particular embodiment of the invention. Likewise, a group of items linked with the conjunction 'and' should not be read as requiring that each and every one of those items be present in the grouping, but rather should be read as 'and/or' unless expressly stated otherwise. Similarly, a group of items linked with the conjunction 'or' should not be read as requiring mutual exclusivity among that group, but rather should be read as 'and/or' unless expressly stated otherwise.

Where a range of values is provided, it is understood that the upper and lower limit, and each intervening value between the upper and lower limit of the range is encompassed within the embodiments.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity. The indefinite article 'a' or 'an' does not exclude a plurality. A single processor or other unit may fulfill the functions of several items recited in the claims. The mere fact that certain measures are recited in mutually different dependent claims does not indicate that a combination of these measures cannot be used to advantage. Any reference signs in the claims should not be construed as limiting the scope.

It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases 'at least one' and 'one or more' to introduce claim recitations. However, the use of such phrases should not be construed to imply that the introduction of a claim recitation by the indefinite articles 'a' or 'an' limits any particular claim containing such introduced claim recitation to embodiments containing only one such recitation, even when the same claim includes the introductory phrases 'one or more' or 'at least one' and indefinite articles such as 'a' or 'an' (e.g., 'a' and/or 'an' should typically be interpreted to mean 'at least one' or 'one or more'); the same holds true for the use of definite articles used to introduce claim recitations. In addition, even if a specific number of an introduced claim recitation is explicitly recited, those skilled in the art will recognize that such recitation should typically be interpreted to mean at least the recited number (e.g., the bare recitation of 'two recitations,' without other modifiers, typically means at least two recitations, or two or more recitations). Furthermore, in those instances where a convention analogous to 'at least one of A, B, and C, etc.' is used, in general such a construction is intended in the sense one having skill in the art would understand the convention (e.g., 'a system having at least one of A, B, and C' would include but not be limited to systems that have A alone, B alone, C alone, A and B together, A and C together, B and C together, and/or A, B, and C together, etc.). In those instances where a convention analogous to 'at least one of A, B, or C, etc.' is used, in general such a construction is intended in the sense one having skill in the art would understand the convention (e.g., 'a system having at least one of A, B, or C' would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase 'A or B' will be understood to include the possibilities of 'A' or 'B' or 'A and B.'

All numbers expressing quantities of ingredients, reaction conditions, and so forth used in the specification are to be understood as being modified in all instances by the term 'about.' Accordingly, unless indicated to the contrary, the numerical parameters set forth herein are approximations that may vary depending upon the desired properties sought to be obtained. At the very least, and not as an attempt to limit the application of the doctrine of equivalents to the scope of any claims in any application claiming priority to the present application, each numerical parameter should be construed in light of the number of significant digits and ordinary rounding approaches.

Furthermore, although the foregoing has been described in some detail by way of illustrations and examples for purposes of clarity and understanding, it is apparent to those skilled in the art that certain changes and modifications may be practiced. Therefore, the description and examples should not be construed as limiting the scope of the invention to the specific embodiments and examples described herein, but rather to also cover all modification and alternatives coming with the true scope and spirit of the invention.

The present disclosure can be described further with respect to the following clauses:
1. A machine-executed method of continuous analyte monitoring for a host to facilitate management of the host's blood glucose level, the method comprising:
   receiving a first input from a timekeeping/scheduling module executed by an electronic device, the first input including information about an upcoming event;
   receiving a second input from a continuous analyte monitoring (CAM) device including analyte concentration data of the host;
   processing the first and second inputs by analyzing an event or an operational mode associated with either of the timekeeping/scheduling module or the CAM device; and
   producing an output by synchronizing the event or the operational mode of at least one of the timekeeping/scheduling module and the CAM device with the other of the timekeeping/scheduling module and the CAM device.
2. The method of Clause 1, wherein the output is to the timekeeping/scheduling module to schedule an event.
3. The method of any of the preceding Clauses, wherein the event is to eat, to obtain a reference glucose value, or to inject insulin.
4. The method of any of the preceding Clauses, wherein the output is sent to a user, a caretaker, a parent, a guardian, or a healthcare professional.
5. The method of any of the preceding Clauses, wherein the output comprises a recommendation provided via screen prompt, text message, e-mail message, or a post to a social network.
6. The method of any of the preceding Clauses, wherein the output comprises a change in the operating mode of the electronic device.
7. The method of any of the preceding Clauses, wherein the operating mode is a vibrate mode or a silent mode.
8. The method of any of the preceding Clauses, wherein the processing comprises analyzing a user's blood glucose data.
9. The method of any of the preceding Clauses, wherein the output comprises instructions to schedule an event on the timekeeping/scheduling module.
10. The method of any of the preceding Clauses, wherein the event is insertion of a new continuous analyte sensor, to eat, or to exercise.
11. The method of any of the preceding Clauses, wherein the output comprises a recommendation.
12. The method Clause 11, wherein the recommendation comprise one or more of a therapy, to schedule a doctor's appointment, to call a caretaker, to send data to a caretaker, to send data to a doctor, to eat a meal, to exercise, to replace a sensor, to calibrate a sensor, to check blood glucose, to upload or sync data to a cloud computing system.
13. The method of any of the preceding Clauses, wherein the recommendation is provided via screen prompt, text message, email message, or social network posting.
14. A system configured to implement the method of any of Clauses 1-13, the system comprising an electronic computing device and the CAM device.
15. The system of Clause 14, wherein the electronic device is a mobile computing device.
16. A machine-executed method of continuous analyte monitoring for a host to facilitate management of the host's blood glucose level, the method comprising:
   receiving a first input from an activity monitor executed by an electronic device, wherein the activity monitor provides information regarding the activity;
   receiving a second input from a continuous analyte monitoring (CAM) device, wherein the CAM device measures a concentration of an analyte within the host and outputs a value that represents the host's blood glucose level;
   processing the first and second inputs, wherein correlations or impacts of the activity on the host's blood glucose level are determined to associate the host's blood glucose level with the activity; and
   producing an output associated with the activity responsive to the determined associations.
17. The method of Clause 16, wherein processing the first and second inputs comprises applying one or more algorithms to two streams of input, including algorithms useful for correlating data and/or recognizing patterns.
18. The method of any of Clauses 16-17, wherein the output comprises data that is stored locally or remotely for retrospective analysis.
19. The method of any of Clauses 16-18, wherein the output comprises a warning of a low blood glucose level while the user is engaged in certain activities that might amplify a level of danger associated with the low blood glucose level, wherein the warning is distinct from a standard alert or alarm typically issued when the user is not engaged one of the certain activities.
20. The method any of Clauses 16-19, wherein the output comprises a warning of a low blood glucose level while the user is engaged in certain activities that might amplify a level of danger associated with the low blood glucose level, wherein the warning is provided at a different blood glucose threshold than a threshold that would trigger an alert when the user is not engaged one of the certain activities.
21. A system configured to implement the method of any of Clauses 16-20, the system comprising an electronic computing device and the CAM device.
22. A machine-executed method of continuous analyte monitoring for a host to facilitate management of the host's blood glucose level, the method comprising:
   receiving a first input from an image capture module executed by an electronic device, the first input providing information about at least one of a location, an activity, or a food;
   receiving a second input from a continuous analyte monitoring (CAM) device including analyte concentration data of the host;
   processing the first and second inputs by correlating information associated with the first input with the analyte concentration data of the host; and
   outputting information associated with the first input.
23. The method Clause 22, wherein the second input is an estimated glucose value at a time substantially corresponding to a time when the photograph was taken.
24. A system configured to implement the method of any of Clauses 22-23, the system comprising an electronic computing device and the CAM device.
25. A machine-executed method of continuous analyte monitoring for a host to facilitate management of the host's blood glucose level, the method comprising:
   receiving a first input from a continuous analyte monitor (CAM) device including analyte concentration data of the host;
   receiving or determining an expected maximum value and an expected minimum reference value;
   receiving a first reference value from a reference analyte monitor;
   processing the first reference value by comparing it to the expected maximum value and the expected minimum value; and
   producing an output of an alert to obtain a second reference value if the first reference value does not meet the range defined by the expected maximum value and the expected minimum value.
26. The method of Clause 25, wherein the method of Clause 25 is performed by a hand-held computing device in wireless communication with the CAM, and wherein the CAM comprises a continuous analyte sensor coupled to CAM electronics, the CAM electronics configured to calibrate the continuous analyte sensor output.
27. The method of any of Clauses 25-26, wherein the CAM electronics determines the expected maximum reference value and the expected minimum reference value and transmits each of the expected maximum reference value, the expected minimum reference value and the calibrated continuous analyte sensor output to the hand-held computing device.
28. The method of any of Clauses 25-27, further comprising periodically and regularly establishing a wireless communication channel between the CAM electronics and the hand-held computing device, and wherein the transmitting occurs within an established communication channel.
29. The method of any of Clauses 25-28, wherein the CAM electronics periodically generates a calibrated continuous sensor data value.
30. The method of Clause 29, wherein both the period for establishing the communication channel and the period for generating the calibrated continuous sensor data value are substantially the same.
31. The method of Clause 30, wherein both of the establishing period and the generating period are 1, 5 or 10 minutes.
32. The method of any of Clauses 25-31, wherein the reference analyte monitor is a single-point blood glucose monitor, and wherein the receiving the first reference analyte value comprises receiving a user input on a user interface of the hand-held computing device.
33. The method of any of Clauses 25-32, further comprising displaying the first reference analyte value on the display of the hand-held computing device if the first reference analyte value meets the range defined by the expected maximum value and the expected minimum value, wherein the first reference value is displayed on the hand-held computing device until a calibrated continuous analyte sensor value based on the first reference value is received from the CAM and displayed on the hand-held computing device display.
34. The method of Clause 33, wherein the first reference value is displayed differently than the calibrated continuous analyte sensor value by displaying the first reference value is a different color and/or font size than the calibrated CGM value.
35. A system for continuous analyte monitoring for a host to facilitate management of the host's blood glucose level, the system comprising a hand-held computing device configured to implement any one of the methods of Clauses 25-34.
36. A machine-executed method of continuous glucose monitoring for a host to facilitate management of the host's blood glucose level, the method comprising:
   displaying a blood glucose trend graph on a display of an electronic device;
   receiving an input of at least one location on the trend graph;
   processing the input by determining a value of the host's glucose concentration at the at least one location; and
   producing an output of the value on the display.
37. The method of Clause 36, wherein the input is locations of two points on the trend graph where the electronic device has sensed a user has placed his or her fingers.
38. The method of any of Clauses 36-37, wherein the processing comprises determining values of glucose concentration at the two points.
39. The method of any of Clauses 36-38, wherein the output comprises a value of a difference between glucose concentration values at the two points.
40. The method of any of Clauses 36-39, wherein the output comprises a value of a rate of change of glucose concentration between the two points.
41. The method of any of Clauses 36-40, wherein the glucose concentration trend graph comprises a macro trend graph that displays the user's glucose concentration data for a given length of time, and a micro trend graph that displays the user's glucose concentration data for a segment of the time shown on the macro trend graph.
42. The method of any of Clauses 36-41, wherein a movable bar on the macro trend graph highlights the segment of the time on the macro trend graph that is displayed on the micro trend graph.
43. The method of Clause 42, wherein the bar is movable to select any desired window of time on the macro trend graph to be displayed on the micro trend graph.
44. The method any of Clauses 42-43, wherein a width of the bar is adjustable to adjust a length of time displayed on the micro trend graph.
45. The method of Clause 44, wherein the width of the bar is adjustable by selecting one of a plurality of time select icons displayed on the display.
46. The method Clause 45, wherein if a scale of the macro trend graph is changed by a given factor, values of the time select icons change by a corresponding factor.
47. A system for continuous analyte monitoring for a host to facilitate management of the host's blood glucose level, the system comprising a hand-held computing device configured to implement any one of the methods of Clauses 36-46.
48. A machine-executed method of continuous analyte monitoring for a host to facilitate management of the host's analyte concentration level, the method comprising:
   transmitting, using a computing device, an identification of a version of an operating system of the computing device and/or a continuous analyte monitoring (CAM) software application installed on the computing device to a remote server, the CAM software application comprising instructions, when executed by a processor of the computing device, that cause the computing device to process and display analyte concentration information;
   receiving a response from the remote server responsive indicative of a compatibility check of the version of the operating system with the version of the CAM software application; and
   producing an output to responsive to the response.
49. The method of Clause 48, wherein the output comprises continuing operating the CAM module.
50. The method of any of Clauses 48-49, wherein the output comprises disabling one or more features of the CAM module.
51. The method of any of Clauses 48-50, wherein the output comprises preventing operation of the CAM module.
52. The method of any of Clauses 48-51, wherein the output comprises displaying a message to a user of the electronic device indicative of the compatibility check.
53. The method of Clause 52, wherein the message instructs the user to change at least one of the operating system and the CAM module to a different version.
54. The method of any of Clauses 48-53, wherein the CAM module is a software application stored on the electronic device.
55. The method of any of Clauses 48-54, wherein the transmitting is programically initiated responsive to detecting that a new operating system has been installed on the computing device.
56. The method of any of Clauses 48-55, wherein the remote server comprises a list of compatible versions of operating systems with version of the CAM software application stored in computer memory.
57. The method of any of Clauses 48-56, further comprising initiating a notification to the computing device regarding the compatibility of a new version of the operating system with the version of the CAM software application installed on the computing device prior to the new version of the operating system being installed on the computing device.
58. A system for continuous analyte monitoring for a host to facilitate management of the host's blood glucose level, the system comprising a hand-held computing device configured to implement any one of the methods of Clauses 48-57.

## Claims

1. A method of activating a hypoglycemic indicator based on continuous glucose sensor data, the method comprising:
evaluating sensor data to determine whether a real time glucose value meets a first user settable threshold;
evaluating sensor data using a predictive algorithm to determine whether a predicted glucose value meets one or more second fixed parameters that are not user settable;
activating a hypoglycemic indicator if either the first user settable threshold or the one or more second fixed parameters are met; and
providing an output based on the activated hypoglycemic indicator.

2. The method of claim 1, wherein the evaluating sensor data to determine whether the real time glucose value meets the first user settable threshold comprises determining whether the real-time glucose value passes a glucose threshold.

3. The method of claim 2, wherein the evaluating sensor data to determine whether the real time glucose value meets the first user settable threshold further comprises determining whether an amplitude of rate of change or direction of rate of change meets a rate of change criterion.

4. The method of claim 1, wherein the evaluating sensor data to determine whether the real time glucose value meets the first user settable threshold comprises evaluating a static risk of a substantially real time glucose value.

5. The method of claim 1, wherein the evaluating sensor data using the predictive algorithm to determine whether the predicted glucose value meets the one or more second fixed parameters comprises applying a priori knowledge dynamically.

6. The method of claim 1, wherein evaluating the sensor data allows for increased warning time of hypoglycemic alerts without substantially increasing the number of alerts.

7. The method of claim 1, wherein the predictive algorithm comprises a prediction horizon.

8. The method of claim 1, wherein the hypoglycemic indicator comprises a particular set of instructions.

9. The method of claim 1, wherein the output comprises at least one of an audible or visual output.

10. The method of claim 1, wherein providing an output comprises suspending insulin delivery in an insulin delivery device.

11. The method of any preceding claim, further comprising setting a default for the first user settable threshold, and optionally, further comprising receiving a user input to override the default setting.

12. The method of any preceding claim, further comprising outputting a predictive alert when a severe hypoglycemic event is predicted.

13. The method of claim 12, wherein the output comprises the text Going LOW, and optionally, wherein the predictive alert is configured to override any application or mode of a smartphone to interrupt or prioritize the display of the predictive alert on the smartphone.

14. A system for processing data, the system comprising:
a continuous analyte sensor configured to be implanted within a body; and
sensor electronics configured to receive and process sensor data output by the sensor, the sensor electronics including a processor configured to perform the method of any of claims 1 to 13.

15. The system of claim 14, further comprising an insulin delivery device for insulin delivery.
